# EUROPEAN PATENT APPLICATION

(11) **EP 4 534 129 A1**
(43) Date of publication of application: **09.04.2025**
(21) Application number: 23885136.4
(22) Date of filing: 06.11.2023
(51) Int. Cl.: A61M 16/06, A61F 5/56, A61M 16/00

(54) **MODEL SELECTION METHOD FOR NEGATIVE PRESSURE FACE MASK ASSEMBLY, AND MODEL SELECTION APPARATUS**

(30) Priority: 04.11.2022 CN 202211379985; 04.11.2022 CN 202222948733 U; 04.11.2022 CN 202222948215 U; 04.11.2022 CN 202222948281 U; 04.11.2022 CN 202211379575
(71) Applicant: BMC Medical Co., Ltd., Beijing 100073 (CN)
(72) Inventor: ZHOU, Mingzhao, Beijing 100073 (CN); HE, Long, Beijing 100073 (CN); ZHUANG, Zhi, Beijing 100073 (CN)
(74) Representative: Grünecker Patent- und Rechtsanwälte PartG mbB
(86) International application number: PCT/CN2023/129918
(87) International publication number: WO 2024/094212

(57) **Abstract**

A model selection method for a negative pressure face mask assembly, and a model selection apparatus. The model selection method includes: obtaining an interval distance between both earlobes of a patient, and determining a width of the negative pressure face mask assembly according to the interval distance; obtaining a perimeter of a neck of the patient, and determining a curvature of a side of the negative pressure face mask assembly in contact with the patient according to the perimeter; and determining a model of the negative pressure face mask assembly according to the width and the curvature. The model selection method is able to precisely determine the model of the negative pressure face mask assembly that suits the patient.

## Description

### CROSS-REFERENCE TO RELATED APPLICATION

The present disclosure claims the priority of the following Chinese patent applications submitted to CNIPA on November 4, 2022, with application numbers 202211379985.4 (entitled: Model Selection Method for Negative Pressure Face Mask Assembly and Model Selection Apparatus), 202222948281.6 (entitled: Model Selection Apparatus for Negative Pressure Face Mask), 202211379575.X (entitled: Negative Pressure Treatment Apparatus), 202222948215.9 (entitled: Negative Pressure Face Mask and Negative Pressure Therapy Apparatus), and 202222948733.0 (entitled: Headband for Fixing Negative Pressure Face Mask to Patient's Underjaw and Negative Pressure Ventilation Apparatus), the entire contents of which are incorporated into this application by reference.

### FIELD

The present disclosure relates to the technical field of negative pressure therapy, in particular to a model selection method for a negative pressure face mask assembly and a model selection apparatus.

### BACKGROUND

A non-invasive negative pressure ventilation apparatus, e.g., a negative pressure therapy apparatus, is effective therapy means for snoring. It usually includes a negative pressure face mask assembly that surrounds the patient's neck, a negative pressure pump that provides continuous suction forces, and a hose that connects the negative pressure face mask assembly and the negative pressure pump. When in use, the negative pressure face mask assembly surrounds the outside of the patient's respiratory tract (e.g., below the underjaw and around the neck). After the negative pressure pump is turned on, a continuous negative pressure causes an air pressure outside the patient's respiratory tract to be less than an air pressure inside the respiratory tract. The patient's respiratory tract is opened under an air pressure difference, which effectively prevents snoring. The negative pressure therapy apparatus is widely applied in obstructive sleep apnea (OSA).

There are usually two ways in which a patient can wear a negative pressure face mask assembly: 1, self-adhesive glue is arranged on the negative pressure face mask assembly, and the negative pressure face mask assembly is bonded to the patient's neck through the self-adhesive glue; and 2, the negative pressure face mask assembly is fixed by a neckband that wraps around the patient's neck only. However, the above two ways are less comfortable to wear, and there is no guarantee of good seal between the patient and the negative pressure face mask assembly.

The negative pressure face mask assemblies vary depending on different body structures of different patients. It is necessary to select appropriate negative pressure face mask assemblies according to the body structures of different patients in order to carry out effective therapy. The existing model selection method for a negative pressure face mask assembly is usually to measure the size of the patient's neck, the height from the underjaw to the top of the chest, etc., with a measuring tape, and determine a model of the selected negative pressure face mask assembly according to measurements of a plurality of sizes. However, this method cannot accurately determine which type of negative pressure face mask assembly is suitable for the patient, resulting in the difficulty in model selection for the negative pressure face mask assembly.

Moreover, different models of negative pressure face mask assemblies need to be selected according to the patient's body structures to meet the patient's needs. The usual model selection method is used for the patient to try out different models of negative pressure face mask samples to determine which model is suitable. However, this model selection method is not convenient, and the reuse of negative pressure face mask samples can easily lead to disease transmission.

Moreover, the negative pressure pump is usually operated artificially. This method cannot maintain the stability of the air pressure in the negative pressure face mask, which causes discomfort for the therapy of the patient.

Moreover, when the negative pressure pump provides an excessive suction force due to mechanical failure or artificial misoperation, if the suction force is greater than the tolerance of the human body for long-term wear, damages such as skin strain in a part (e.g., the neck) of the patient to wear will be caused.

Therefore, a new technical solution is required to solve the above technical problems.

### SUMMARY

An objective of the present disclosure is to provide a new technical solution for a model selection method for a negative pressure face mask assembly.

According to an aspect of the present disclosure, a model selection method for a negative pressure face mask assembly is provided. The method includes: obtaining a gender of a patient, wherein under the condition that the patient is a female, an inner surface of the negative pressure face mask assembly forms a cambered surface structure, under the condition that the patient is a male, the inner surface of the negative pressure face mask assembly forms a cambered surface structure, and a make-way notch or a flexible component is arranged in the middle of the cambered surface structure; obtaining an interval distance between both earlobes of the patient, and determining a width of the negative pressure face mask assembly according to the interval distance; obtaining a perimeter of the patient's neck, and determining a curative of a side of the negative pressure face mask assembly in contact with the patient according to the perimeter; and determining a model of the negative pressure face mask assembly according to the width and the curative.

Optionally, the method further includes: obtaining a first included angle formed between the underjaw and the neck of the patient when the patient views at eye level; and determining a model of the negative pressure face mask assembly according to the width, the curative and the first included angle.

Optionally, the method further includes: obtaining a second included angle formed between the underjaw and the neck of the patient when the patient is looking up; and determining a model of the negative pressure face mask assembly according to the width, the curative, the first included angle and the second included angle.

Optionally, the model of the negative pressure face mask assembly determined according to the interval distance is taken as a standard in the case that the model of the negative pressure face mask assembly determined according to the interval distance is inconsistent with a model of the negative pressure face mask assembly determined according to the perimeter.

Optionally, the model of the negative pressure face mask assembly determined according to the perimeter is taken as a standard in the case that the model of the negative pressure face mask assembly determined according to the perimeter is inconsistent with the model of the negative pressure face mask assembly determined according to the second included angle.

Optionally, the model of the negative pressure face mask assembly determined according to the second included angle is taken as a standard in the case that the model of the negative pressure face mask assembly determined according to the first included angle is inconsistent with the model of the negative pressure face mask assembly determined according to the second included angle.

Optionally, the models of the negative pressure face mask assemblies corresponding to the genders of the patient include first-type negative pressure face mask assemblies, second-type negative pressure face mask assemblies, and third-type negative pressure face mask assemblies; a size of the first-type negative pressure face mask assembly is greater than that of the second-type negative pressure face mask assembly, and a size of the second-type negative pressure face mask assembly is greater than that of the third-type negative pressure face mask assembly; the interval distance between the first-type negative pressure face mask assemblies is greater than the interval distance between the second-type negative pressure face mask assemblies, and the interval distance between the second-type negative pressure face mask assemblies is greater than the interval distance between the third-type negative pressure face mask assemblies; and the curvature of the first-type negative pressure face mask assembly is greater than the curvature of the second-type negative pressure face mask assembly, and the curvature of the second-type negative pressure face mask assembly is greater than the curvature of the third-type negative pressure face mask assembly.

According to a second aspect of the present disclosure, a model selection apparatus applied to the above model selection method is provided. The apparatus includes a sheet body, wherein the sheet body is provided with a plurality of notches each having an arc-shaped structure; the plurality of notches have different sizes to correspond to different types of negative pressure face mask assemblies, respectively; and both ends of each notch are used to suit both earlobes of the patient respectively to determine the model of the negative pressure face mask assembly.

Optionally, the sheet body includes a first part and a second part which are connected together; the first part and the second part can be folded in half to form a folded line therebetween; the notches corresponding to a male patient are located on one side of the first part away from the folded line; and the notches corresponding to a female patient are located on one side of the second part away from the folded line.

According to a third aspect of the present disclosure, a model selection apparatus applied to the above model selection method is provided. The apparatus includes a sheet structure, wherein an edge of the sheet structure is provided with a plurality of model selection protrusions that protrude outward; each model selection protrusion includes a first side and a second side which are arranged at a set included angle; the plurality of model selection protrusions are arranged at different set included angles to correspond to different types of negative pressure face mask assemblies, respectively; and the model selection protrusions are used to be clamped between the underjaw and the neck of the patient to determine the model of the negative pressure face mask assembly.

Optionally, the sheet structure includes a third part and a fourth part which are connected together; the third part and the fourth part can be folded in half to form a folded line therebetween; the model selection protrusions corresponding to the male patient are located on one side of the third part away from the folded line; and the model selection protrusions corresponding to the female patient are located on one side of the fourth part away from the folded line.

In a fourth aspect of the present disclosure, a model selection apparatus applied to the above model selection method is provided. The apparatus includes a sheet body, wherein a plurality of model selection protrusions are arranged in the sheet body; the model selection protrusions are connected to the sheet body through easy-to-tear parts; the plurality of model selection protrusions are arranged at different set included angles to correspond to different types of negative pressure face mask assemblies, respectively; and the model selection protrusions are used to be clamped between the underjaw and the neck of the patient to determine the model of the negative pressure face mask assembly.

Optionally, the model selection protrusions can be bent to protrude from the sheet body toward side surfaces; or the model selection protrusions can be withdrawn from the sheet body.

Optionally, the model selection protrusions are of a triangular structure, and an angle of the triangular structure that is used to contact with the patient forms a chamfer.

Optionally, each easy-to-tear part includes an easy-to-tear strip; and the model selection protrusions are of a triangular structure, and two sides of the triangular structure which are used to contact with the patient are connected to the sheet body through the easy-to-tear strips.

According to a fifth aspect of the present disclosure, a model selection apparatus for a negative pressure face mask assembly is provided. The model selection apparatus includes: a first module, configured to acquire an interval distance between both earlobes of a patient; a second module, configured to acquire a perimeter of the patient's neck; and a processor, configured to acquire the interval distance and the perimeter; determine a width of the negative pressure face mask assembly according to the interval distance; determine a curvature of a side of the negative pressure face mask assembly in contact with the patient according to the perimeter; and determine a model of the negative pressure face mask assembly according to the width and the curvature.

Optionally, the model selection apparatus further includes: a third module configured to acquire a first included angle formed between the underjaw and the neck of the patient when the patient views at eye level; and the processor is further configured to determine a model of the negative pressure face mask assembly according to the width, the curative and the first included angle.

Optionally, the third module is further configured to acquire a second included angle formed between the underjaw and the neck of the patient when the patient is looking up; and the processor is further configured to determine the model of the negative pressure face mask assembly according to the width, the curvature, the first included angle and the second included angle.

Optionally, the processor is further configured to take the model of the negative pressure face mask assembly determined according to the interval distance as a standard in the case that the model of the negative pressure face mask assembly determined according to the interval distance is inconsistent with a model of the negative pressure face mask assembly determined according to the perimeter.

Optionally, the processor is further configured to take the model of the negative pressure face mask assembly determined according to the perimeter as a standard in the case that the model of the negative pressure face mask assembly determined according to the perimeter is inconsistent with the model of the negative pressure face mask assembly determined according to the second included angle.

Optionally, the processor is further configured to take the model of the negative pressure face mask assembly determined according to the second included angle as a standard in the case that the model of the negative pressure face mask assembly determined according to the first included angle is inconsistent with the model of the negative pressure face mask assembly determined according to the second included angle.

Optionally, the model selection apparatus further includes a gender obtaining module, configured to obtain a gender of a patient; and the processor is further configured to, under the condition that the patient is a female, an inner surface of the negative pressure face mask assembly forms a cambered surface structure; and under the condition that the patient is a male, the inner surface of the negative pressure face mask assembly forms a cambered surface structure, and a make-way notch or a flexible component is arranged in the middle of the cambered surface structure.

According to a sixth aspect of the present disclosure, a headband is provided. The headband is applied to a negative pressure ventilation apparatus; and the headband includes a headband body, wherein the headband body is constructed to cover a head top and an occipital bone of a patient, and a hollow structure is formed in the middle of the headband body; and a plurality of connecting bands, wherein one end of each connecting band is connected to an edge of the headband body, the at least one connecting band is provided with a headband fixing structure, a part of the at least one connecting band that is connected to the headband body is located behind the patient's ears, the plurality of connecting bands extend toward the patient's underjaw and are symmetrically arranged relative to the headband body, and the headband fixing structure is connected to a negative pressure face mask.

According to a seventh aspect of the present disclosure, a negative pressure face mask is provided. The negative pressure face mask includes: a main body and a safety valve connected to the main body, wherein the safety valve includes a mounting cylinder and a sealing member; the mounting cylinder includes an internal channel having an internal port and an external port; the sealing member is arranged at the external port; the sealing member includes a self-sealing sheet and a sealing opening; the self-sealing sheet protrudes outward from an internal channel; the sealing opening is formed in the self-sealing sheet; the self-sealing sheet can seal the sealing opening to seal the internal channel; and the self-sealing sheet opens the sealing opening when an air pressure difference between the inside and the outside of the internal channel exceeds a critical pressure.

According to an eighth aspect of the present disclosure, a negative pressure therapy apparatus is provided. The negative pressure therapy apparatus includes a negative pressure generation apparatus. The negative pressure generation apparatus includes: a housing, wherein a chamber is formed in the housing, an air extraction pipe communicated with the chamber is arranged on the housing, and an airway valve is arranged on the air extraction pipe; and a suction apparatus, wherein the suction apparatus is arranged in the housing, and the suction apparatus is configured to suction air from the chamber; and under the condition that the pressure intensity in the negative pressure face mask is greater than or equal to a first threshold, the airway valve is opened.

According to a ninth aspect of the present disclosure, a model selection apparatus for a negative pressure face mask is provided. The apparatus is used to measure an included angle from an underjaw to a neck of a patient; and the model selection apparatus includes: a first arm; a second arm, wherein one end of the second arm is rotatably connected to one end of the first arm, the first arm and the second arm are respectively used to contact with the underjaw and the neck of the patient, and an interval distance from the second arm to a center is greater than an interval distance from the first arm to the center; and a scaleplate, wherein the scaleplate is connected to the other end of the first arm, a model selection scale line is arranged on the scaleplate, the second arm can swing along the scaleplate relative to the first arm so that the first arm and the second arm are arranged at a set included angle, and the model of the negative pressure face mask is determined according to a position of the second arm on the model selection scale line.

The headband provided by the present disclosure can ensure good seal between the negative pressure face mask assembly and the patient and is comfortable to wear.

According to the negative pressure face mask provided by the present disclosure, the sealing member includes a self-sealing sheet and a sealing opening. Moreover, the self-sealing sheet protrudes outward from the internal channel. A negative pressure is formed in the internal channel. Under an action of an air pressure difference between inside and outside, the self-sealing sheet is extruded inward. At this time, the sealing opening is well sealed under mutual extrusion of the self-sealing sheets. When the air pressure difference exceeds a critical pressure, the self-sealing sheet can be elastically deformed under the action of pressure, and then the sealing opening is opened inward to allow outside air to enter, thereby ensuring that the air pressure in a component where the safety valve is mounted does not exceed the critical pressure. The negative pressure face mask can effectively protect the patient.

The negative pressure face mask has a simple structure, is easy to machine, and can achieve a function of automatic opening when the air pressure difference exceeds a critical air pressure and automatic closing when the air pressure difference does not exceed the critical air pressure without requiring electromagnetic drive or pressure drive.

Moreover, since the self-sealing sheet can be elastically deformed to open the sealing opening, a detection probe can be inserted through the safety valve to detect the air pressure, temperature and other parameters inside a component where the safety valve is located. After the probe is pulled out, the self-sealing sheet returns to a position where the sealing opening is closed, thereby ensuring that the sealing opening is leak-proof.

The negative pressure therapy apparatus provided by the present disclosure includes a negative pressure generation apparatus. The negative pressure generation apparatus is provided with an airway valve, and the airway valve can be opened by controlling the airway valve to make the pressure intensity in the negative pressure face mask greater than or equal to the first threshold, such that the negative pressure face mask can be vacuumized for the therapy of the patient. This method can maintain the stability of the air pressure in the negative pressure face mask, thereby improving the comfort of the patient in the therapy.

According to the model selection apparatus provided by the present disclosure, the size of an included angle between the underjaw and the neck of the patient can be determined by the swing of the second arm relative to the first arm and the position of the second arm on the model selection scale line, and the model of the negative pressure face mask can be determined according to the size of the included angle.

The apparatus has the characteristics of being easy to use and carry, and high measurement accuracy.

Moreover, the negative pressure face mask can be made on the basis of the size of the included angle, such that the negative pressure face mask can be made more accurately.

The model selection method provided by the present disclosure is able to precisely determine the model of the negative pressure face mask assembly that suits the patient.

By the detailed description of the exemplary embodiments of the present disclosure with reference to the following accompanying drawings, other features and advantages of the present disclosure will become more apparent.

### BRIEF DESCRIPTION OF THE DRAWINGS

The accompanying drawings that constitute a part of the description describe the embodiments of the present disclosure, and are used together with the description to explain the principles of the present disclosure.
FIG. 1 is a perspective view of a liner assembly according to an embodiment of the present disclosure.
FIG. 2A, FIG. 2B, FIG. 2C, FIG. 2D, FIG. 2E, FIG. 2F, FIG. 2G, and FIG. 2H are sectional views of liner assemblies of various structures in FIG. 1 along a section A.
FIG. 3A and FIG. 3B are schematic diagrams of sealing between a liner assembly and a patient's skin.
FIG. 4A is an exploded view of another liner assembly according to an embodiment of the present disclosure.
FIG. 4B is a sectional view of the liner assembly shown in FIG. 4A along a section B.
FIG. 5A, FIG. 5B, FIG. 5C, and FIG. 5D are schematic diagrams of assembling a soft rubber part and a cup according to an embodiment of the present disclosure.
FIG. 5E is a diagram showing an assembly principle of the soft rubber part and the cup shown in FIG. 5D.
FIG. 6A, FIG. 6B and FIG. 6C are schematic structural diagrams of a frame according to an embodiment of the present disclosure.
FIG. 7A and FIG. 7B are schematic structural diagrams of an elbow according to an embodiment of the present disclosure.
FIG 7C and FIG. 7D are schematic structural diagrams of a hose according to an embodiment of the present disclosure.
FIG. 8A is a schematic structural diagram of a negative pressure face mask assembly according to an embodiment of the present disclosure.
FIG. 8B, FIG. 8C, and FIG. 8D are sectional views of negative pressure face mask assemblies of various structures in FIG. 8A along a section C.
FIG. 9A is a schematic structural diagram of a liner assembly provided with a groove structure according to an embodiment of the present disclosure.
FIG. 9B and FIG. 9C are sectional views of the liner assembly shown in FIG. 9A along a section D.
FIG. 9D is a schematic structural diagram of another liner assembly provided with a groove structure according to an embodiment of the present disclosure.
FIG. 9E, FIG. 9F, FIG. 9G, FIG. 9H, FIG. 9I, FIG. 9J, FIG. 9K, and FIG. 9L are sectional views of liner assemblies of various structures in FIG. 9D.
FIG. 9M and FIG. 9N are schematic diagrams of sealing between a liner assembly sealing the patient's skin according to an embodiment of the present disclosure.
FIG. 10A is a schematic diagram of a first headband according to an embodiment of the present disclosure.
FIG. 10B is a schematic diagram of a second headband according to an embodiment of the present disclosure.
FIG. 10C is a schematic diagram of a third headband according to an embodiment of the present disclosure;
FIG. 10D is a schematic diagram of a fourth headband according to an embodiment of the present disclosure.
FIG. 10E is a schematic diagram of a fourth headband according to an embodiment of the present disclosure from another perspective.
FIG. 11A is an assembly diagram of a headband fixing structure, a connecting band and a frame according to an embodiment of the present disclosure.
FIG. 11B is an exploded view of FIG. 11A.
FIG. 12A is an assembly diagram of a self-adhesive film, a negative pressure face mask assembly and a hose according to an embodiment of the present disclosure.
FIG. 12B is a schematic diagram of a self-adhesive film according to an embodiment of the present disclosure.
FIG. 12C is an assembly diagram of a self-adhesive film, a negative pressure face mask assembly and a hose according to an embodiment of the present disclosure from another perspective.
FIG. 12D is a sectional view of FIG. 12C.
FIG. 13A is a schematic diagram of a liner assembly provided with an air permeable layer according to an embodiment of the present disclosure.
FIG. 13B is a sectional view of FIG. 13A along a section E.
FIG. 13C is a partially enlarged view of FIG. 13B.
FIG. 13D and FIG. 13E are partially sectional views of a soft rubber part and a cup.
FIG. 14A is a perspective diagram of a safety valve according to an embodiment of the present disclosure.
FIG. 14B is a sectional view of a safety valve according to an embodiment of the present disclosure.
FIG. 14C is an assembly diagram of a safety valve and a tri-branch tube according to an embodiment of the present disclosure.
FIG. 14D is a partially sectional view of FIG. 14C.
FIG. 14E is an assembly diagram of a safety valve and a negative pressure face mask assembly according to an embodiment of the present disclosure.
FIG. 15A, FIG. 15B, FIG. 15C, FIG. 15D, and FIG. 15E are schematic diagrams of measurements for a patient according to an embodiment of the present disclosure.
FIG. 15F is a flowchart of model selection for a negative pressure face mask assembly according to an embodiment of the present disclosure.
FIG. 15G is a schematic structural diagram of a caliper according to an embodiment of the present disclosure.
FIG. 15H is a schematic diagram of a measuring tape according to an embodiment of the present disclosure.
FIG. 15I are a schematic structural diagram of another caliper according to an embodiment of the present disclosure.
FIG. 15J is a schematic structural diagram of a model selection apparatus for a negative pressure face mask assembly according to an embodiment of the present disclosure.
FIG. 15K is a schematic structural diagram of another model selection apparatus for a negative pressure face mask assembly according to an embodiment of the present disclosure.
FIG. 15L is a rear view of FIG. 15K.
FIG. 15M is a schematic diagram of the assembly of a first arm and a scaleplate in FIG. 15K.
FIG. 15N is a schematic structural diagram of another model selection apparatus for a negative pressure face mask assembly according to an embodiment of the present disclosure.
FIG. 15O is a schematic diagram of the assembly of a first arm and a scaleplate in FIG. 15N.
FIG. 15P is a schematic structural diagram of another model selection apparatus for a negative pressure face mask assembly according to an embodiment of the present disclosure.
FIG. 15Q is a schematic structural diagram of another model selection apparatus for a negative pressure face mask assembly according to an embodiment of the present disclosure.
FIG. 16A is a perspective diagram of a negative pressure generation apparatus according to an embodiment of the present disclosure.
FIG. 16B is a decompostion view of a negative pressure generation apparatus according to an embodiment of the present disclosure.
FIG. 16C is a principle diagram of a negative pressure generation apparatus according to an embodiment of the present disclosure.
FIG. 16D is a schematic diagram of a negative pressure therapy apparatus according to an embodiment of the present disclosure.
FIG. 16E is a flowchart of a control method for a negative pressure therapy apparatus according to an embodiment of the present disclosure.

### DETAILED DESCRIPTION OF THE EMBODIMENTS

The exemplary embodiments of the present disclosure are described now in detail below with reference to the accompanying drawings. It should be noted that, unless specified otherwise, the relative arrangements, the mathematical formulas and numerical values of the components and steps described in these embodiments do not limit the scope of the present disclosure.

The following descriptions for at least one exemplary embodiment are actually descriptive only, and shall not be intended to limit the present disclosure and any application or use thereof.

The techniques and devices well known to a person skilled in the related arts may not be discussed in detail where applicable, such techniques and devices should be deemed as a part of the description.

Among all the examples shown and discussed here, any specific values should be interpreted as merely exemplary and not as limitations. Therefore, other examples of the exemplary embodiments may have different values.

It should be noted that similar signs and letters in the following drawings represent similar items. Therefore, once defined in one drawing, an item may not be further discussed in the followed drawings.

### <I. Negative pressure therapy apparatus>

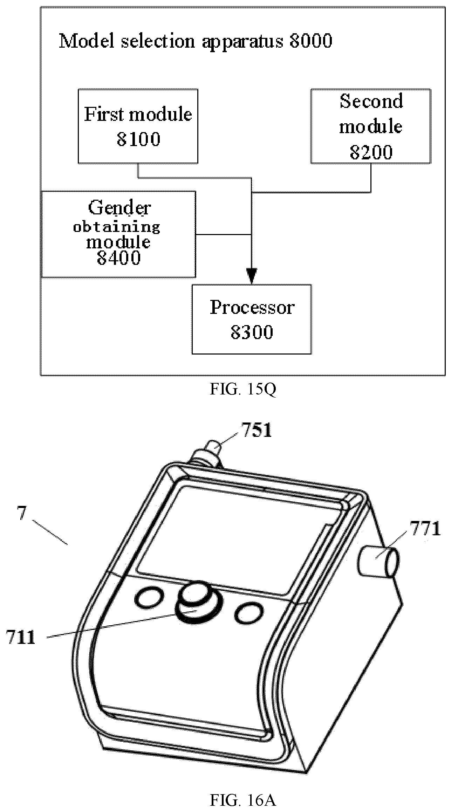

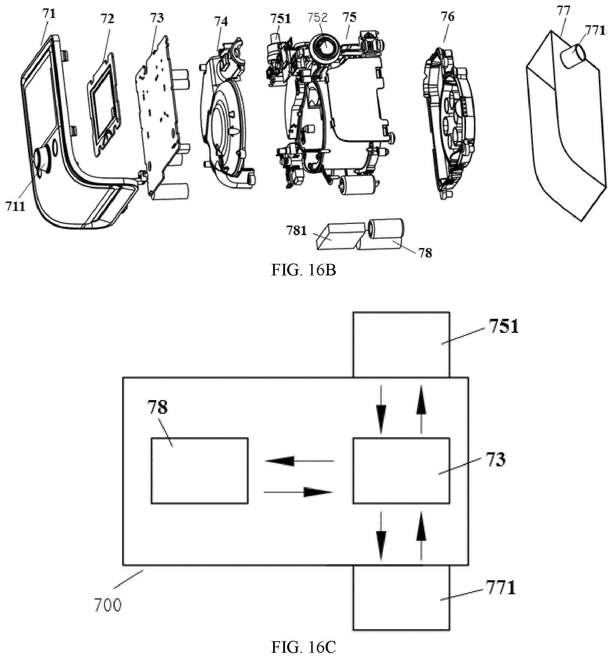

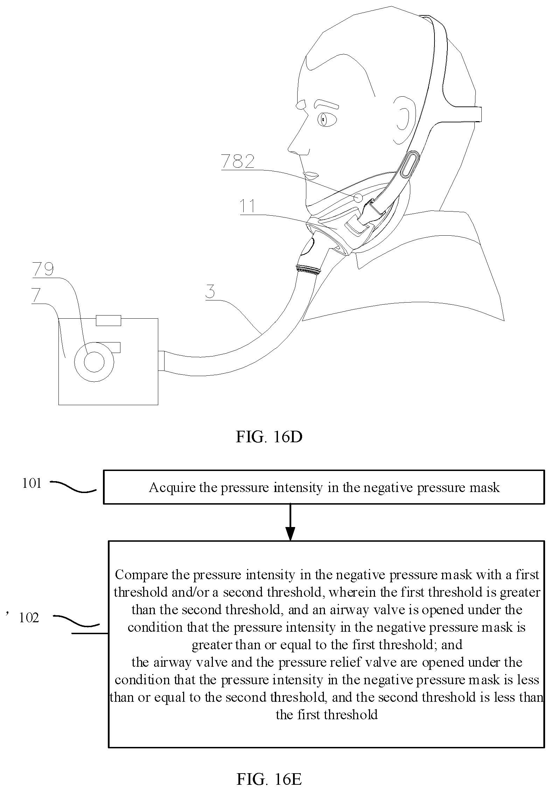

As shown in FIG. 16D, a negative pressure therapy apparatus includes a negative pressure face mask assembly, a hose 4, a headband 5, an air extraction pipe 6, a negative pressure generation apparatus 7, etc. The negative pressure face mask assembly includes a liner assembly 1 and a frame 2.

In some embodiments, the negative pressure face mask assembly further includes an elbow 3, a hose, etc. The negative pressure face mask assembly is used to contact with a patient. For example, the liner assembly 1 comes in contact with the patient's skin and forms a sealed negative pressure chamber at the underjaw and the neck of the patient. The liner assembly 1 is of a mask-shaped structure with an opening. The opening is used to be docked with the patient and create a negative pressure chamber. A first mounting hole 13 is formed in the middle of the liner assembly. The first mounting hole 13 extends toward a direction away from the opening. The first mounting hole 13 is used to connect to the elbow 3 or the hose 4.

The frame 2 is arranged outside the liner assembly 1, e.g., sleeved outside the first mounting hole 13. The frame 2 provides a supporting force for the liner assembly 1 to ensure the stability of the negative pressure chamber.

The elbow 3 is connected between the liner assembly 1 and the negative pressure generation apparatus 7 to form an air channel. Alternatively, the hose 4 is directly connected between the liner assembly 1 and the negative pressure generation apparatus to form an air channel. Alternatively, the elbow 3 is connected to the hose 4. The elbow 3 is connected to the liner assembly 1. The hose 4 is connected to the negative pressure generation apparatus 7.

The headband 5 fixes the negative pressure face mask assembly around the patient's neck. The negative pressure generation apparatus 7 extracts air from the negative pressure chamber, so that an air pressure in the negative pressure chamber is less than an outside air pressure of the liner assembly 1.

### <II. Liner assembly>

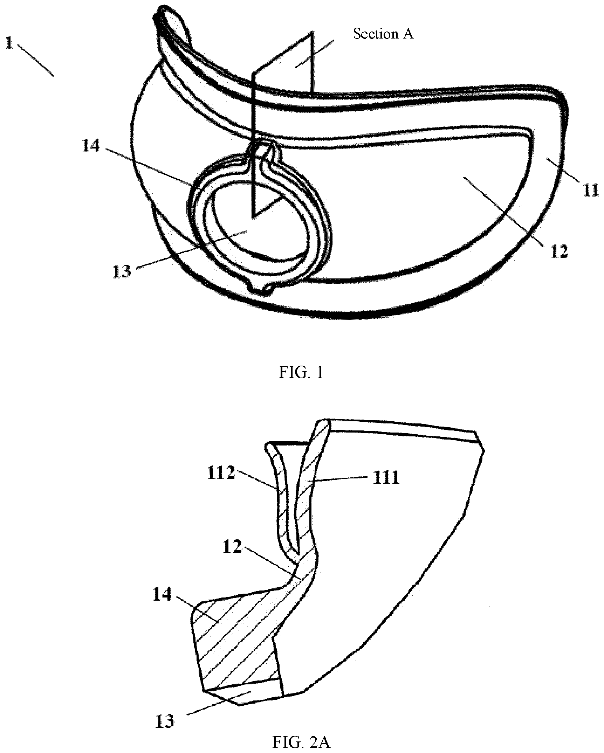

An embodiment of the present disclosure provides a liner assembly 1 applied to a negative pressure therapy apparatus. As shown in FIG. 1, the liner assembly 1 is used to come in contact with the patient. The liner assembly 1 is of a bilateral symmetry structure as a whole. The liner assembly 1 includes a soft rubber part 11, a cup 12, and a first mounting hole 13. The cup 12 is of a mask-shaped structure. The cup 12 is arranged around the first mounting hole 13 and connected to one end of the first mounting hole 13. The first mounting hole 13 is connected to the elbow 3 or the hose 4. The soft rubber part 11 is arranged around the cup 12 and connected to an edge of the cup 12.

Optionally, the soft rubber part 11 is made of a soft material. The soft material may be, but not limited to, silicone, gel, TPE, TPU, etc. When worn, the soft rubber part 11 comes in direct contact with the patient's skin. A structural strength of the cup 12 is greater than that of the soft rubber part 11. For example, a hardness of the cup 12 is greater than that of the soft rubber part 11. The cup 12 may be made of PC, PP, etc. That is, the cup 12 is made of the same material as the soft rubber part 11, but a thickness of the cup 12 is greater than that of the soft rubber part 11. In this way, the structural strength of the cup 12 is greater than that of the soft rubber part 11.

Further, the cup 12 has a set rigidity to keep its shape fixed. When in use, a negative pressure chamber is formed between the cup 12 and the patient's skin. For example, a stiffness of the cup 12 can withstand a pressure intensity difference of more than 20 hpa between inside and outside of the negative pressure chamber, while its shape is maintained.

The first mounting hole 13 is fixedly connected to the cup 12. For example, the first mounting hole 13 is located in the middle of the cup 12 and extends to a side away from the negative pressure chamber. Certainly, the first mounting hole may also be located in a middle left or right position of the cup 12. The first mounting hole 13 is used to mount the elbow 3 or the hose 4, and communicated with the negative pressure generation apparatus 7 by the elbow 3 and/or the hose 4. The first mounting hole 13 may also be used to mount the frame 2. For example, an outer wall of the first mounting hole 13 is provided with mounting protrusions 14. The mounting protrusions 14 are arranged circumferentially around the first mounting hole 13. Fixing holes are formed in the middle of the frame 2. The fixing holes are clamped in the mounting protrusions 14, so that the frame 2 is fixed outside the first mounting hole 13. The frame 2 is connected to the headband 5, so that the liner assembly 1 comes in close contact with the patient's skin, and the negative pressure chamber is formed between the liner assembly 1 and the patient.

In one example, the soft rubber part 11 includes a plurality of soft rubber layers. The plurality of soft rubber layers include a first soft rubber layer 111 and a second soft rubber layer 112. The first soft rubber layer 111 and the second soft rubber layer 112 each have a first side and a second side opposite each other along a width direction. The first side of the first soft rubber layer 111 and the first side of the second soft rubber layer 112 are connected to the cup 12, and the second side of the first soft rubber layer 111 and the second side of the second soft rubber layer 112 extend outward. The first soft rubber layer 111 is used to come in contact with the patient's skin, and the second soft rubber layer 112 is located outside the first soft rubber layer 111. The first soft rubber layer 111 and the second soft rubber layer 112 are spaced from each other. The second side of the second soft rubber layer 112 form a support for the first soft rubber layer 111, so that the first soft rubber layer 111 forms a good sealing effect with the patient's skin.

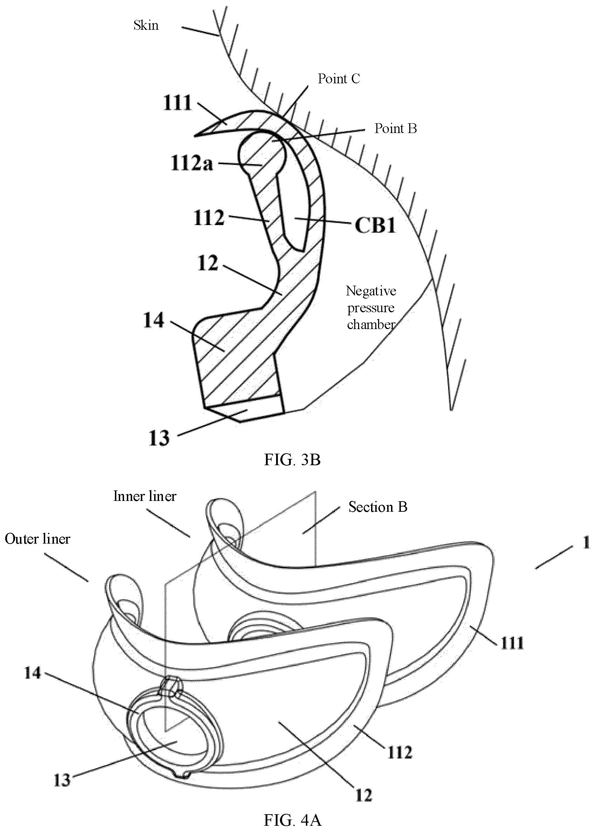

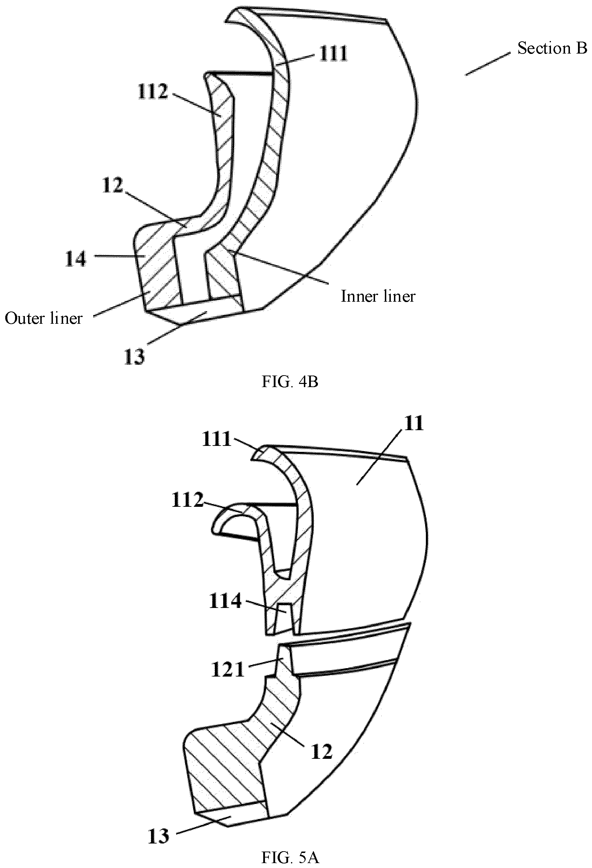

In another example, as shown in FIG. 4A and FIG. 4B, the liner assembly 1 includes an inner liner 11a and an outer liner 11b. For example, the inner liner 11a and the outer liner 11b each include pipe sections 13a, 13b located in the middle, mask bodies 12a, 12b arranged around the pipe sections 13a, 13b, and edge parts arranged around the mask bodies 12a, 12b. The pipe sections 13a, 13b and the mask bodies 12a, 12b are connected to the edge parts together. At least one of the pipe sections 13a, 13b and the mask bodies 12a, 12b is made of a rigid material, and the edge parts are made of a soft material. Alternatively, the pipe sections 13a, 13b, the mask bodies 12a, 12b and the edge parts are made of a soft material, respectively; and the thickness of the pipe sections 13a, 13b and the mask bodies 12a, 12b is greater than the thickness of the edge parts, so that the structural strength of the pipe sections 13a, 13b and the mask bodies 12a, 12b is greater than that of the soft edge parts.

The inner liner 11a and the outer liner 11b are stacked and connected together. For example, the inner liner 11a and the outer liner 11b are connected together by means of bonding, clamping or hot melting. The mask body 12a of the inner liner 11a and the mask body 12b of the outer liner 11b are combined together, e.g., completely fitted or partially fitted to form the cup 12. The pipe section 13a of the inner liner 11a and the pipe section 13b of the outer liner 11b are combined together (e.g., the both are connected), to form the first mounting hole 13. The first soft rubber layer 111 is located at the edge part of the inner liner 11a. The second soft rubber layer 112 is located at the edge part of the outer liner 11b. The pipe section of the outer liner 11b is provided with mounting protrusions 14.

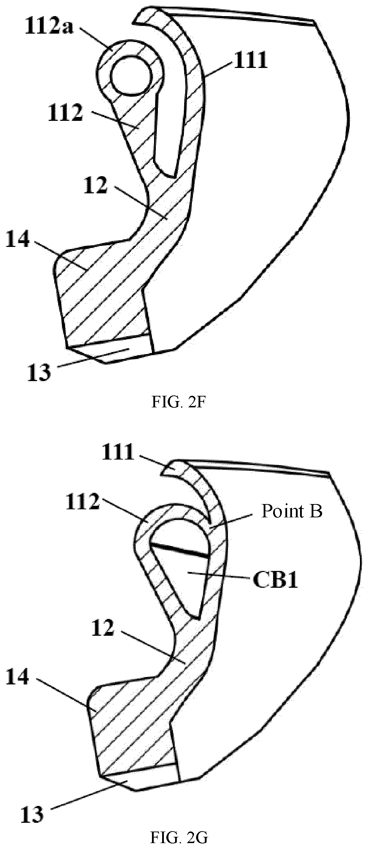

Optionally, the thickness of the second soft rubber layer 112 is larger than that of the first soft rubber layer 111, as shown in FIG. 2A. If the thickness of the second soft rubber layer 112 is large, the structural strength is high, so that a good support for the first soft rubber layer 111 is formed and the first soft rubber layer 111 is kept to be well sealed with the patient's skin. If the thickness of the first soft rubber layer 111 is small, and an elastic deformation can occur, to maintain a good seal with the patient's skin.

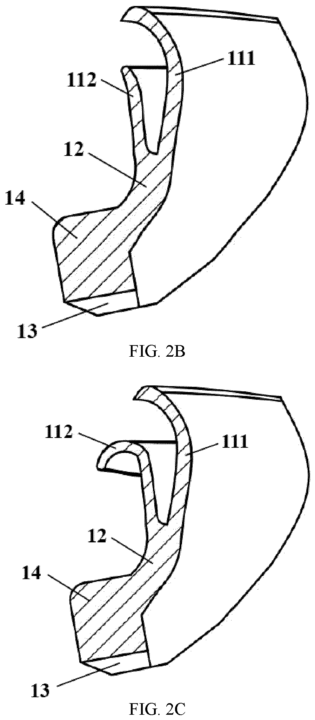

Optionally, the width of the second soft rubber layer 112 is less than that of the first soft rubber layer 111, as shown in FIG. 2B. In this example, the second side of the second soft rubber layer 112 is directly opposite the middle position of the first soft rubber layer 111. The first soft rubber layer 111 has a redundant part relative to the second soft rubber layer 112. The redundant part can increase a contact area between the first soft rubber layer 111 and the patient's skin, to maintain a good seal between the first soft rubber layer 111 and the patient's skin.

Optionally, the second side of the second soft rubber layer 112 is bent away from a side of the first soft rubber layer 111, as shown in FIG. 2C. The second side forms a cambered surface that extends outward. In this example, a distance between the second soft rubber layer 112 and the first soft rubber layer 111 is large, and an activity space for the first soft rubber layer 111 is large. Moreover, when a support is formed, an overlapping area and a contact area between the second soft rubber layer 112 and the first soft rubber layer 111 are both large, so that a sealing area between the first soft rubber layer 111 and the patient's skin is large, leading to a better sealing effect.

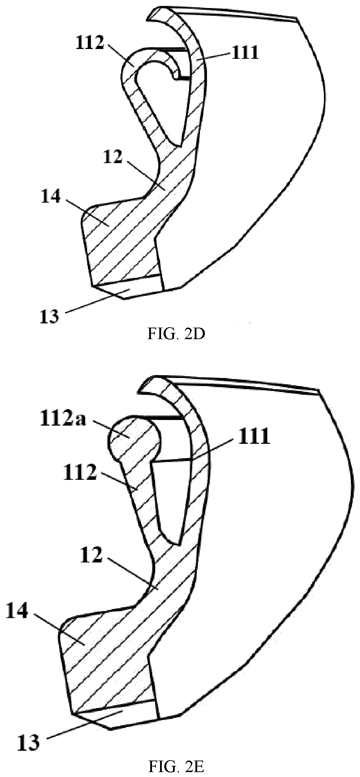

Optionally, the second side of the second soft rubber layer 112 is bent close to a side of the first soft rubber layer 111, as shown in FIG. 2D. In this example, a distance between the second soft rubber layer 112 and the first soft rubber layer 111 is small, and an activity space for the first soft rubber layer 111 is small. Moreover, an overlapping area and a contact area between the second soft rubber layer 112 and the first soft rubber layer 111 after bending are both large, so that a structural support is formed for the first soft rubber layer 111 more effectively.

In one example, the second side of the second soft rubber layer 112 is provided with a flange 112a, as shown in FIG. 2E. In this example, the thickness of the flange 112a is greater than the thickness of a part other than the flange 112a of the second soft rubber layer 112, which makes the overall structural strength of the second soft rubber layer 112 higher, and a supporting force of the second soft rubber layer 112 to the first soft rubber layer 111 greater.

Further, the flange 112a forms a cambered surface close to a side of the first soft rubber layer 111. In this way, when worn, the flange 112a can stably support the first soft rubber layer 111, and the cambered surface can form a slidable fulcrum B, to adapt to movements of the patient's head.

Moreover, this makes the contact area between the second soft rubber layer 112 and the first soft rubber layer 111 larger, and the contact area between the first soft layer 111 and the patient's skin larger, so that the negative pressure chamber has a better sealing effect.

Optionally, a cross-section of the flange 112a is round, elliptical, etc. The flange 112a having the above shape can improve a sealing effect of the negative pressure chamber.

Further, a channel is arranged in the flange 112a along an extension direction of the flange 112a. The channel can reduce an overall weight of the flange 112a without weakening the overall structural strength of the second soft rubber layer 112, and then reduce the overall weight of the second soft rubber layer 112.

Moreover, the channel causes the flange 112a to undergo an elastic deformation when it is extruded by an external force, so that the first soft rubber layer 111 can adjust a local structure according to the structures of the neck and the underjaw of the patient, so that the first soft rubber layer 111 can be more suitable for the patient's skin.

Optionally, a cross-section of the channel is of a shape of circular, rectangular, trapezoidal, triangular, elliptical, semicircular, etc. The above structures can reduce the weight of the second soft rubber layer 112, and make the first soft rubber layer 111 fit the patient's skin.

In one example, the soft rubber part 11 has different sizes and shapes in different positions in a circumferential direction of the cup 12. For example, at a position in contact with the underjaw (a position of a section A in FIG. 1) of the patient, in order to well seal with the underjaw, the first soft rubber layer 111 is bent to a side away from the second soft rubber layer 112, as shown in FIG. 2A. The first soft rubber layer 111 at the patient' s neck is bent to a side close to the second soft rubber layer 112, as shown in FIG. 2B-FIG. 2H. In this way, the first soft rubber layer 111 can effectively contact the patient's skin.

Certainly, the sizes and shapes of the soft rubber part 11 in different positions in the circumferential direction of the cup 12 are not limited to the above embodiments, and may be selected by those skilled in the art according to actual needs.

In one example, as shown in FIG. 2G, a second end of the second soft rubber layer 112 is bent to a side close to the first soft rubber layer 111 and connected to the middle part of the first soft rubber layer 111 to form a fulcrum B, as shown in FIG. 2G. The fulcrum B can effectively support the first soft rubber layer 111, so that the first soft rubber layer 111 forms a good seal with the patient's skin.

Further, a first chamber CB1 is formed between the second soft rubber layer 112 and the first soft rubber layer 111. The first chamber CB1 allows a local deformation of the first soft rubber layer 111, to adapt to the structures of the underjaw and the neck of the patient, thereby maintaining a good sealing effect.

It should be noted that because the fulcrum B is immovable, a sealing point formed between the first soft rubber layer 111 and the patient's skin is also immovable, so the sealing cannot be dynamically adjusted according to patient's actions.

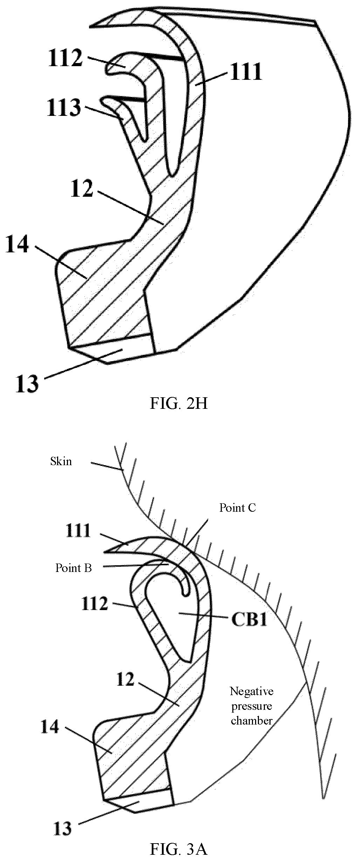

In order to solve the above-mentioned technical problems, in one embodiment, as shown in FIG. 2H, the soft rubber part 11 includes a first soft rubber layer 111, a second soft rubber layer 112, and a third soft rubber layer 113.

In one example, the soft rubber part can implementing a dynamically sealing, that is, the sealing is dynamically adjusted according to patient's actions. As shown in FIG. 3A and FIG. 3B, the liner assembly 1 forms a stable negative pressure chamber with the patient's skin. An air pressure inside the negative pressure chamber is lower than an air pressure outside the negative pressure chamber.

A seal, that is, a sealing point C, is formed at a contact point between the first soft rubber layer 111 of the soft rubber part 11 and the patient's skin. The second soft rubber layer 112 is supported outside the first soft rubber layer 111. The second soft rubber layer 112 provides a supporting force for the seal between the first soft rubber layer 111 and the patient's skin. A supporting point, that is, a fulcrum B, is formed between the second soft rubber layer 112 and the first soft rubber layer 111.

In this embodiment, when the sealing point C is opposite to the fulcrum B in position, that is, the sealing point C and the fulcrum B are respectively located inside and outside the same position of the first soft rubber layer 111, the supporting force of the second soft rubber layer 112 for the first soft rubber layer 111 acts on the patient's skin by the sealing point C. Under such condition, the sealing effect of the first soft rubber layer 111 with the patient's skin is the best. Conversely, if the sealing point C is not opposite to the fulcrum B in position, poor seal will be caused between the first soft rubber layer 111 and the patient's skin.

When the patient has head movements (e.g., nodding, head shaking), swallowing movements (e.g., movements of prominentia laryngea and laryngeal muscles) that cause the movements of the liner assembly 1, or when the liner assembly 1 is artificially dragged or shaken, the position of the sealing point C will change dynamically. In this case, if the position of the fulcrum B between the second soft rubber layer 112 and the first soft rubber layer 111 is fixed, as shown in FIG. 2G, or the soft rubber part 11 is of a single-layer structure, a relative position of the fulcrum B to the sealing point C can have a displacement, which can cause a sealing failure between the first soft rubber layer 111 and the patient's skin.

In a preferred embodiment, as shown in FIG. 3A and FIG. 3B, the second soft rubber layer 112 forms a support to the first soft rubber layer 111 at the fulcrum B. Under the support of the second soft rubber layer 112, the first soft rubber layer 111 forms extrusion and seal with the patient's skin at the sealing point C. The second end of the second soft rubber layer 112 is not connected to the first soft rubber layer 111. A first chamber CB1 is formed between the second soft rubber layer 112 and the first soft rubber layer 111.

When the patient moves the head, swallows, or is dragged artificially, the position of the sealing point C shifts. A contact point (that is, a fulcrum B) between the second soft rubber layer 112 and the first soft rubber layer 111 will be automatically shift on an outer surface of the first soft rubber layer 111. That is, the fulcrum B will be automatically adjusted in position with the shifting of the sealing point C, to maintain a good seal between the first soft rubber layer 111 and the patient's skin. In this way, the sealing point C and the fulcrum B are always kept inside or outside the same position of the first soft rubber layer 111 respectively, so that a dynamic sealing effect is achieved.

Further, when the liner assembly 1 is extruded, the first chamber CB1 provides a sufficient elastic deformation space for the second soft rubber layer 112 and the first soft rubber layer 111, thereby increasing wearing comfort of the liner assembly 1. Moreover, the first chamber CB1 provides a sufficient adjustment space for the second soft rubber layer 112 and the first soft rubber layer 111, thereby increasing a sealability of the liner assembly 1 with the patient's skin.

### <III. Connection modes between the cup and the soft rubber part>

An embodiment of the present disclosure provides connection modes of the cup 12 and the soft rubber part 11. The cup 12 and the soft rubber part 11 may be integrally molded with the same material, and the material may be, but not limited to, rubber, silicone, etc. The cup 12 and the soft rubber part which are integrally molded may be of structures shown in FIGs. 2A - 2H, FIG. 3A, FIG. 3B, and FIG. 4B. Certainly, the cup 12 and the soft rubber part may also be independently molded, and then the cup 12 and the soft rubber part 11 are connected together by means of connection.

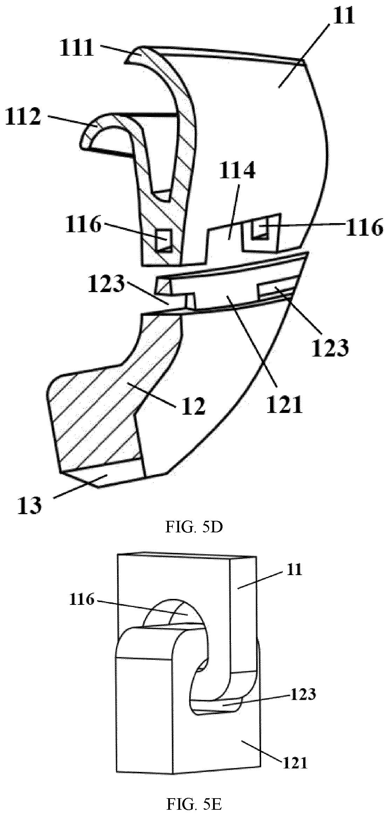

In one example, as shown in FIGs. 5A-5C, the cup 12 and the soft rubber part 11 are connected together by means of a mechanical assembly.

In one example, as shown in FIG. 5A, circumferentially extending grooves are formed in any one of an outer edge of the cup 12 and an inner edge of the soft rubber part 11, and circumferentially extending protrusions are arranged on the other one. For example, circumferentially extending first protrusions 121 are arranged on the cup 12. Circumferentially extending first grooves 114 are formed on the soft rubber part.

It should be noted that the first protrusions 121 may be arranged continuously around the cup 12, or the first protrusions 121 are partially arranged on the cup 12 in a circumferential direction, or a plurality of first protrusions 121 may be intermittently arranged on the cup 12 in the circumferential direction. The first groove 114 may be arranged continuously around the soft rubber part 11, or the first grooves 114 are partially formed in the soft rubber part 11 in a circumferential direction, or a plurality of first grooves 114 may be intermittently formed in the soft rubber part 11 in the circumferential direction.

During assembly, the first protrusions 121 are inserted into the first grooves 114 to realize the seal between the cup 12 and the soft rubber part 11. For example, the first protrusions 121 and the first grooves 114 are connected together by means of interference fit.

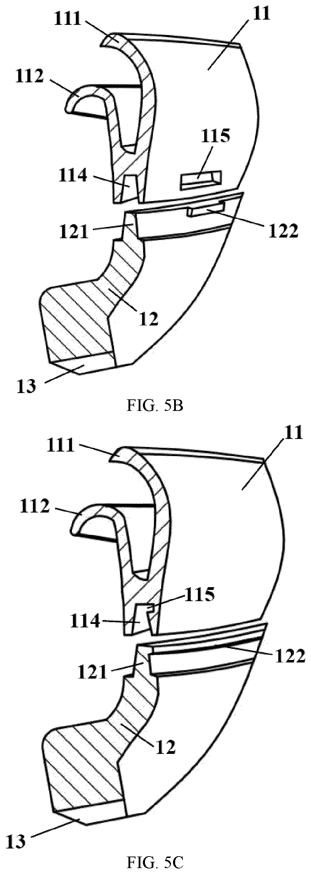

The soft rubber part 11 and the cup 12 together form a negative pressure chamber, and the sealability and stability between the soft rubber part 11 and the cup 12 are essential for maintaining the negative pressure in the negative pressure chamber. In order to achieve a more stable sealing effect, in one example, as shown in FIG. 5B and FIG. 5C, second protrusions 122 are arranged on the cup 12. The second protrusions 122 are arranged on lateral parts of the first protrusions 121. A protrusion direction of the second protrusions 122 is perpendicular or basically perpendicular to a protrusion direction of the first protrusions 121. For example, an included angle between the protrusion directions of the two types of protrusions is 80°-110°. Second grooves 115 are formed in the soft rubber part 11. Each second groove 115 is formed on a side wall of the first groove 114. A recessed direction of the second groove 115 is perpendicular or basically perpendicular to a recessed direction of the first grooves 114. For example, an included angle between the recessed directions of the two types of grooves is 80° -110°. During assembly, the first protrusions 121 form a clamping connection with the first grooves 114. Meanwhile, the second protrusions 122 form a clamping connection with the second grooves 115. Because the clamping connection between the second protrusions 122 and the second grooves 115 interferes with a separation of the first protrusions 121 from the first grooves 114, the separation of the cup 12 from the soft rubber part 11 can be effectively avoided, so that a sealing effect of the cup 12 and the soft rubber part 11 is improved.

Moreover, the first protrusions 121 form a clamping connection with the first grooves 114, and the second protrusions 122 form a clamping connection with the second grooves 115. Both of the clamping connections are detachable connections. When the cup 12 or the soft rubber part is aged and undamaged, any one of them can be replaced.

In other examples, a side wall of each first groove 114 is provided with the second protrusion 122. A side wall of each first protrusion 121 is provided with the second groove 115. During assembly, the first protrusions 121 form a clamping connection with the first grooves 114. Meanwhile, the second protrusions 122 form a clamping connection with the second grooves 115. In this way, this effect can also be achieved.

Optionally, as shown in FIG. 5C, the second protrusions 122 may be continuously arranged along one side wall of the first protrusion 121 or one side wall of the first groove 114 in a whole circle, or may be arranged intermittently. The second grooves 115 may be continuously arranged along one side wall of the first protrusion 121 or one side wall of the first groove 114 in a whole circle, or may be arranged intermittently.

Optionally, the second groove 115 is a through hole that penetrates through one side wall of the first groove 114 or a through hole that penetrates through two side walls of the first protrusion; or the second groove 115 is a non-penetrating hole.

In one example, the first protrusions 121 and the first grooves 114 are bonded by an adhesive. For example, the adhesive is arranged in the first groove 114. In an assembled state, the first protrusions 121 and the first grooves 114 are fixed together by means of the bonding of the adhesive, thereby realizing the connection and seal between the cup 12 and the soft rubber part. The adhesive includes, but is not limited to, polyacrylic resin, a polyurethane adhesive, a synthetic adhesive, a UV adhesive, etc. The adhesive is applied to the first grooves 114 and/or the first protrusions 121 by means of glue dispensing, manual coating, spraying, rolling, pressure dipping, vacuum dipping, etc.

Further, a surface treatment process may also be used between the soft rubber part 11 and the cup 12 to increase a adhesion therebetween. The surface treatment process includes, but is not limited to, plasma treatment, in-mold decoration (IMD), UV aging treatment, surface wiredrawing treatment, electroplating, etc.

Optionally, the soft rubber part 11 and the cup 12 are made of materials that can be adhered in an injection mold. For example, the soft rubber part 11 and the cup 12 are connected together by a secondary injection molding or a two-color molding process. In one example, the cup 12 is made of a polycarbonate material (PC), and the soft rubber part is made of silicone, TPE, TPU and other materials that can be bonded with a polycarbonate material. During molding, the cup 12 made of the PC material is first molded in a liquid state in the injection mold, and then molded again with silicone, TPE, TPU and other materials to form a soft rubber part 11 on the cup 12. After curing, the cup 12 and the soft rubber part 11 are fixed together. Because the material of the soft rubber part 11 and the material of the cup 12 can form good adhesion, the soft rubber part 11 can be tightly adhered with the cup 12, so that the liner assembly 1 has high structural strength and good sealability.

In one example, the soft rubber part 11 and the cup 12 may be molded by means of a secondary molding or two-color molding process. One of the soft rubber part 11 and the cup 12 is first subjected to injection molding. A hinge hole is formed in a part of the soft rubber part 11 which is used to connect or a part of the cup 12 which is used to connect. In the secondary molding process, an injection molding material enters the hinge hole. After curing, a part of the soft rubber part 11 which is used to connect or a part of the cup 12 which is used to connect are hinged together. In this way, the soft rubber part 11 and the cup 12 have higher adhesive strength and are connected more firmly, and the overall structural strength of the liner assembly 1 is higher.

For example, the cup 12 is provided with a hinge hole 123. The cup 12 is subjected to a secondary injection molding to form the soft rubber part 11. In the secondary molding process, the injection molding material flows through the hinge hole 123. A via hole 116 is formed after the injection molding material is cured. The hinge hole 123 and the via hole 116 are hinged together with each other, so that the cup 12 and the soft rubber part 11 are connected to each other. In this example, the connection strength between the cup 12 and the soft rubber part 11 depends on the strength of materials of the soft rubber part 11 and the cup 12. If the connection between the cup 12 and the soft rubber part 11 is to be destroyed, the material of the cup 12 and/or the material of the soft rubber part 11 need to be broken.

In this way, the connection strength between the soft rubber part 11 and the cup 12 is more reliable, with better sealability.

Moreover, the adhesion between the material of the soft rubber part 11 and the material of the cup 12 is not considered by in the secondary injection molding process, so a selectivity of the materials is wider.

Certainly, in other examples, the materials of the soft rubber part 11 and the cup 12 have strong adhesion to each other, which makes the liner assembly 1 have better connection strength and sealability.

### <IV. Frame>

The outside of the negative pressure chamber is atmospheric pressure intensity. After vacuumizing, the pressure intensity in the negative pressure chamber is less than the outside atmospheric pressure intensity. In this way, there is a pressure intensity difference between the inside and the outside of the negative pressure chamber. The pressure intensity difference causes the liner assembly 1 to be adsorbed on the patient's skin. However, an air pressure in the negative pressure chamber is unstable. For example, movements of a head and contraction of laryngeal muscles of the patient can easily cause a leakage between the liner assembly 1 and the patient's skin, resulting in a change in the air pressure in the negative pressure chamber.

Therefore, a fixing apparatus applied to a negative pressure therapy apparatus needs to be provided to keep the position of the liner assembly 1 stable. The fixing apparatus generally includes a frame 2 and a headband 5. During use, the fixing apparatus stabilizes the liner assembly 1 at the underjaw and the neck of the patient, and ensures the stability of the negative pressure chamber.

An embodiment of the present disclosure provides a frame 2 applied to a negative pressure therapy apparatus. The frame 2 is supported outside the liner assembly 1 and connected to the headband, and provides a supporting force for the liner assembly 1 to ensure the stability of the air pressure in the negative pressure chamber.

The frame 2 includes a frame main body 21 and fixed interfaces 22. For example, fixed interfaces 22 and second mounting holes 23 are formed in the frame main body 21. The fixed interfaces 22 are connected to the headband 5. The fixed interfaces 22 are symmetrically distributed on both sides of the frame main body 21. For example, there are two or four fixed interfaces 22. A first cylinder 221 is arranged on one side of each fixed interface 22. The headband 5 passes through from the fixed interface and bypasses the first cylinder 221, and forms a connection with the frame 2 by the first cylinder 221. Optionally, the first cylinder 221 may be, but not limited to, a prism, a column, etc.

A second mounting hole 23 is formed in the middle of the frame main body 21. The second mounting hole 23 is a round hole, a square hole or other polygonal hole. The second mounting hole 23 is in contact fit with the mounting protrusion 14. When in use, the second mounting hole 23 is sleeved outside the mounting protrusion 14 of the liner assembly 1, so that the frame 2 can form a constraint on the liner assembly 1. The elbow 3 extends out of the first mounting hole 13 and the second mounting hole 23.

Further, the second mounting hole 23 is provided with a rotation-stopping apparatus 231. For example, the rotation-stopping apparatus 231 is a rotation-stopping protrusion, a rotation-stopping groove, etc. For example, the second mounting hole 23 is provided with a rotation-stopping groove. The rotation-stopping protrusion is arranged on the mounting protrusion 14. The rotation-stopping protrusion is clamped into the rotation-stopping groove to form a rotation-stopping fit. Alternatively, the second mounting hole 23 is provided with a rotation-stopping protrusion. A rotation-stopping groove is formed in the mounting protrusion 14. In this way, a rotation-stopping fit can also be formed.

Optionally, the frame 2 is made of a rigid material, which may be, but not limited to, plastic, metal, ceramic, glass, etc. The plastic may be, but are not limited to, PC, PP, PA, or other materials. The frame main body 21 may be integrated by means of injection molding, and meanwhile, the second mounting holes 23, the fixed interfaces 22, the first cylinders 221, the rotation-stopping apparatus 231 and other structures are formed on the frame main body 21.

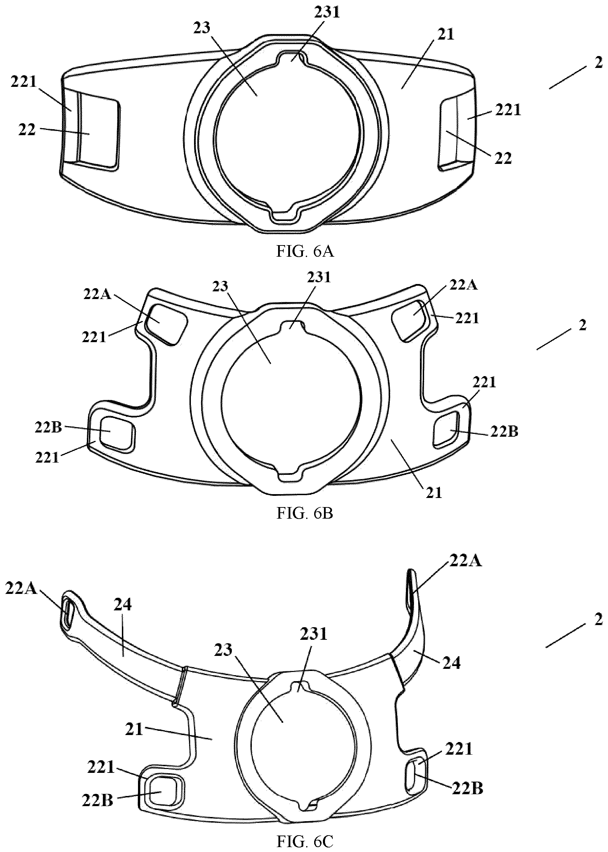

In one example, a number of the fixed interfaces 22 is greater than two. When in use, the plurality of fixed interfaces 22 are connected to the headband 5 to jointly form a fixing apparatus for the negative pressure therapy apparatus together. For example, the number of the fixed interfaces 22 are four, as shown in FIG. 6B.

Specifically, the fixed interfaces 22 include first fixed interfaces 22A and second fixed interfaces 22B. Two first fixed interfaces 22A and two second fixed interfaces 22B are provided, respectively. Moreover, the two first fixed interfaces 22A are symmetrically arranged with respect to the second mounting hole 23, and the two second fixed interfaces 22B are symmetrically arranged with respect to the second mounting hole 23. The two first fixed interfaces 22A are located above the two second fixed interfaces 22B. The first fixed interfaces 22A located on the same side are spaced apart from the second fixed interfaces 22B.

When in use, the two first fixed interfaces 22A and the two second fixed interfaces 22B are connected to different parts of the headband 5, respectively.

In this example, the two first fixed interfaces 22A are located close to the patient's underjaw. The two second fixed interfaces 22B are located close to the patient's neck. After being connected to the headband 5, the frame 2 forms a tightening force on the liner assembly 1 at four positions, so that the liner assembly 1 is more tightly sealed with the patient, thereby effectively avoiding an occurrence of air leakage in the negative pressure chamber caused by the movements of the head and the contraction of laryngeal muscles of the patient.

In one example, as shown in FIG. 6C, the frame 2 further includes a connecting arm 24. The connecting arm 24 is connected to the frame main body 21. The connecting arm 24 is a strip-like structure. The connecting arm 24 extends outward from the frame main body 21. The fixed interface 22 and the first cylinder 221 are arranged at a tail of the connecting arm 24. The connecting arm 24 can swing and deform relative to the frame main body 21, so that positions of the headband 5 and the connecting arm 24 can be dynamically adjusted. By arranging the connecting arm 24, when the patient wears the negative pressure face mask assembly, the negative pressure face mask assembly can dynamically adjust the position of the fixed interface 22 with the movements of the patient's neck (movements of the head, neck muscles, etc.), thereby reducing an influence of the movements of the patient's neck on a sealing position of the liner assembly 1 and the patient's skin, and improving a stability of the sealed negative pressure chamber.

Further, the connecting arm 24 is of a flat, thin-walled structure with set hardness and flexibility, for example, the connecting arm 24 is made of PP, PA, etc. The thin-walled structure makes the connecting arm 24 bend, deform in a thickness direction and have a certain elasticity.

Preferably, a plurality of connecting arms 24 are symmetrically distributed on both sides of the frame main body 21. In this way, a structure formed by the connecting arms 24 and the headband 5 is more stable.

Moreover, a tightening force of the headband 5 and the frame 2 on the liner assembly 1 is more balanced.

In one specific example, as shown in FIG. 6C, four connecting arms 24 are provided. The four connecting arms 24 are symmetrically in pairs connected to the frame main body 21. There are four fixed interfaces 22. The four fixed interfaces 22 are arranged at the ends of the four connecting arms 24, respectively. The two connecting arms 24 are symmetrically arranged on an upper side of the frame main body 21, and are used to extend the positions of the two first fixed interfaces 22A to a direction away from the frame main body 21. The other two connecting arms 24 are symmetrically arranged on a lower side of the frame main body 21, and are used to extend the positions of the two first fixed interfaces 22B to a direction away from the frame main body 21 (not shown). This setting allows for more room for the patient's head to move and ensures a good sealing effect during the movements of the patient's head.

Preferably, a flexible sleeve (not shown) is arranged outside the connecting arm 24. The flexible sleeve is made of a flexible material. The flexible sleeve is wrapped or attached to an outside of the connecting arm 24. The flexible sleeve has a good haptic feel and can improve the wearing comfort of the patient. The flexible material may be a textile material. For example, the textile material is natural cotton, down feather, natural silk and other materials. The flexible material may also be a textile material formed by a natural material or artificial fibers by means of weaving, knitting, coating, crocheting or bonding. The flexible sleeve may be a single-layer textile material or a multi-layer textile material. The flexible sleeve may also be a multi-layered textile made of various textile materials and non-textile materials. The non-textile materials may be a polyurethane foam material, etc. The flexible sleeve may also be a textile containing a plastic material, such as a nylon and polyester blend, a nylon and spandex blend, a polyester and spandex blend, a nylon, polyester and spandex blend, microfiber or a polyurethane blend, etc.

### <V. Elbow and hose>

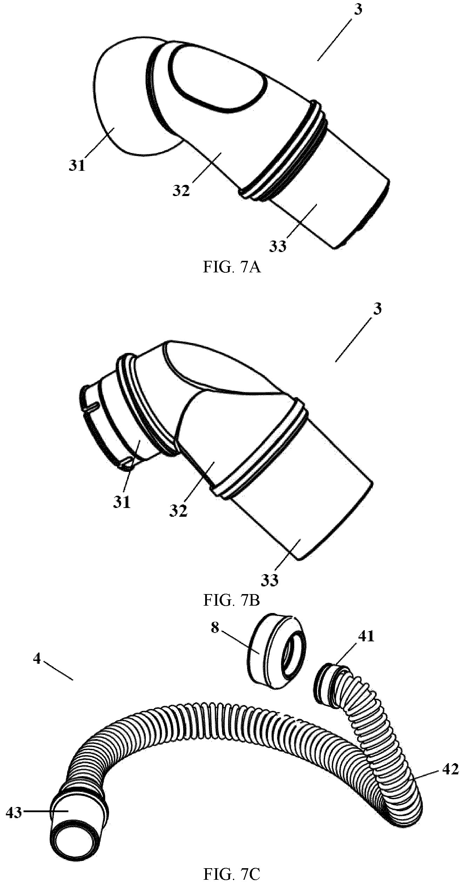

An embodiment of the present disclosure provides an elbow 3 applied to a negative pressure therapy apparatus. As shown in FIG. 7A and FIG. 7B, the elbow 3 is of a hollow pipe structure. The elbow 3 forms a part of a negative pressure ventilation air path. The elbow 3 is connected between the liner assembly 1 and an air extraction pipe 6, and can provide a certain rotational degree of freedom and/or swing degree of freedom, to reduce an influence of the swing or drag of the air extraction pipe 6 on an air impermeability of the negative pressure chamber.

In one example, as shown in FIG. 7A and FIG. 7B, the elbow 3 includes a first interface 31, an elbow main body 32 and a second interface 33. The first interface 31 may be assembled and connected to the frame 2, or may also be assembled and connected to the liner assembly 1.

For example, the first interface 31 may be of a ball joint structure, as shown in FIG. 7A. During assembly, the ball joint structure forms a spherical connection with the liner assembly 1 or the frame 2. The spherical connection makes the elbow 2 constitute two degrees of freedom of rotation and swing in a form of a spherical pair relative to the liner assembly 1 or the frame 2.

Alternatively, the first interface 31 may also be of a cylindrical structure, as shown in FIG. 7B. During assembly, the cylindrical structure forms a cylindrical connection with the liner assembly 1 or the frame 2. The cylindrical connection makes the elbow 2 constitute a rotation degree of freedom in a form of a cylindrical pair relative to the liner assembly 1 or the frame 2.

The second interface 33 may be assembled and connected to the hose 4, or may also be assembled and connected to the air extraction pipe 6. As shown in FIG. 7A, the second interface 33 may be of a male connector structure. During assembly, the hose 4 or the air extraction pipe 6 is sleeved outside the second interface 33. Certainly, the second interface 33 may also be of a female connector structure, as shown in FIG. 7B. During assembly, the hose 4 or the air extraction pipe 6 is inserted into an inner side of the second interface 33.

Further, a connection mode between the second interface 33 and the hose 4 or the air extraction pipe 6 may be a cylindrical pair connection, to constitute a rotation degree of freedom. The hose 4 or the air extraction pipe 6 can rotate relative to the elbow 3. In this way, both an influence of the swing or drag of the air extraction pipe 6 on the elbow 3 and an influence of the above-mentioned swing or drag on the air impermeability of the negative pressure chamber can be reduced.

An embodiment of the present disclosure provides a hose 4 applied to a negative pressure therapy apparatus. For example, the hose 4 is a pipe composed of a flexible material. Optionally, the hose 4 may also be a corrugated pipe. The hose 4 forms a part of a negative pressure ventilation air path. The hose 4 can be bent and deformed during use.

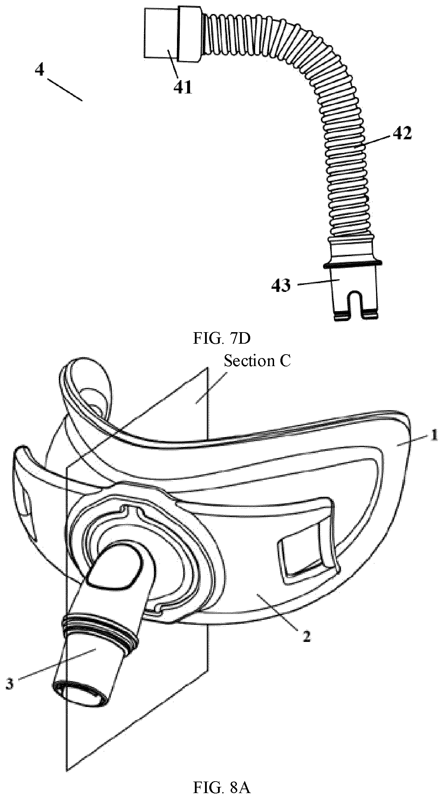

In one example, as shown in FIG. 7C and FIG 7D, the hose 4 includes a third interface 41, an elbow main body 42 and a fourth interface 43. The third interface 41 may be directly assembled and connected to the frame 2 or the liner assembly 1, or may also be assembled and connected to the elbow 3. The above-mentioned connection mode may be a cylindrical connection, to constitute a rotation degree of freedom. Alternatively, the fourth interface 43 is connected to the air extraction pipe 6 or a negative pressure source 7. Similarly, a connection mode between the fourth interface 4 and the air extraction pipe 6 or the negative pressure source 7 may be a cylindrical connection.

Preferably, an intermediate part 8 is further included, as shown in FIG. 7C. The intermediate part 8 is located between the frame 2 and the elbow 3 or between the frame 2 and the hose 4.

Optionally, an assembly and connection structure mode among the intermediate part 8, the elbow 3, the frame 2 and the hose 4 may be, but not limited to, the following embodiments.

The negative pressure therapy apparatus includes a liner assembly 1, a frame 2, an elbow 3, an air extraction pipe 6 and a negative pressure source 7. The elbow 3 is connected between the frame 2 and the air extraction pipe 6.

The negative pressure therapy apparatus includes a liner assembly 1, a frame 2, a hose 4, an air extraction pipe 6 and a negative pressure source 7. The hose 4 is connected between the frame 2 and the air extraction pipe 6.

The negative pressure therapy apparatus includes a liner assembly 1, a frame 2, an elbow 3, an air extraction pipe 6, a negative pressure source 7 and an intermediate part 8. The elbow 3 is connected between the frame 2 and the air extraction pipe 6 through the intermediate part 8.

The negative pressure therapy apparatus includes a liner assembly 1, a frame 2, a hose 4, an air extraction pipe 6, a negative pressure source 7 and an intermediate part 8. The hose 4 is connected between the frame 2 and the air extraction pipe 6 through the intermediate part 8.

The negative pressure therapy apparatus includes a liner assembly 1, a frame 2, an elbow 3, a hose 4, an air extraction pipe 6 and a negative pressure source 7. The elbow 3 is connected between the frame 2 and the hose 4. The hose 4 is connected to the air extraction pipe 6.

The negative pressure therapy apparatus includes a liner assembly 1, a frame 2, an elbow 3, a hose 4, an air extraction pipe 6, a negative pressure source 7 and an intermediate part 8. The elbow 3 is connected between the frame 2 and the hose 4 through the intermediate part 8. The hose 4 is connected to the air extraction pipe 6.

### <VI. Connection mode of elbow and/or hose, frame, and liner assembly>

An embodiment of the present disclosure provides a connection mode of a liner assembly 1 applied to a negative pressure therapy apparatus, a frame 2, an elbow 3, a hose 4 and other components. A connection mode among the above components may be, but not limited to, ultrasonic welding, laser welding, high-frequency welding, adhesion and other connection modes.

Preferably, the liner assembly 1, the frame 2, the elbow 3 and the hose 4 are connected in a detachable manner, in such a way that a component can be removed from the whole machine for easy cleaning and replacement.

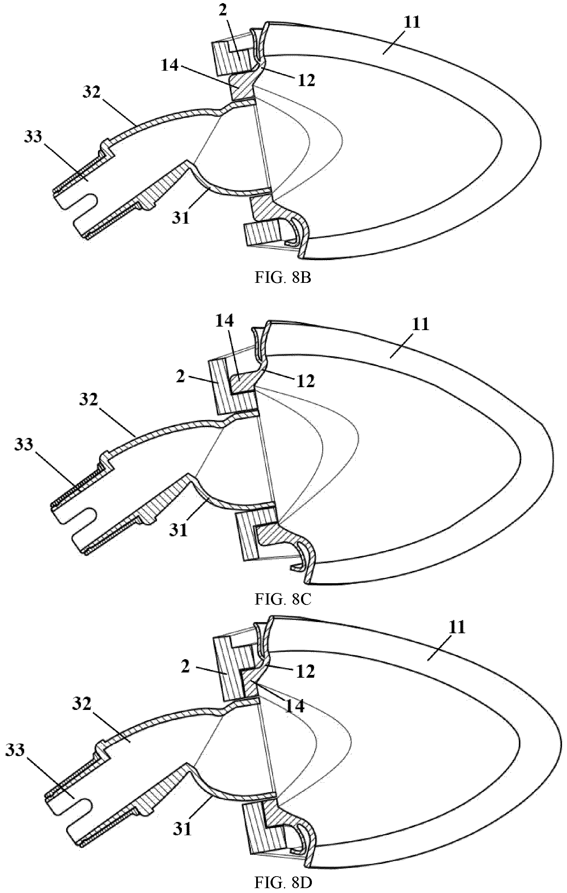

In one example, as shown in FIG. 8B, the liner assembly 1 is assembled and connected to the frame 2. The elbow 3 is connected to the liner assembly 1.

Specifically, the liner assembly is provided with mounting protrusions 14, as shown in FIG. 1 and FIG. 4A. The mounting protrusions 14 are clamped in the second mounting holes 23 of the frame 2, as shown in FIG. 8A-FIG. 8B. The liner assembly 1 is provided with protrusions or grooves. The protrusions or grooves form a rotation-stopping fit with the rotation-stopping apparatus 231 of the frame 2 (as shown in FIGs. 6A-6C) to prevent a relative rotation between the liner assembly 1 and the frame 2.

The first interface 31 of the elbow 3 is assembled in the first mounting hole 13 of the liner assembly 1 (as shown in FIG. 1-FIG. 5D). The connection between the elbow 3 and the liner assembly 1 may be a cylindrical connection or a spherical connection.

In another example, as shown in FIG. 8C, the frame 2 is assembled and connected to the liner assembly 1. The second mounting hole 23 has an annular protrusion that extends inward. The annular protrusion is embedded in the mounting protrusion 14. The elbow 3 is connected to the frame 2.

As shown in FIG. 1 and FIG. 5D, a protrusion or groove is arranged in the mounting protrusion 14 to form a rotation-stopping fit between the liner assembly 1 and/or the frame 2. The first interface 31 of the elbow 3 is assembled in the second mounting hole 23 of the frame 2. The connection between the first interface 31 and the second mounting hole 23 may be a cylindrical connection or a spherical connection.

In another example, as shown in FIG. 8D, the liner assembly 1 is assembled and connected to the frame 2. The first mounting hole 13 is communicated with the second mounting hole 23 to enclose a channel together. The frame 2 is arranged outside the mounting protrusion 14. The elbow 3 is connected into the first mounting hole 13 and the second mounting hole 23.

Specifically, the mounting protrusion 14 of the liner assembly 1 is clamped into the frame 2. The rotation-stopping fit is formed between the liner assembly 1 and the frame 2 to prevent relative rotation therebetween. The first mounting hole 13 of the liner assembly 1 and the second mounting hole 23 of the frame 2 together form a mounting hole position connected to the first interface 31 of the elbow 3. During assembly, the first interface 31 of the elbow 3 is simultaneously assembled into the first mounting hole 13 and the second mounting hole 23. The connection between the elbow 3 and the liner assembly 1 as well as the frame 2 may be a cylindrical connection or a spherical connection.

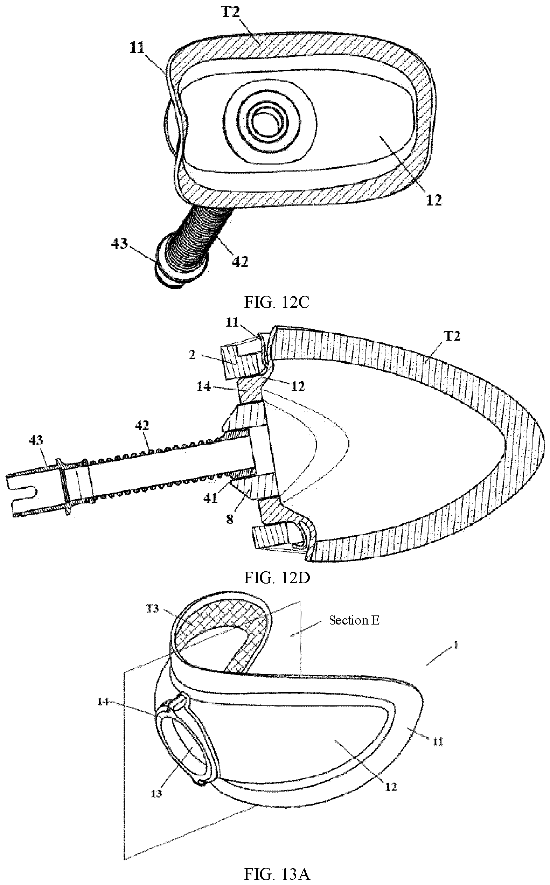

In other examples, the elbow 3 in the above embodiment is replaced with a hose 4, as shown in FIG. 12D. In this example, a connection mode between the hose 4 and the liner assembly 1 as well as the frame 2 adopts a connection mode between the elbow 3 and the liner assembly 1 as well as the frame 2 in FIG. 8A-FIG. 8D, and is not repeated herein.

In other examples, the elbow 3 and/or the hose 4 may also be connected to the liner assembly 1 and/or the frame 2 through the intermediate part 8. The structure of the intermediate part 8 is shown in FIG. 7.

Preferably, the elbow 3 and/or the hose 4 are/is detachably connected to the liner assembly 1 and/or the frame 2.

For example, the cylindrical or spherical connection described above may be set as a detachable connection. If the elbow 3 and/or the hose 4 are/is damaged, the elbow 3 and/or the hose 4 may be detached from the liner assembly 1 and/or the frame 2 for replacement, thereby preventing the liner assembly 1, the frame 2, etc., from being scrapped. If the elbow 3 and/or the hose 4 are/is dirty, the elbow 3 and/or the hose 4 may be detached from the liner assembly 1 and/or the frame 2 for cleaning. When the patient needs to interrupt the therapy, the elbow 3 and/or the hose 4 may be detached from the liner assembly 1 and/or the frame 2, while the entire negative pressure therapy apparatus does not need to be removed from the patient's neck, so that the liner assembly 1 can be effectively avoided from causing poor sealability due to repeated dismantling. When components on the elbow 3 and/or the hose 4 need to be replaced or added or reduced, the elbow 3 and/or the hose 4 can be detached from the liner assembly 1 and/or the frame 2 for ease of operation.

### <VII. Groove>

An embodiment of the present disclosure provides another liner assembly 1 applied to a negative pressure therapy apparatus. The liner assembly 1 includes a soft rubber part 11, a cup 12, and a first mounting hole 13. A groove 15 that protrudes outwards from the negative pressure chamber is formed in the cup 12 and/or the soft rubber part 11. The head extrudes and stretches the liner assembly 1 as it moves. During extrusion, the cup 12 is prone to extrusion deformation or tensile deformation. The above-mentioned deformation can weaken a transmission of a force of the head movement to the soft rubber part 11, thereby reducing a movement at a sealing point C, making the sealing of the negative pressure chamber more stable, and reducing a risk of air leakage.

Moreover, the negative pressure source 7 can dynamically compensate for a change of a negative pressure value caused by a volume change of the negative pressure chamber. However, when head movement and/or laryngeal muscle movement (e.g., swallowing action) occurs, the air extraction of the negative pressure source 7 needs to be transmitted over a long distance through the air extraction pipe 6 before it can act on the negative pressure chamber. Therefore, the negative pressure value of the negative pressure chamber cannot be adjusted in real time. At a moment of head movement, the dynamic compensation of the negative pressure source 7 cannot be in place in real time. An air pressure of the negative pressure chamber is inversely proportional to a volume of the negative pressure chamber. That is, if the volume of the negative pressure chamber increases suddenly, the air pressure in the negative pressure chamber will decrease suddenly; and if the volume of the negative pressure chamber decreases suddenly, the air pressure in the negative pressure chamber will increase suddenly. In this example, the cup 12 is provided with a groove 15. The groove 15 can dynamically compensate for the volume of the negative pressure chamber in real time when the head movements (e.g., nodding and head shaking) and/or laryngeal muscle movements (e.g., swallowing) occur, thereby reducing the change of the air pressure value in the negative pressure chamber, further ensuring the stability of the negative pressure chamber and reducing the risk of air leakage.

For example, the groove 15 is made of a soft rubber material, and can be deformed by stretching or extrusion as the head moves. The soft rubber material may be, but not limited to, a rubber, or a silicone.

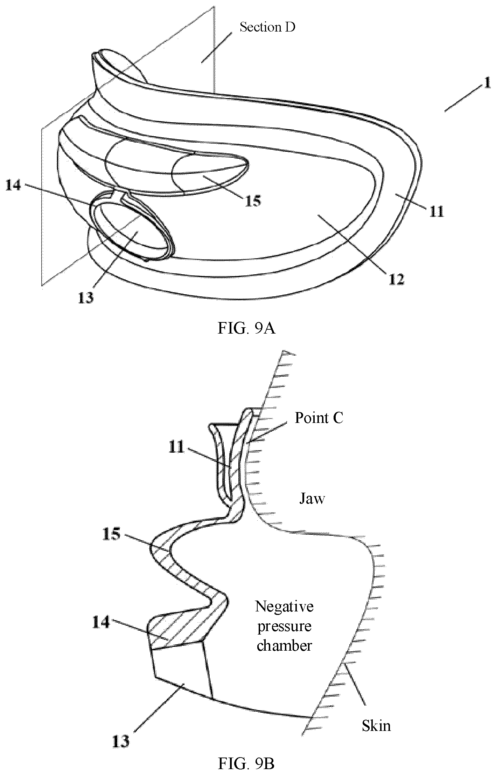

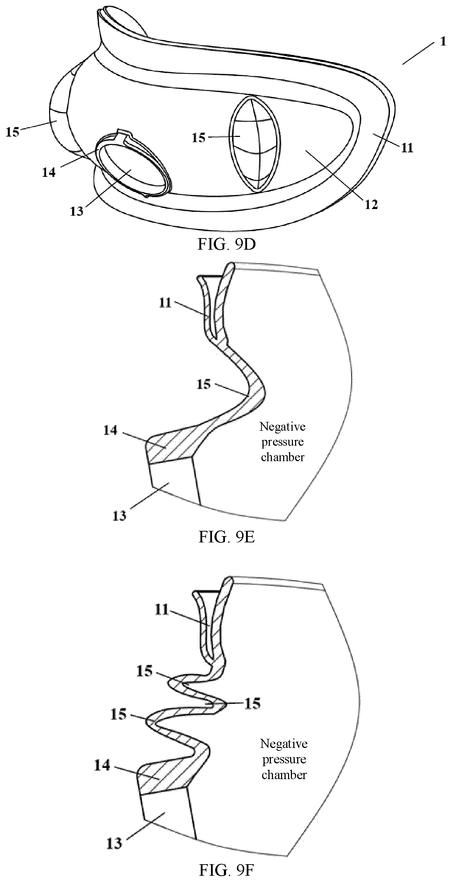

In one example, as shown in FIG. 9A-FIG. 9C, the soft rubber part 11 and the cup 12 are made of the same soft rubber material. The soft rubber part 11 and the cup 12 are integrally molded. Grooves 15 are formed in the cup 12. For example, a depression direction of the grooves 15 is away from the negative pressure chamber and extends in a horizontal direction. The grooves 15 are arranged axisymmetrically, or may also be arranged asymmetrically.

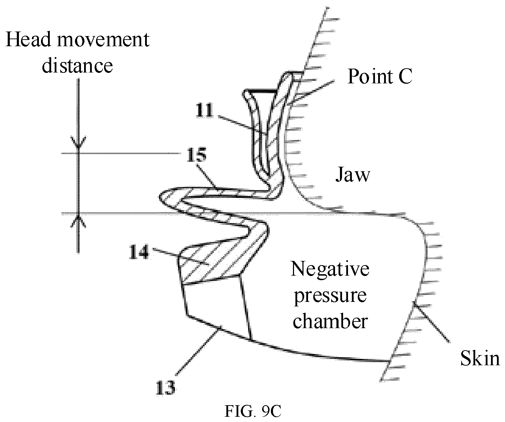

When the patient nods his/her head, the patient's underjaw will move in a vertical direction. FIG. 9B and FIG. 9C illustrate states of the grooves 15 when the patient's underjaw is in a high position and a low position respectively during the nodding movement. Specifically, when the patient's underjaw is in the high position, as shown in FIG. 9B, the groove 15 is in a tensile deformation state, and the volume of the negative pressure chamber is relatively large; and when the patient's underjaw is in the low position, as shown in FIG. 9C, the groove 15 is in a compression deformation state, and the volume of the negative pressure chamber is relatively small.

Because the state change of the grooves 15 absorbs the deformation of the soft rubber part 11 during the nodding movement, the position of the sealing point C does not move during the nodding movement, so that the sealing failure caused by the movement of the sealing point C is avoided, thereby ensuring the sealing stability of the negative pressure chamber.

Preferably, the grooves 15 are arranged at a position of the cup 12 close to the patient's underjaw. In this way, the deformation of the soft rubber part 11 caused by the movement of the patient's underjaw can be effectively absorbed.

Further, the width of each groove 15 gradually decreases from the middle to both sides in a horizontal direction. The middle of the groove 15 in the horizontal direction is opposite to the middle of the patient's underjaw. During the nodding process of the patient, the displacement of the middle position of the underjaw is maximum, and gradually decreases from the middle position to both sides. In this example, the width of each groove 15 is set according to the displacements of different parts of the patient's underjaw, so that an imbalance of the materials at various parts of the groove 15 is avoided.

During the nodding movement, the grooves 15 can adjust the volume of the negative pressure chamber in real time. As the patient's underjaw moves from a high position to a low position, the volume of the negative pressure chamber decreases. At this moment, the groove 15 expands outwards to dynamically compensate for a reduction of the volume of a part of the negative pressure chamber, and prevents a suddenly sharp increase of the pressure value in the negative pressure chamber. As the patient's underjaw moves from a low position to a high position, the volume of the negative pressure chamber increases. At this moment, the groove 15 collapses inward to dynamically reduce an increment of the volume of the negative pressure chamber, and prevents a sharp decrease of the air pressure in the negative pressure chamber. In this way, the grooves 15 ensure the stability of the negative pressure chamber and reduce the risk of air leakage.

In another example, the groove 15 recesses outward and extends in a vertical direction. Preferably, the groove 15 is formed in the left and/or right side of the liner assembly 1.

As shown in FIG. 9D, the soft rubber part 11 and the cup 12 are made of the same soft rubber material. The soft rubber part 11 and the cup 12 are integrally molded, e.g., by means of injection molding. The grooves 15 are formed in the cup 12. For example, the grooves 15 are arranged in the left and right sides of the cup 12. The two grooves 15 are arranged symmetrically. When the patient has a head shaking movement, the groove 15 may be undergo a tensile deformation or compression deformation accordingly, so that the transmission of the force at the time of head shaking to the soft rubber part 11 is avoided, the movement at the sealing point C is reduced, and the sealing failure at the sealing point C is prevented, thereby ensuring the stability of the negative pressure chamber.

Moreover, the groove 15 can dynamically compensate for the volume of the negative pressure chamber in real time, and make up for a sharp change of the air pressure in the negative pressure chamber during the head shaking movement. The stability of the negative pressure chamber is further ensured.

In other examples, the grooves 15 may also be formed in parts of the cup 12 near the patient's underjaw and on the left and right sides of the cup 12. In this way, the stability of the negative pressure chamber during both the nodding and head shaking movements of the patient can also be ensured.

In other examples, one groove 15 is provided. This groove 15 is arranged around the first mounting hole 13 in one circle. This groove 15 can absorb the movement of the patient's head in any direction.

In other examples, a plurality of grooves 15 are provided. The plurality of grooves 15 are arranged around the first mounting hole 13. The grooves 15 can absorb the movement of the patient's head in any direction.

In one example, as shown in FIG. 9F, the grooves 15 are formed in the cup 12. Each groove 15 recesses toward the inner side of the negative pressure chamber. The positions of the grooves 15 are close to the positions of the mounting protrusions 14. In this way, the grooves 15 can absorb the deformation of the cup 12 caused by the patient's nodding.

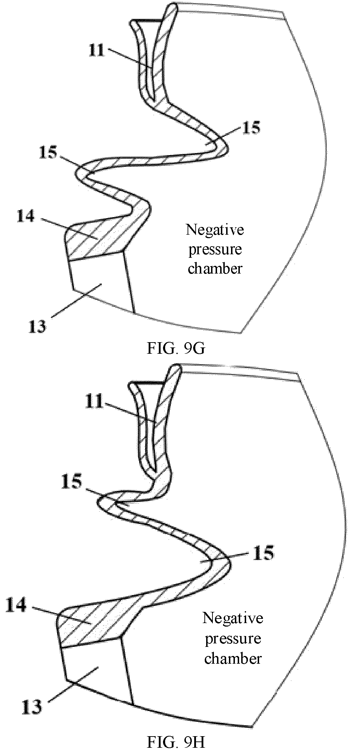

In one example, as shown in FIG. 9G, a plurality of (e.g., three) grooves 15 are provided. The grooves 15 recess outward from the negative pressure chamber. The other groove 15 recess toward the inner side of the negative pressure chamber, and is located between two grooves that recess outward. In this way, the plurality of grooves 15 can more effectively absorb the deformation of the cup 12 caused by the patient's nodding, and avoid the destruction of the seal formed between the soft rubber part 11 and the patient's skin.

In one example, as shown in FIG. 9G, grooves 15 that recess outward from the negative pressure chamber are formed near the mounting protrusion 14. The grooves 15 that recess toward the inner side of the negative pressure chamber are formed in a position close to the soft rubber part 11. The two grooves 15 can more effectively absorb the deformation of the cup 12 caused by the patient's nodding, and avoid the destruction of the seal formed between the soft rubber part 11 and the patient's skin.

In one example, as shown in FIG. 9H, grooves 15 that recess toward the inner side of the negative pressure chamber are formed in a position close to the mounting protrusion 14. The grooves 15 that recess outward the negative pressure chamber are formed in a position close to the soft rubber part 11. The two grooves 15 can more effectively absorb the deformation of the cup 12 caused by the patient's nodding, and avoid the destruction of the seal formed between the soft rubber part 11 and the patient's skin.

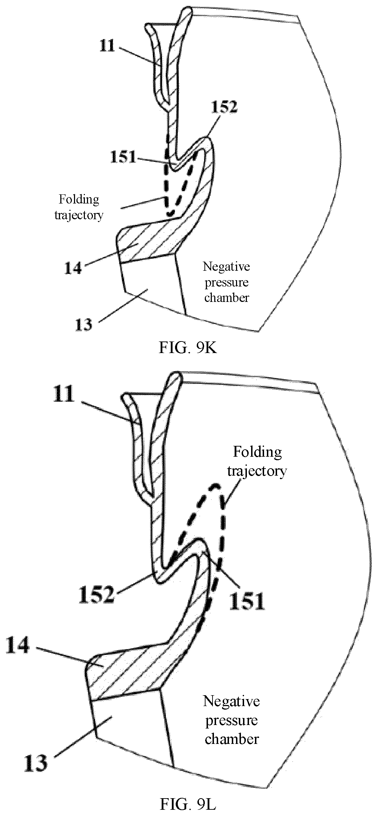

In one example, the groove 15 is configured to allow local folding to occur during the head movements of the patient. As shown in FIG. 9K, the groove 15 is located at a position shown by a solid line in FIG. 9K in a normal state. When the patient nods, the groove 15 is able to move downward. For example, the groove 15 moves to a dotted line position in FIG. 9K. By the local folding of the groove 15, a downward force received by the liner assembly 1 when the patient nods can be effectively absorbed, so that the soft rubber part 11 is avoided from moving on the patient's skin and the occurrence of leakage is reduced. For example, the local folding of the groove 15 is realized by controlling the thickness of different parts of the groove 15.

Specifically, the soft rubber part 11 and the cup 12 are both integrally molded from the same soft rubber material, and the groove 15 is formed in the cup 12. A first bending part 151 is formed at a part on the edge of the groove 15 that is in contact with the cup 12. A second bending part 152 is formed at the bottom of the groove 15. The thickness of the first bending part 151 is smaller than that of the second bending part 152. Therefore, when subjected to an external force, the first bending part 151 is more likely to be deformed than the second bending part 152. When the patient moves his head, the position of the first bending part 151 will move, causing the groove 15 to fold.

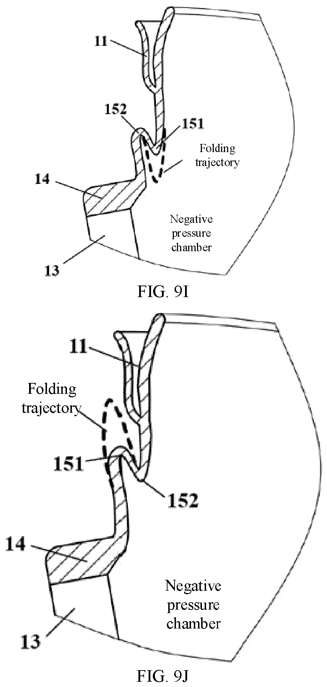

FIG. 9I to FIG. 9K show folding positions and directions of the first bending parts 151 having different structures.

In one example, as shown in FIG. 9I, the first bending part 151 is connected to the soft rubber part 11. The first bending part 151 recesses from the outside of the negative pressure chamber to the inside of the negative pressure chamber. The second bending part 152 is connected to the mounting protrusion 14. The second bending part 152 recesses outward from the negative pressure chamber. The first bending part 151 is located outside the second bending part 152. The thickness of the first bending part 151 is smaller than that of the second bending part 152.

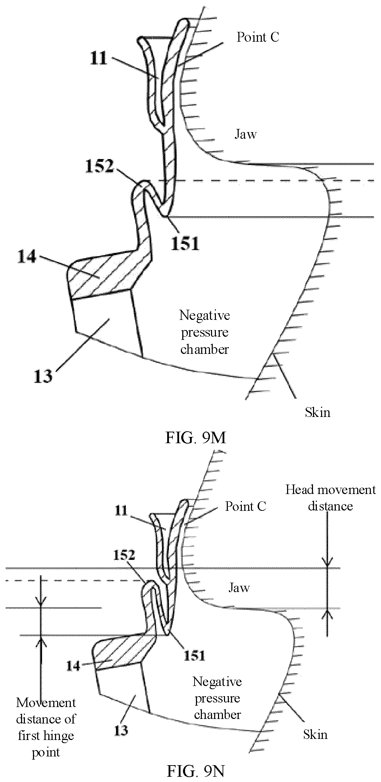

Specifically, the groove 15 is formed in a position of the cup 12 close to the patient's underjaw. When the patient nods his/her head, the patient's underjaw will move in a vertical direction. FIG. 9M and FIG. 9N illustrate states and positions of the first bending part 151 and the second bending part 152 respectively when the patient's underjaw is in a high position and a low position during the nodding movement. When the patient's underjaw is in a high position (as shown in FIG. 9M), the groove 15 is not folded, and the volume of the negative pressure chamber is relatively large. When the patient's underjaw is in a low position (as shown in FIG. 9N), the position of the second bending part 152 remains unchanged. Because the thickness of the first bending part 151 is relatively thin, it is folded downward on the inner side of the negative pressure chamber, and the volume of the negative pressure chamber is reduced. In this way, the position of the sealing point C does not move during the nodding movement, so that the air leakage of the negative pressure chamber caused by the sealing failure at the sealing point C can be avoided, thereby ensuring the stability of the negative pressure chamber.

Moreover, during the nodding movement, the volume of the negative pressure chamber can be adjusted in real time when the first bending part 151 is folded. Specifically, when the patient's underjaw moves from a high position (as shown in FIG. 9M) to a low position (as shown in FIG. 9N), the volume of the negative pressure chamber is reduced. At this moment, the groove 15 expands in a direction away from the negative pressure chamber to dynamically make up for the decrement of the volume of the negative pressure chamber, and prevents the sharp increase of the pressure in the negative pressure chamber. As the patient's underjaw moves from a low position (as shown in FIG. 9N) to a high position (as shown in FIG. 9M), the volume of the negative pressure chamber increases. At this moment, the groove 15 is folded inward from the outside of the negative pressure chamber to dynamically reduce the increment of the volume of the negative pressure chamber, and prevents the sharp decrease of the air pressure in the negative pressure chamber. In this way, the stability of the negative pressure chamber can be ensured to reduce the risk of air leakage of the negative pressure chamber.

In one example, as shown in FIG. 9J, the first bending part 151 is connected to the mounting protrusion 14. The first bending part 151 recesses outward from the inside of the negative pressure chamber. The second bending part 152 is connected to the soft rubber part 11. The second bending part 152 recesses inward from the outside of the negative pressure chamber. The first bending part 151 is located outside the second bending part 152. The thickness of the first bending part 151 is smaller than that of the second bending part 152. When a head movement occurs, the first bending part 151 is folded upward toward the outside of the negative pressure chamber.

In one example, as shown in FIG. 9K, the first bending part 151 is connected to the soft rubber part 11. The first bending part 151 recesses outward from the inside of the negative pressure chamber. The second bending part 152 is connected to the mounting protrusion 14. The second bending part 152 recesses from the outside to the inside of the negative pressure chamber. The first bending part 151 is located outside the second bending part 152. The thickness of the first bending part 151 is smaller than that of the second bending part 152. In this example, when a head movement of the patient occurs, the first bending part 151 is folded downward toward the outside of the negative pressure chamber.

In a fourth embodiment, as shown in FIG. 9L, the first bending part 151 is connected to the mounting protrusion 14. The first bending part 151 recesses from the outside of the negative pressure chamber to the inside of the negative pressure chamber. The second bending part 152 is connected to the soft rubber part 11. The second bending part 152 recesses outward from the negative pressure chamber. The first bending part 151 is located inside the second bending part 152. The thickness of the first bending part 151 is smaller than that of the second bending part 152. When a head movement occurs, the first bending part 151 is folded upward toward the inside of the negative pressure chamber.

Certainly, numbers of the first bending part 151 and the second bending part 152 are not limited herein, and may be set by those skilled in the art according to actual needs.

### <VIII. Headband>

An embodiment of the present disclosure provides a headband which is applied to a negative pressure ventilation apparatus. The headband includes a headband body and a plurality of connecting bands.

The headband body is constructed to cover a head top and an occipital bone of the patient. A middle part of the headband body is of a hollow structure. One end of each connecting band is connected to an edge of the headband body. A part of at least one of the connecting bands that is connected to the headband body is located behind the patient's ears. The plurality of connecting bands extend towards the patient's underjaw. The plurality of connecting bands are arranged symmetrically relative to the headband body. A headband fixing structure 54 is arranged on at least one of the connecting bands. The other end of the connecting band is bent and then connected to the headband fixing structure 54. The headband fixing structure 54 is constructed to adjust a length of the connecting band. The headband fixing structure 54 is used to connect with the negative pressure face mask.

Specifically, the headband is made of a textile material. For example, the textile material may be cotton, down feather, natural silk or other textile materials. Alternatively, the headband may also be a textile formed by a natural material or artificial fibers by means of weaving, knitting, coating, crocheting or bonding.

Alternatively, the headband is a composite material, that is, includes a multi-layer material. for example, a textile material layer, or a non-textile material layer. The non-textile material may be pore-forming polyurethane foams, etc.

In other examples, the material of the headband may include a plastic material, such as a nylon and polyester blend, a nylon and spandex blend, a polyester and spandex blend, a nylon, polyester and spandex blend, microfiber or a polyurethane blend, etc.

Certainly, the material of the headband is not limited to the above embodiments, and may be selected by those skilled in the art according to actual needs.

The headband body covers the head top and the occipital bone of the patient. In this way, the headband body can provide a binding force from a plurality of directions to the negative pressure face mask assembly, thereby meeting a sealing requirement of the negative pressure face mask assembly.

The middle part of the headband body is of a hollow structure. In this way, the headband body is of a circular structure. Because the middle part of the headband body is hollowed, it can be adapted to head shapes of different patients, which makes the headband body highly applicable.

Moreover, the middle part of the headband body is of a hollow structure to facilitate ventilation on the head top of the patient.

The connecting bands are used to connect the headband body and the negative pressure face mask assembly, so that the negative pressure face mask assembly can be sealed to the underjaw and neck of the patient. One end of each connecting band is connected to the edge of the headband body, for example, by means of sewing, integrated knitting, etc.

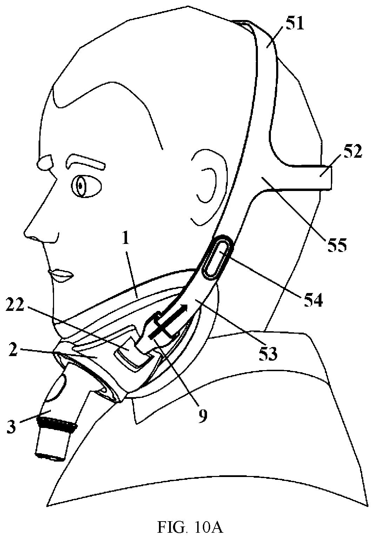

In this example, a part of at least one of the connecting bands that is connected to the headband body is located behind the patient's ears. This connecting band is connected to the negative pressure face mask assembly through rears of the ears. As shown in FIG. 10A, the connecting band extends towards the patient's underjaw for easy connection to the negative pressure face mask assembly. The plurality of connecting bands are arranged symmetrically relative to the headband, thereby ensuring a force balance of different parts of the negative pressure face mask assembly.

In this example, the other end of the connecting band passes through the headband fixing structure 54 and is bent. The headband fixing structure 54 is used to adjust an effective length of the connecting band.

As shown in FIG. 10A, the headband body includes a first sub-band 51 and a second sub-band 52. The first sub-band 51 passes over the head top of the patient. The second sub-band 52 passes over the occipital bone of the patient. Both ends of the first sub-band 51 are connected to both ends of the second sub-band 52, respectively. The first connecting band 53 is connected to the first sub-band 51 and the second sub-band 52. The first connecting band 53 is connected to the headband body at a first connecting part 55. The first connecting part 55 is located behind the patient's ears. In this way, the first connecting band 53 can provide an upward force to the negative pressure face mask assembly, and this force causes the negative pressure face mask assembly to seal well with the patient.

Moreover, the upward force allows the head top of the patient to bear a weight of the negative pressure face mask assembly, which significantly improves the patient's wearing comfort compared to the weight of the negative pressure face mask assembly being borne by other parts of the patient's head.

As shown in FIG. 10A, the first sub-band 51 and the second sub-band 52 are connected at the first connecting part 55. In this way, the first connecting band 53, the first sub-band 51 and the second sub-band 52 are connected at the first connecting part 55. This makes the structure of the headband simple.

Moreover, an extension direction of the first sub-band 51 is consistent with an extension direction of the first sub-band 53, so that the weight of the negative pressure face mask passes through the head top of the patient acting on the first connecting band 53 and the first sub-band 51.

In one example, a quick-disassembly structure 9 is arranged on the connecting band. The quick-disassembly structure 9 has a hook 91. The hook 91 is used for detachable connection with the negative pressure face mask assembly.

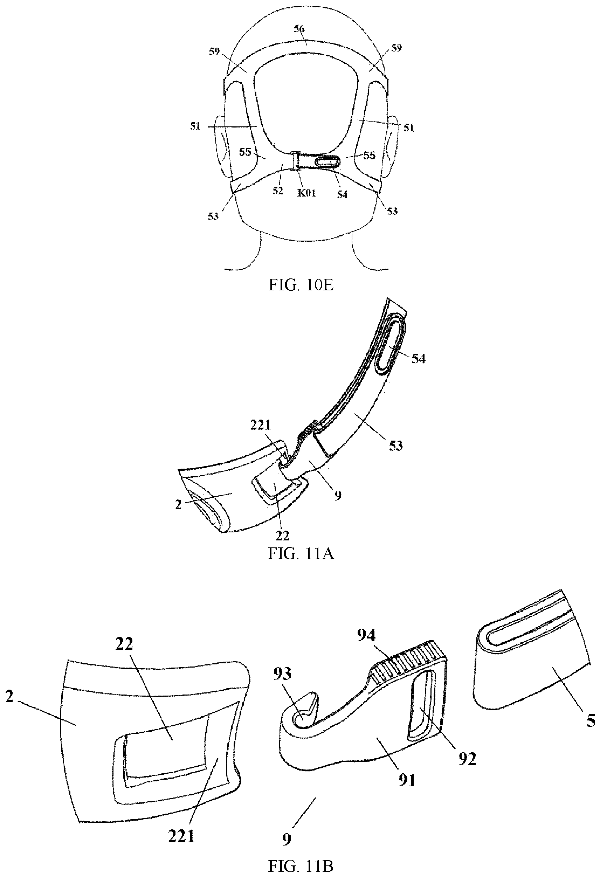

For example, the two first connecting bands 53 are symmetrically arranged with respect to the first sub-band 51. The other ends of the two first connecting bands 53 are connected to the quick-disassembly structure 9. As shown in FIG. 11A-FIG. 11B, one end of the quick-disassembly structure 9 is provided with a strip-shaped hole 92. The first connecting band 53 passes through the strip-shaped hole 92, and is bent and then connected to the headband fixing structure 54. The headband fixing structure 54 fixes the bent first connecting band 53. For example, the headband fixing structure 54 includes a clamping slot and a plurality of protrusions. The plurality of protrusions are arranged at intervals in a length direction of the first connecting band 53. The clamping slot is formed in the end of the first connecting band 53. After the first connecting band 53 is bent, it is clamped with different protrusions according to needs, so that the effective length of the first connecting band 53 is adjusted.

As shown in FIG. 10A, FIG. 11A, and FIG. 11B, the negative pressure face mask assembly includes a liner assembly 1, an elbow 3, and a frame 2. The elbow 3 is connected to the liner assembly 1. The frame 2 is sleeved outside the elbow 3 and is located in front of the liner assembly 1. A fixed interface 22 is respectively formed in left and right sides of the frame 2. A first cylinder 221 is arranged on one side of each fixed interface 22. A hook 91 is arranged at the other end of the quick-disassembly structure 9. The hook 91 is hooked on the first cylinder 221. The hook 91 can be quickly disassembled from the first cylinder 221, thereby facilitating the patient to perform other activities.

As shown in FIG. 11B, the quick-disassembly structure 9 includes a quick-disassembly main body and a hook 91. A strip-shaped hole 92 is formed in one end of the quick-disassembly main body, and the hook 91 is connected to the other end of the quick-disassembly main body. Anti-skid protrusions 94 are arranged on both sides of the quick-disassembly main body for facilitating an operator to grip. The connecting band passes through the strip-shaped hole 92, is reversely bent and then fixed through the headband fixing structure 54. The hook 91 is connected to the first cylinder 221 of the fixed interface 22. The hook 91 can rotate around the first cylinder 221. Anti-falling protrusions 93 are arranged inside the hook 91. The anti-falling protrusions 93 can form interference with the first cylinder 221, thereby preventing the first cylinder 221 from detaching from the hook 91.

Preferably, the first cylinder 221 and the hook 91 are made of a magnetic material and are thus able to attract each other. By a magnetic attraction of this magnetic material, the connection of the first cylinder 221 and the hook 91 become easy. Certainly, it is also possible that one of the first cylinder and the hook 91 is made of a magnetic material, and the other is made of a metal material that can be magnetically attracted, such as iron, cobalt or nickel. In this way, the first cylinder may also be connected to the hook 91.

When the patient normally uses a negative pressure ventilation apparatus for therapy, in an event of encountering a situation that the therapy needs to be interrupted, for example, the patient needs to go to a bathroom, the patient needs to undergo a medical examination, an air pressure in the negative pressure face mask assembly caused by a mechanical failure of the negative pressure generation apparatus changes suddenly, or when the patient suddenly has a stress reaction, the negative pressure face mask assembly and the headband can be quickly removed from the patient's head by the quick-disassembly structure 9, thereby providing a convenience for the patient.

In other examples, the quick-disassembly structure 9 is magnetically connected. For example, the quick-disassembly structure 9 includes two parts that are magnetically attached to each other, one part of which is connected to the first connecting band 53, and the other part is connected to the frame 2. When the two parts approach each other, the first connecting band 53 is connected to the frame 2 by means of magnetic attraction. In an event of emergency, the patient is able to separate the two parts of the quick-disassembly structure 9 at any time.

In one example, two connecting bands are arranged on the same side of the headband body. One of the two connecting bands is located behind the patient's ears and the other in front of the patient's ears.

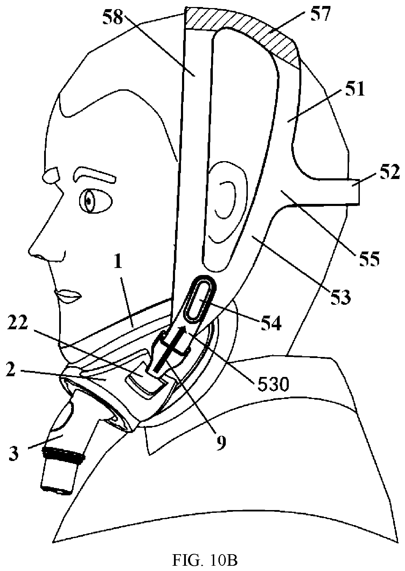

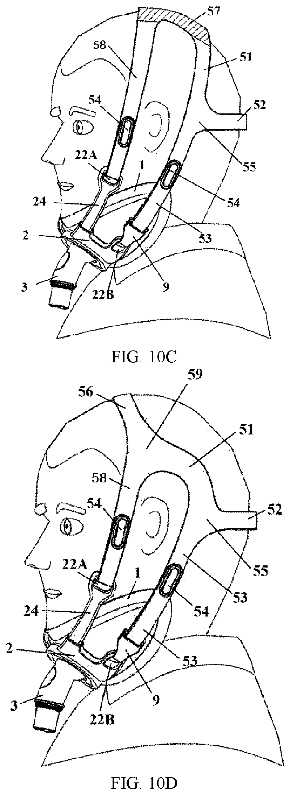

As shown in FIG. 10B and FIG. 10C, the first connecting band 53 is located behind the patient's ears. The second connecting band 58 is led out from a part of the headband body located on the head top of the patient. The second connecting band 58 extends toward the patient's underjaw and passes over the front of the patient's ears.

In this way, the second connecting band 58 and the first connecting band 53 reach the negative pressure face mask assembly through the front and rear of the patient's ears respectively, and are, for example, both connected to the frame 2. In this way, the weight of the negative pressure face mask assembly is spread over the two connecting bands, thereby avoiding an excessive force on the patient's head and reducing the wearing comfort of the headband.

Moreover, the first connecting band 53 and the second connecting band 58 make the force on the head top of the patient head more evenly balanced.

It should be noted that two first connecting bands 53 and two second connecting bands 58 are both provided. The two first connecting bands 53 and the two second connecting bands 58 are symmetrically arranged with respect to the first sub-band 51.

In one example, parts of the two connecting bands that are located under the patient's ears are connected together. A headband fixing structure 54 is arranged on a part where the two connecting bands are connected.

As shown in FIG. 10B, the first connecting band 53 and the second connecting band 58 are connected together under the patient's ears. A part 530 where the first connecting band 53 and the second connecting band 58 are connected passes through the strip-shaped hole of the quick-disassembly structure 9 and is bent and then fixed through the headband fixing structure 54.

In this example, since the two connecting bands are connected together, the negative pressure face mask assembly may be connected to, for example, the frame 2 through a connecting structure, such as the quick-disassembly structure 9. This makes the connection of the headband simple.

In other examples, as shown in FIG. 10C, the two connecting bands are arranged side by side and connected to the negative pressure face mask assembly, e.g., the frame 2, respectively. The quick-disassembly structure 9 is connected to the first connecting band 53. The first connecting band 53 is connected to the first fixed interface 22A of the frame 2 through the quick-disassembly structure 9. The second connecting band 58 is directly connected to the second fixed interface 22B of the frame 2. The headband fixing structure 54 is respectively arranged on the first connecting band 53 and the second connecting band 58, to facilitate the adjustment of the effective lengths of the first connecting band 53 and the second connecting band 58.

In this example, since the two connecting bands are arranged side by side, the lengths of the two connecting bands can be adjusted separately according to the patient's head structure, making the headband more comfortable to wear.

In one example, as shown in FIG. 10D, a connecting part between the connecting band in front of the patient's ears and the headband body is located above the patient's ears. A part (e.g., a first connecting part 55) of the first connecting band 53 that is connected to the headband body is located behind the patient's ears. A part (e.g., a second connecting part 59) of the second connecting band 58 that is connected to the headband body is located above the patient's ears.

As shown in FIG. 10D, the headband body is of a circular structure. The headband body includes a first sub-band 51, a second sub-band 52 and a third sub-band 56. The first sub-band 51 is located between the second sub-band 52 and the third sub-band 56. One end of the first sub-band 51 and one end of the second sub-band 52 are connected at the first connecting part 55. The other end of the first sub-band 51 and the third sub-band 56 are connected at the second connecting part 59. Two first sub-bands 51 are provided and symmetrically arranged with respect to the second sub-band 52. The second sub-band 52 is opposite to the third sub-band 56. The second sub-band 52 is located at the occipital bone of the patient. The third sub-band 56 is located at the head top of the patient. The first connecting band 53 is connected to the first sub-band 51 and the second sub-band 52 at the first connecting part 55. The second connecting band 58 is connected to the first sub-band 51 and the third sub-band 56 at the second connecting part 59.

In this example, since the second connecting part 59 is located above the patient's ears, a sufficient tension can be provided to the front of the negative pressure face mask assembly. Since the first connecting part 55 is located behind the patient's ears, a sufficient tension can be provided to the rear of the negative pressure face mask assembly. In this way, the negative pressure face mask assembly has a better sealing effect with the patient.

In one example, the headband body is of a trapezoidal or triangular shape. As shown in FIG. 10E, the headband body is of a trapezoidal shape, such as an isosceles trapezoid. The third sub-band 56 is an upper bottom. The second sub-band 52 is a lower bottom. The two first sub-bands 51 are waists of the trapezoid. The upper bottom of this trapezoid is longer than the lower bottom.

In other examples, the headband body is in a shape of a triangle, such as a regular triangle or an inverted triangle.

The above shape allows for a large contact area between the headband body and the patient's head. The force on the patient's head is more even, which makes the headband more comfortable to wear.

Certainly, in other examples, the headband body can also take on other shapes.

In one example, the headband body consists of four sub-bands that are connected together end to end. The four sub-bands form a quadrilateral. A plurality of connecting bands are formed by extending outward from both ends of two opposite sub-bands.

As shown in FIG. 10E, the two first connecting bands 53 are formed by extending outward from both ends of the second sub-band 52. The two second connecting bands 58 are formed by extending outward from both ends of the third sub-band 56. The direction of the two first connecting bands 53 is consistent with that of the second sub-band 52. The direction of the two second connecting bands 58 is consistent with that of the third sub-band 56.

In this way, a force received by the two first connecting bands 53 is directly transferred to the second sub-band 52; and a force of the two second connecting bands 58 can be directly transferred to the third sub-band 56, so that the headband is deformed due to the imbalance in the hands when the negative pressure face mask assembly is tightened can be avoided.

Moreover, a tightening force of the negative pressure face mask assembly can be transferred directly to the first sub-band 51 and the third sub-band 56, so that the negative pressure face mask assembly can more easily form a seal with the patient's skin.

In one example, a telescopic structure is arranged in at least one of the four sub-bands.

The telescopic structure can change the length of the sub-band in which it is located. For example, as shown in FIG. 10E, the telescopic structure is arranged on the second sub-band 52. The second sub-band 52 is divided into two parts. The telescopic structure includes a connecting frame k01 and the headband fixing structure 54 mentioned above. The connecting frame k01 is in a shape of a Chinese character "Ri(Sun)" character or a Chinese character "Kou(Mouth)". One of the two parts is fixedly connected to the connecting frame k01, and the other is bent after passing through the connecting frame k01 and fixed by the above-mentioned headband fixing structure 54. The telescopic structure allows the length of the sub-band to be adjusted according to the shape of the patient's head, so that the patient can wear the headband comfortably.

In other examples, the telescopic structure may also be arranged on the four sub-bands. Certainly, the telescopic structure is not limited to the above embodiments, and may be selected by those skilled in the art according to actual needs.

In one example, an air permeable layer 57 is arranged on a part of the headband body opposite the head top of the patient.

As shown in FIG. 10B-FIG. 10C, for example, the air permeable layer 57 is made of plastic, silicone, rubber or other materials. The air permeable layer 57 is provided with through holes which are distributed. The through holes can make outside air contact with the head top of the patient, avoiding swelling, ulcers and other phenomena on the head top of the patient in the case of long-term wearing.

Certainly, the material of the air permeable layer 57 is not limited to the above embodiments, and may be selected by those skilled in the art according to actual needs.

Another embodiment of the present disclosure provides a negative pressure ventilation apparatus. This negative pressure ventilation apparatus includes a negative pressure face mask assembly and the above-mentioned headband, wherein the negative pressure face mask assembly is connected to the headband.

As shown in FIG. 10A, FIG. 11A, and FIG. 11B, the negative pressure face mask assembly includes a liner assembly 1, an elbow 3, and a frame 2. The elbow 3 is connected to the liner assembly 1. The frame 2 is sleeved outside the elbow 3 and is located in front of the liner assembly 1. A fixed interface 22 is respectively formed in left and right sides of the frame 2. A first cylinder 221 is arranged on one side of each fixed interface 22. A hook 91 is arranged at the other end of the quick-disassembly structure 9. The hook 91 is hooked on the first cylinder 221.

The negative pressure ventilation apparatus has characteristics of good sealing effect and comfortable wear.

### <IX. Self-sealing apparatus>

Since a pressure intensity in the negative pressure chamber of the negative pressure therapy apparatus is less than an outside atmospheric pressure intensity, the outside atmosphere exerts a pressure on the liner assembly 1 towards the patient. This pressure prevents the negative pressure face mask assembly from falling off the patient's neck, ensuring the sealing stability of the negative pressure face mask assembly. Although the headband applied to the negative pressure therapy apparatus can play a certain role in a gradual sealing of the negative pressure face mask assembly during use, wearing the headband increases the patient's sense of restraint surrounded by the headband. In order to solve the above problems, an embodiment of the present disclosure provides a self-sealing apparatus applied to a negative pressure therapy apparatus.

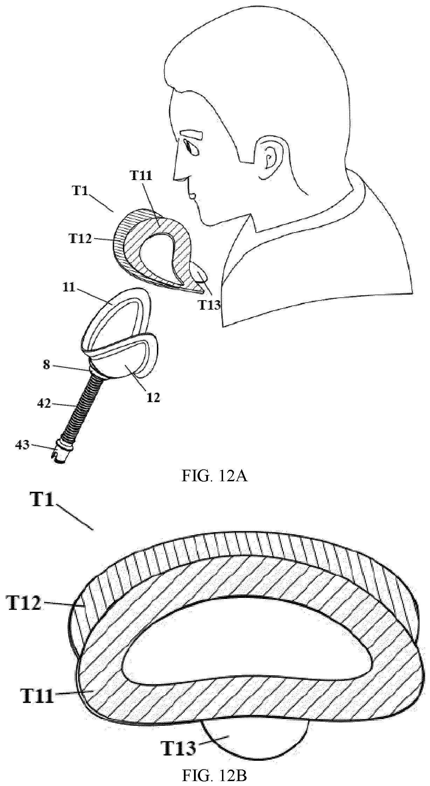

As shown in FIG. 12A, the self-sealing apparatus applied to the negative pressure therapy apparatus includes a self-adhesive film T1. The self-adhesive film T1 is made of a biocompatible material, and has adhesive properties. During use, the self-adhesive film T1 is adhered between the patient's neck and the liner assembly 1. One side of the self-adhesive film T1 is bonded to the skin of the underjaw and the neck of the patient, and the other side is adhered to the outer surface of the liner assembly 1. In this way, the negative pressure face mask assembly is adhered to the patient's underjaw and neck.

Specifically, as shown in FIG. 12A and FIG. 12B, the self-adhesive film T1 includes a first adhesive film T11, and both sides of the first adhesive film T11 each have an adhesive coating T2. A make-way hole is formed in a central zone of the first adhesive film T11. A shape of the first adhesive film T11 is consistent with a shape of the soft rubber part 11 of the liner assembly 1. During use, the first adhesive film T11 is bonded to the skin of the patient's neck, and the other side is adhered to the outer surface of the soft rubber part 11.

Optionally, the self-adhesive film T1 further includes a second adhesive film T12. The second adhesive membrane T12 has an adhesive coating T2 on a side opposite the patient's skin, while the adhesive coating is not arranged on a side away from the patient's skin. The second adhesive film T2 surrounds the first adhesive film T11 and is connected to the first adhesive film T11. The second adhesive film at least partially protrudes outward from an outer edge of the first adhesive film. The second adhesive film T12 can be adhered to the patient's skin at the outer edge of the first adhesive film T11, which further enhances an adhesive strength and sealability between the self-adhesive film T1 and the patient's skin.

It should be noted that human skin has many sweat pores, and will sweat or discharge secretions with the human metabolism. Both sweat and secretions reduce the adhesion of the self-adhesive film T1 to the patient's skin. A structure of the underjaw of the patient changes greatly, and a curvature of the skin at the underjaw varies greatly. Therefore, poor adhesion between the self-adhesive film T1 and the patient's skin is more likely to occur, resulting in air leakage. Preferably, the second adhesive film T12 is located in the patient's underjaw, as shown in FIG. 12A. The second adhesive film T2 can significantly enhance the adhesive strength and sealability between the self-adhesive film T1 and the patient's underjaw.

Optionally, the self-adhesive film T1 further includes a non-adhesive film T13. The non-adhesive film T13 is not coated on a side opposite the patient's skin or on a side away from the patient's skin. The non-adhesive film T13 is fixed at an edge of the first adhesive film T11 and/or the second adhesive film T12. The non-adhesive film T13 provides an access point for an operator to adhere the self-adhesive film T1. In the case of adhering the self-adhesive film T1, the operator takes the non-adhesive film T13 with his hand to operate. The non-adhesive film T13 allows the operator to adhere the self-adhesive film T1 to the patient's neck or the liner assembly 1 or remove it from the patient's neck or the liner assembly 1.

In order to ensure medical treatment and public health, the self-adhesive film T1 is usually used disposably. In order to increase the adhesion of the self-adhesive film T1 to the patient's skin, before the self-adhesive film T1 is adhered, a drug coating may also be applied to the skin of the underjaw and neck of the patient. On the one hand, the drug coating can improve a roughness of the sweat pores on the patient's skin and increase the adhesion ability of the self-adhesive film T1 to the patient's skin; and on the other hand, it can reduce an irritation of the adhesive coating of the self-adhesive film T1 to the patient's skin and prevent an occurrence of eczema and other skin diseases during long-term wearing.

Optionally, the adhesive coating T2 may be, but not limited to, polyacrylic resin, a polyurethane adhesive, a synthetic adhesive, a UV adhesive, etc. The adhesive coating T2 is attached to the first adhesive film T11 and/or the second adhesive film T12 during adhesion by means of glue dispensing, manual coating, spraying, rolling, pressure dipping, vacuum dipping, etc.

### <X. Air permeable liner>

The negative pressure therapy apparatus is often used to treat diseases such as obstructive sleep apnea (OSA) and chronic obstructive pulmonary emphysema (COPD), often requiring a long-term wearing of the negative pressure face mask assembly. Because the soft rubber part 11 of the liner assembly 1 compresses the patient's skin for a long time, a normal physiological metabolism of the skin in contact with the soft rubber part 11 is hindered. Bacteria can easily grow between the soft rubber part 11 and the patient's skin, resulting in skin diseases such as erythema and pressure ulcers. A main reason for skin diseases is that water vapor produced by the patient's skin metabolism is bound by hydrophilic molecules in the soft rubber part 11 and cannot volatilize into air, resulting in long-term wetness of the skin in contact with the soft rubber part 11 and a breeding of a large number of anaerobic bacteria. Increasing the air permeability of the soft rubber part 11 at a position in contact with the patient's skin can greatly avoid water vapor retention, reduce the growth of bacteria, and effectively solve the skin erythema and pressure ulcers caused by long-term wearing. However, an increased air permeability can lead to a sealing failure between the soft rubber part 11 and the patient's skin.

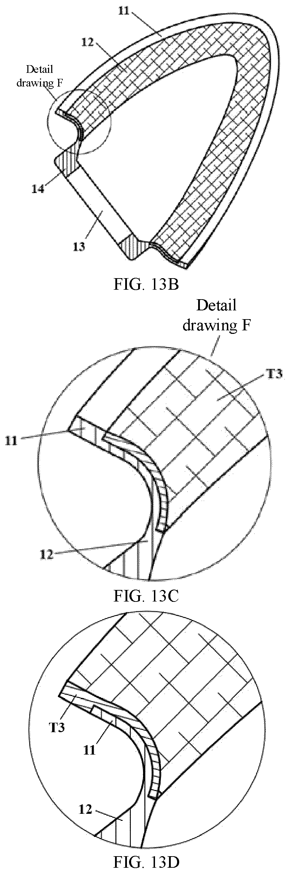

In view of the above technical problems, an embodiment of the present disclosure provides a liner assembly 1 applied to a negative pressure therapy apparatus. The liner assembly includes an air permeable layer T3 as shown in FIG. 13A- FIG. 13C. It should be noted that FIG. 13A-FIG. 13C take a single-layer liner assembly as an example, and the air permeable layer T3 is also applicable to a double-layer liner assembly and a multi-layer liner assembly.

The air permeable layer T3 may be connected to the soft rubber part 11 or directly to the cup 12.

As mentioned before, the soft rubber part 11 may be selected from a soft material, such as silicone, TPE, or TPU. The soft rubber material is usually a non-air-permeable material. In this example, an air permeable layer T3 is arranged at a position of the soft rubber part 11 in contact with the patient's skin. Optionally, the air permeable layer T3 is a sponge or textile material. The textile material may be a material such as natural cotton, down feather or natural silk; a textile material formed by a natural material or artificial fibers by means of weaving, knitting, coating, crocheting or bonding (e.g., by means of chemical, mechanical or hot solvent treatment); or a multi-layer textile formed jointly by the plurality of textile materials. The air permeable layer T3 may also include a non-textile material, such as elastically stretchable pore-forming polyurethane foams; or a textile containing a plastic material, such as a nylon and polyester blend, a nylon and spandex blend, a polyester and spandex blend, a nylon, polyester and spandex blend, microfiber or a polyurethane blend, etc.

Optionally, the air permeable layer T3 may be arranged around the liner assembly 1 in all areas of the liner assembly 1 in contact with the patient's skin. Alternatively, the air permeable layer T3 is only distributed in areas with a strong sense of oppression.

Optionally, the soft rubber part 11 has a thickness of 0.1 mm to 1.0 mm. Preferably, the soft rubber part 11 has a thickness of 0.15 mm to 0.5 mm. The air permeable layer T3 has a thickness of 0.1 mm to 1.0 mm.

An air permeability of the air permeable layer T3 enables water vapor generated by the skin metabolism at a position in contact with the air permeable layer T3 to be discharged, to prevent the growth of bacteria between the air permeable layer and the patient's skin, and avoid skin diseases such as erythema and pressure ulcers.

Furthermore, the soft rubber part 11 located outside the air permeable layer T3 has a certain sealing effect, preventing the occurrence of air leakage.

Moreover, the air permeable layer T3 has a good contact with the patient's skin, which increases the wearing comfort of the liner assembly 1.

A connection mode between the air permeable layer T3 and the non-air-permeable soft rubber part 11 can adopt mechanical crimping, ultrasonic welding, laser welding, hot melting, etc. An adhesive may also be adopted for adhesion. The adhesive includes, but is not limited to, polyacrylic resin, a polyurethane adhesive, a synthetic adhesive, a UV adhesive, etc. The adhesion may be performed by means of glue dispensing, manual coating, spraying, rolling, pressure dipping, vacuum dipping, etc.

In one example, as shown in FIG. 13B-FIG. 13C, the air permeable layer T3 may be stacked with the soft rubber part 11. The air permeable layer T3 is located on a side of the soft rubber part close to the patient's skin. The air permeable layer T3 has a width smaller than that of the soft rubber part 11. The air permeable layer T3 is at least embedded into the soft rubber part 11. The edge of the soft rubber part 11 is flush with the air permeable layer T3, so that both the soft rubber part 11 and the air permeable layer T3 are in contact with the patient's skin. In this way, the air permeability of the liner assembly 1 can be maintained, and the air leakage of the liner assembly 1 can be effectively avoided.

In one example, as shown in FIG. 13D, a stepped structure is formed at the edge of the soft rubber part 11. The air permeable layer T3 forms a stepped structure. The stepped structure of the air permeable layer T3 and the stepped structure of the soft rubber part 11 are complementary, so that a connecting structure formed by the soft rubber part 11 and the air permeable layer T3 makes a smooth transition at the junction therebetween.

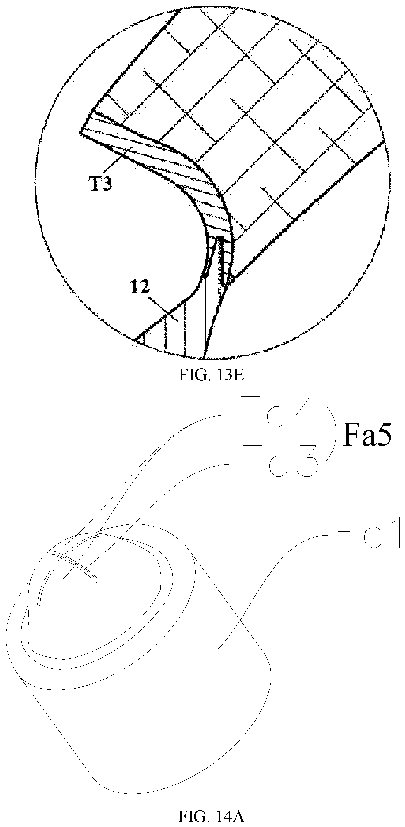

In one example, as shown in FIG. 13E, a tapered protrusion structure is formed at the edge of the cup 12. A recessed structure with a tapered section is formed at the edge of the air permeable layer T3. The protrusion structure is inserted into the recessed structure and adhered with an adhesive. In this example, the protrusion structure and the recessed structure increase a connection area between the cup 12 and the air permeable layer T3, thus making the connection therebetween firmer. In this example, the liner assembly 1 is not provided with a soft rubber part 11, and the air permeable layer T3 is directly connected to the cup 12.

### <XI. Safety valve>

A negative pressure pump in the negative pressure therapy apparatus may cause an excessive suction force when it encounters a mechanical failure or misoperation, which may cause damages to the patient's skin. In view of this, there is a need to provide a solution to adjust the air pressure in the negative pressure therapy apparatus when the suction force is too large, thereby avoiding skin damages to the patient's skin.

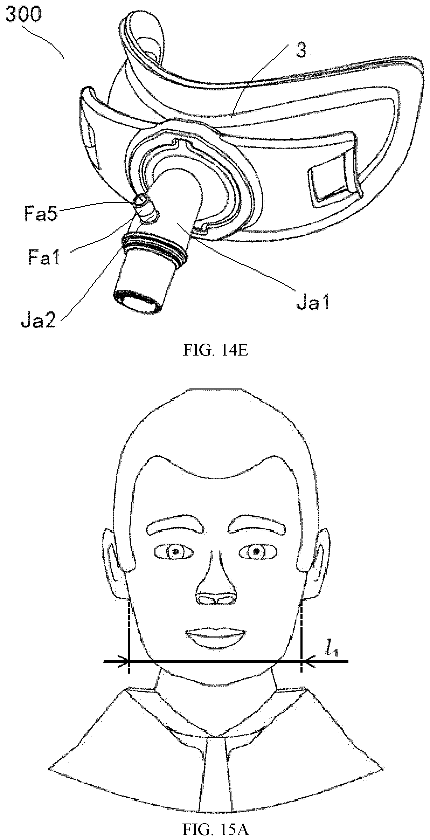

An embodiment of the present disclosure provides a negative pressure face mask 300. The negative pressure face mask 300 is applied to the negative pressure therapy apparatus. As shown in FIG. 14E, the negative pressure face mask 300 includes a main body 3 and a safety valve connected to the main body 3. The negative pressure face mask 300 is used to cover the mouth of the patient. As shown in FIG. 14A, the safety valve includes a mounting cylinder Fa1 and a sealing member Fa5. The mounting cylinder Fa1 includes an internal channel with an internal port Fa12 and an external port Fa13. The sealing member Fa5 is arranged at the external port Fa13.

For example, the mounting cylinder Fa1 is connected to a component where a safety valve needs to be arranged. The external port Fa13 refers to an outward-facing port of the safety valve in a mounted state. The internal port Fa12 refers to a port of the safety valve which faces the mounted component. The internal channel is communicated with the component on which the safety valve is mounted.

As shown in FIG. 14A, the sealing member Fa5 includes a self-sealing sheet Fa4 and a sealing opening Fa3. The self-sealing sheet Fa4 protrudes outward from the internal channel. The sealing opening Fa3 is formed in the self-sealing sheet Fa4. The self-sealing sheet Fa4 seals the sealing opening Fa3, thereby sealing the internal channel. The self-sealing sheet Fa4 opens the sealing opening Fa3 when an air pressure difference between the inside and the outside of the internal channel exceeds a critical pressure.

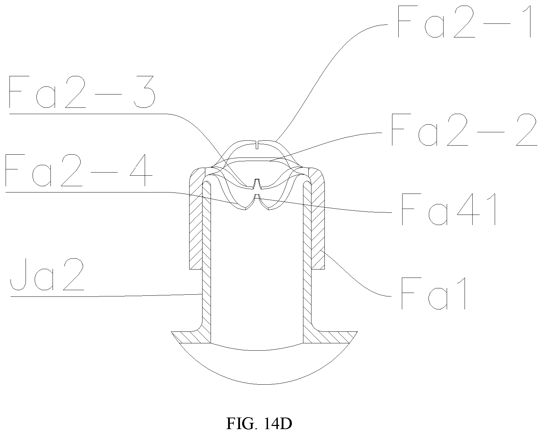

As shown in FIG. 14D, the self-sealing sheet Fa4 is in a critical position when the critical pressure is reached. The internal channel is communicated with a component connected to the safety valve. The air pressure inside the component is negative relative to the outside of the internal channel. The air pressure in the inner channel decreases, and the air pressure difference between the inside and the outside of the inner channel gradually increases. At this time, the self-sealing sheet Fa4 gradually deforms to a side of the internal channel, as shown in Fa2-1 to Fa2-4 in FIG. 14D. When the air pressure difference reaches a critical pressure (e.g., -12 hPa), the sealing sheet changes from an outward protruding state (as shown by Fa2-1 in FIG. 14D) to a straight state, as shown by Fa2-2 in FIG. 14D. In the straight state, a mutual extrusion force between the self-sealing sheets Fa4 is relatively large, so the sealing opening Fa3 can still be kept in a sealed state. The sealing opening Fa3 is opened inward when the air pressure difference exceeds the critical pressure. For example, the self-sealing sheet Fa4 can be turned inward from a straight state to open the sealing opening Fa3, thereby allowing outside air to enter the component. At this time, the air pressure inside the component gradually increases, and the air pressure difference between the inside and the outside gradually decreases. During the reduction of the air pressure difference (e.g., -11 hPa), the self-sealing sheet Fa4 is able to return to an outward protruding state, so that the sealing opening Fa3 is sealed, as shown by Fa2-1 in FIG. 14D.

In the embodiment of the present disclosure, the sealing member Fa5 includes a self-sealing sheet Fa4 and a sealing opening Fa3. Moreover, the self-sealing sheet Fa4 protrudes outward from the internal channel. A negative pressure is formed in the internal channel. Under the action of an air pressure difference between the inside and the outside, the self-sealing sheet Fa4 is extruded inward. At this time, the sealing opening Fa3 is better sealed under mutual extrusion of the self-sealing sheets Fa4. When the air pressure difference exceeds a critical pressure, the self-sealing sheet Fa4 can be elastically deformed under the action of pressure, and then the sealing opening Fa3 opens inward to allow external air to enter, thereby ensuring that the air pressure in the component in which the safety valve is mounted does not exceed the critical pressure. The safety valve can effectively protect the patient.

The safety valve has a simple structure, is easy to machine, and can achieve a function of automatic opening when the air pressure difference exceeds a critical air pressure and automatic closing when the air pressure difference does not exceed the critical air pressure without requiring electromagnetic drive or pressure drive.

Moreover, since the self-sealing sheet Fa4 can be elastically deformed to open the sealing opening Fa3, a detection probe can be inserted through the safety valve to detect the air pressure, temperature and other parameters inside the component where the safety valve is located. After the probe is pulled out, the self-sealing sheet Fa4 returns to a position where the sealing opening Fa3 is closed, thereby ensuring that the sealing opening Fa3 is leak-proof.

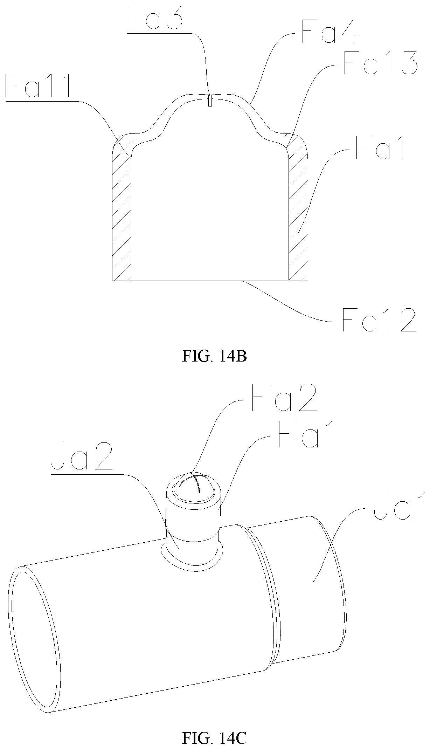

In one example, as shown in FIG. 14A and FIG. 14B, a thickness of a sealing sheet Fa5 is constructed in such a way that it gradually decreases from an edge to a center.

In this example, the edge of the sealing member Fa5 needs to be connected to the mounting cylinder Fa1, and a larger thickness can ensure a high connection strength therebetween. The thickness of the sealing member Fa5 decreases gradually from the edge to the center. A smaller thickness can improve an elastic deformation ability of the self-sealing sheet Fa4, such that the self-sealing sheet Fa4 can easily deform and return to an initial position.

For example, the sealing member Fa5 has a thickness of 0.2 mm-0.8 mm. For example, the sealing member Fa5 at an edge position has a thickness of 0.8 mm in order to ensure a connection strength with the mounting cylinder Fa1. The sealing member Fa5 has a thickness of 0.2 mm in the center in order to ensure the elastic deformation of the self-sealing sheet Fa4.

Certainly, the thickness of the sealing member Fa5 is not limited to the above embodiments, and may be selected by those skilled in the art according to actual needs.

Optionally, the mounting cylinder Fa1 is an annular sleeve. The mounting cylinder Fa1 has a wall thickness of 0.6 mm-2.0 mm. Within this range, the mounting cylinder Fa1 has a high structural strength and a good durability.

In one example, as shown in FIG. 14A and FIG. 14B, the mounting cylinder Fa1 and the sealing member Fa5 form an integral structure. For example, the mounting cylinder Fa1 and the sealing member Fa5 are integrally molded by means of injection molding. In this way, the connection between the mounting cylinder Fa1 and the sealing member Fa5 is firmer.

In other examples, the mounting cylinder Fa1 and the sealing member Fa5 may also be fixed together by means of adhesion or hot melt connection.

Certainly, a make of the safety valve is not limited to the above embodiments, and may be selected by those skilled in the art according to actual needs.

In one example, as shown in FIG. 14A and FIG. 14B, an edge of the sealing member Fa5 is connected to an inner wall of the external port Fa13. In this example, the mounting cylinder Fa1 is sleeved on the outside of the connected component generally. Because the edge of the sealing member Fa5 is located on the inner wall of the external port Fa13, the sealing member Fa5 can at least partially cover an end surface of the connected component, so a sealing effect between the mounting cylinder Fa1 and the connected component can be increased.

Moreover, when the self-sealing sheet Fa4 is deformed, because the sealing member Fa5 is connected to the inner wall of the external port Fa13, the mounting cylinder Fa1 can form a radial outward tensile force on the self-sealing sheet Fa4, rather than a shear force formed as the sealing member Fa5 is arranged on the end surface of the external port Fa13. In this way, the connection between the mounting cylinder Fa1 and the sealing member Fa5 is firmer.

In one example, the self-sealing sheet Fa4 is an elastic sheet and can be self-sealed by the sealing opening Fa3 under its own elastic force to seal the internal channel.

For example, the self-sealing sheet Fa4 is elastic and made of rubber, silicone, etc. A plurality of elastic sheets extrude the sealing opening Fa3 with each other due to their own elastic force, to realize the sealing of the sealing opening Fa3. Each elastic sheet may be in a shape of a triangle, a fan, etc.

Certainly, the shape of the elastic sheet is not limited to the above embodiments, and may be selected by those skilled in the art according to actual needs.

In one example, as shown in FIG. 14A and FIG. 14B, the sealing member Fa5 includes a hook-shaped elastic sheet. Any radial cross-section of the mask-shaped elastic sheet that passes through a center of the mask has a cambered shape. A slit is formed in the mask-shaped elastic sheet to form the self-sealing sheet Fa4, and the slit is used as the sealing opening Fa3.

In this example, due to an elastic extrusion of a cambered profile of the mask-shaped elastic sheet, the self-sealing sheet Fa4 formed by the slit can seal the sealing opening Fa3 under the elastic extrusion of the cambered profile.

Optionally, the mask-shaped elastic sheet may be a conical elastic sheet, a vault-shaped elastic sheet, a hemispherical elastic sheet, etc. The mask-shaped elastic sheet protrudes from the internal channel, so that the sealing opening Fa3 can be extruded to ensure good sealability when the air pressure difference between the inside and outside is formed.

The slit may be one or more. When there are a plurality of slits, it may be cross-shaped slits, *-shaped slits, etc. The plurality of slits form a plurality of petal-shaped self-sealing sheets Fa4. The adjacent self-sealing sheets Fa4 seal the sealing opening Fa3 by extruding each other.

In one example, as shown in FIG. 14A and FIG. 14C, the plurality of slits are intersected in the center of the mask-shaped elastic sheet, and evenly distributed in a circumferential direction.

Because the sizes and shapes of the plurality of self-sealing sheets Fa4 are consistent, the deformation of the plurality of self-sealing sheets Fa4 can be consistent. Because the slits eliminate a connection stress of each self-sealing sheet Fa4, each self-sealing sheet Fa4 tends to maintain the mask-shaped structure (e.g., a vault-shaped structure) as a whole during the deformation process.

Moreover, when the sealing opening Fa3 is opened, a part of the self-sealing sheet Fa4 located in the center remains a protruding state in a direction opposite to an opening direction of the sealing opening Fa3, as shown by Fa41 in FIG. 14D. At this position, the self-sealing sheet Fa4 can keep the sealing opening Fa3 from being opened too large, to avoid the inability to close the sealing opening Fa3 when the air pressure difference is less than the critical air pressure after excessive opening.

Moreover, this protruding state can prevent the central part from turning inward and no longer protruding outward.

Certainly, in other examples, the intersection of the plurality of slits may also be outside the center of the sealing member Fa5.

For example, each slit has a width of 0.05 mm-0.15 mm. Within this range, the sealing opening Fa3 is easy to open, and the plurality of self-sealing sheets Fa4 can quickly seal the sealing opening Fa3.

In one example, as shown in FIG. 14C, FIG. 14D and FIG. 14E, the negative pressure face mask also includes a tri-branch tube. The tri-branch tube is connected to the main body 3. The tri-branch tube includes a main pipe Ja1 and a branch pipe Ja2 located on a side of the main pipe Ja1. The branch pipe Ja2 is communicated with the main pipe Ja1. A diameter of the branch pipe Ja2 is generally smaller than that of the main pipe Ja1 to ensure that a flow rate of the main pipe Ja1 is large.

The mounting cylinder Fa1 is sleeved outside the branch pipe Ja2. The mounting cylinder Fa1 is fixedly connected to the branch pipe Ja2 and forms a seal by interference fitting with the branch pipe Ja2. The mounting cylinder Fa1 expands and deforms under a support of the branch pipe Ja2, and stores a deformation stress in a direction of the branch pipe Ja2. Due to the deformation stress, the safety valve is reliably mounted on the branch pipe Ja2.

Alternatively, the mounting cylinder Fa1 is fixedly connected to the branch pipe Ja2 by an adhesive and forms a seal. During use, the tri-branch tube is mounted in other pipes.

The tri-branch tube is made of plastic, metal, etc. The plastic may be, but is not limited to, PC, PP, PA, etc.

According another embodiment of the present disclosure, a negative pressure therapy apparatus is provided. The negative pressure therapy apparatus includes an apparatus body and the negative pressure face mask described above. The negative pressure face mask is arranged on the apparatus body. For example, a main pipe Ja1 of a tri-branch tube is mounted to an airway pipe of the negative pressure therapy apparatus. For example, between the elbow and the hose or between the elbow and the air extraction pipe, etc.

In one example, the negative pressure therapy apparatus includes a negative pressure generation apparatus. A vacuum pump is arranged in the negative pressure generation apparatus. As shown in FIG. 14E, the vacuum pump is used to vacuumize the negative pressure face mask 300, to form a negative pressure in the negative pressure face mask 300. When an air pressure difference between the inside and the outside of the negative pressure face mask 300 exceeds a critical value, the safety valve is opened, so that external air is supplemented to the negative pressure face mask 300, to avoid the air pressure difference being too large, resulting in damages to a part of the patient wearing the negative pressure face mask 300.

When the air pressure difference between the inside and the outside of the negative pressure face mask 300 does not exceed the critical value, the safety valve is closed, to ensure that the patient can accept the treatment normally.

This negative pressure therapy apparatus has a characteristic of high safety factor.

### <XII. Model selection and make of negative pressure face mask assembly>

When in use, the negative pressure face mask assembly is worn around the patient's neck. However, for different patients, a curved profile of the neck varies greatly, and the negative pressure face mask assembly of a single model cannot meet the needs of different patients. In view of this, it is necessary to provide a new technical solution for model selection and customization applied to a negative pressure face mask assembly.

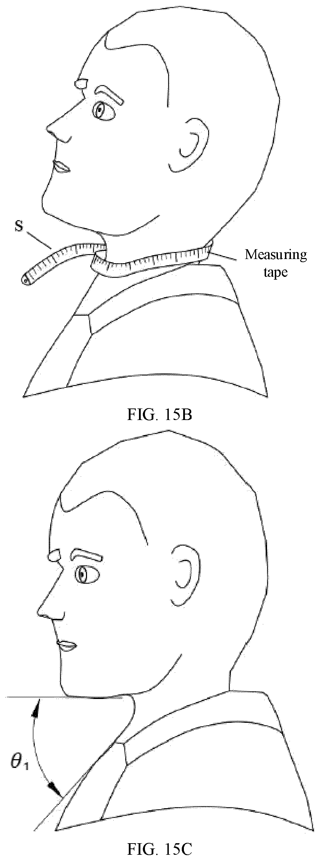

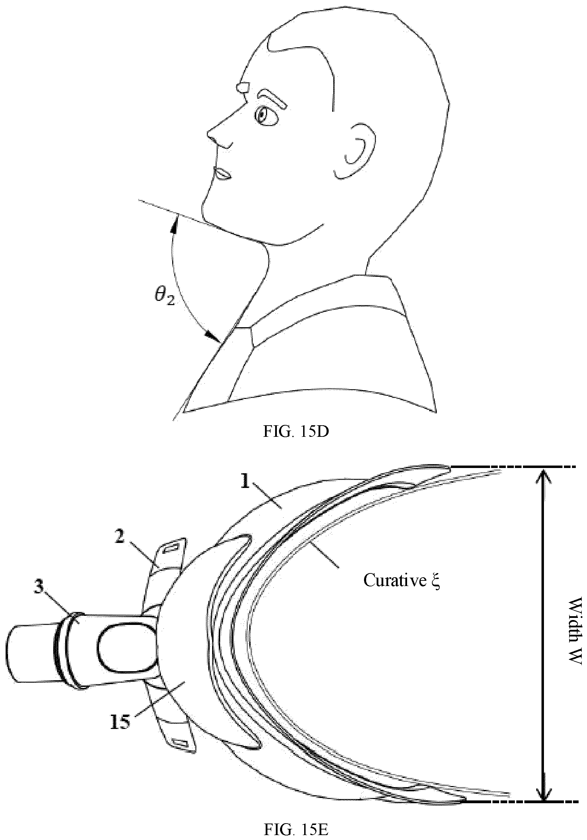

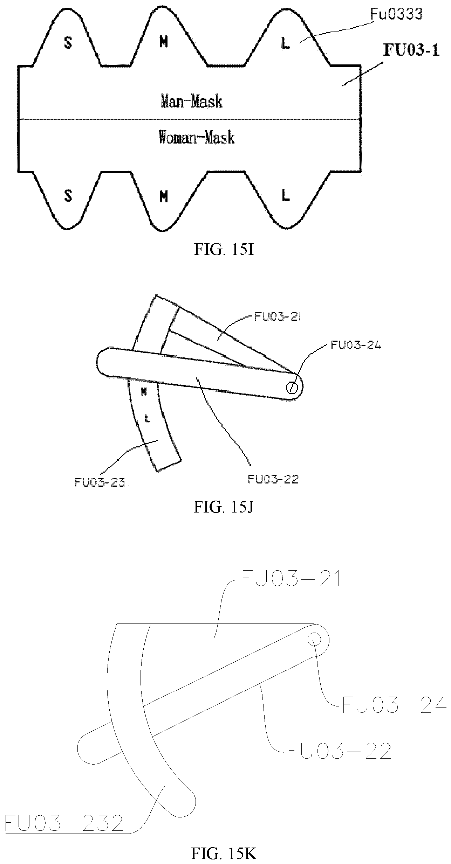

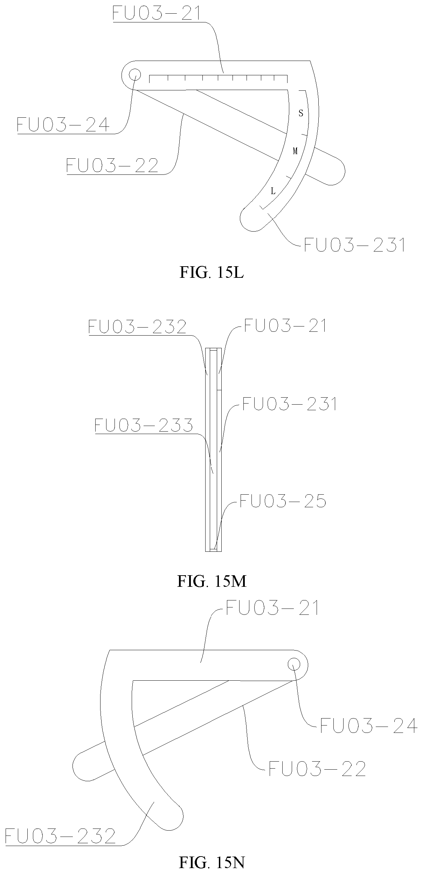

The model selection of the negative pressure face mask assembly is mainly determined according to the following parameters. The main parameters include: a straight-line distance l₁ between both earlobes of the patient, as shown in FIG. 15A; a perimeter of the patient's neck, as shown in FIG. 15B; a first included angle θ₁ between the underjaw and the neck of the patient at eye level, as shown in FIG. 15C; and a second included angle θ₂ between the underjaw and the neck of the patient when looking up, as shown in FIG. 15D.

The straight-line distance l₁ between both earlobes of the patient determines a width dimension W of the negative pressure face mask assembly of the liner assembly, as shown in FIG. 15E. The perimeter of the patient's neck determines a curvature ξ of the negative pressure face mask assembly of the liner assembly, as shown in FIG. 15E. The first included angle θ₁ and the second included angle θ₂ jointly determine a range of movement of the patient's head. Moreover, the first included angle θ₁ and the second included angle θ₂ further determine the size of the groove 15.

Generally, an interval distance l₁ between both earlobes of a person is 8 cm-15 cm. A perimeter S of the patient's neck is 25 cm-50 cm. Generally, the first included angle θ₁ between the underjaw and the neck of the patient at eye level is between 35° and 55°, and the first included angle θ₁ is 17° as a lower limit of an included angle between the underjaw and the neck during a nodding movement. Generally, an included angle θ₂ between the underjaw and the neck of a person when looking up is between 65° and 90°, and the second included angle θ₂ is taken an upper limit of an included angle between the underjaw and the neck during a head-raising movement.

For male and female patients, there are differences in the model selection of the negative pressure face mask assembly. Compared with female patients, male patients have more prominent prominentia laryngea due to an action of male hormones. However, a lower sealing point of the soft rubber part of the liner assembly 1 at the neck is often located near the prominentia laryngea, so the liner assembly 1 for the negative pressure face mask assembly suitable for male patients should have a liner curvature that conforms to the raised prominentia laryngea near the prominentia laryngea. For example, a notch is formed at the prominentia laryngea to make way for the prominentia laryngea of the male patient. In this way, the sealability and comfort of the male patient during the use of the negative pressure face mask assembly can be ensured. For the liner assembly 1 of the negative pressure face mask assembly for female patients, there is no need to arrange a make-way notch at the corresponding prominentia laryngea.

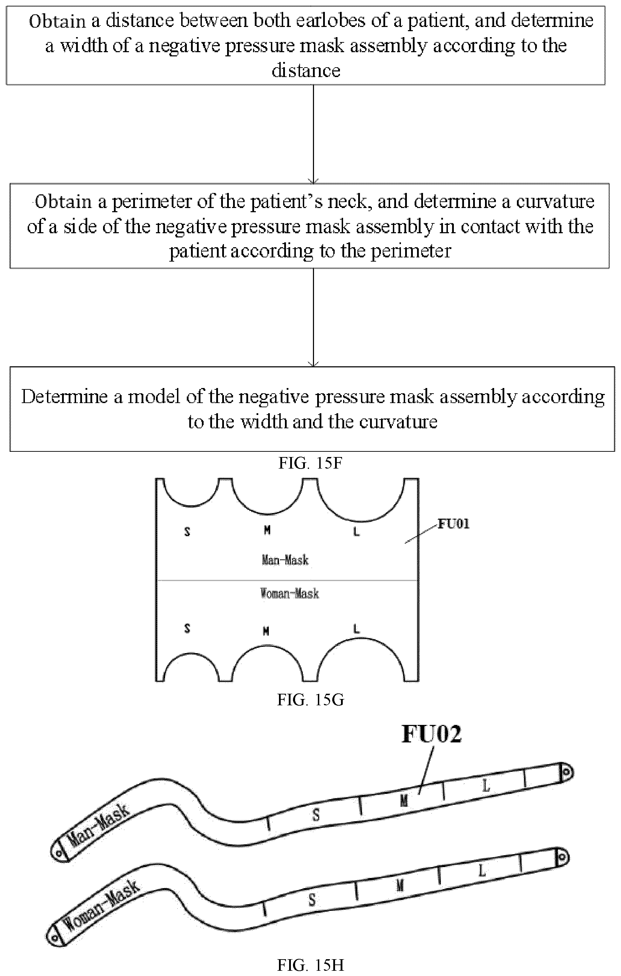

According to an embodiment of the present disclosure, a model selection method for a negative pressure face mask assembly is provided. As shown in FIG. 15F,the method includes the following steps.
obtaining an interval distance between both earlobes of a patient, and determining a width of the negative pressure face mask assembly according to the interval distance;
obtaining a perimeter of the patient's neck, and determining a curvature of a side of the negative pressure face mask assembly in contact with the patient according to the perimeter; and
determining a model of the negative pressure face mask assembly according to the width and the curvature.

For example, the interval distance between both earlobes of the patient can be determined by means of a model selection apparatus. In general cases, the width of the negative pressure face mask assembly should match the interval distance between both earlobes of the patient. The width of the negative pressure face mask assembly can be determined according to the interval distance between both earlobes of the patient. For example, the perimeter of the patient's neck is determined by a measuring tape. The curvature of the patient's neck can be determined according to the perimeter of the patient's neck. This curvature matches the curvature of the negative pressure face mask assembly, such that the negative pressure face mask assembly can form a good seal with the patient's neck.

In this example, the model of the negative pressure face mask assembly is determined by determining the interval distance between both earlobes of the patient of the perimeter of the patient's neck, such that the model selection of the negative pressure face mask assembly is more accurate.

In one example, the model selection method further includes: obtaining a first included angle formed between the underjaw and the neck of the patient when the patient views at eye level.

The model of the negative pressure face mask assembly is determined according to the width, the curvature and the first included angle.

In this example, an angle between the underjaw and the neck of the patient when the patient views at eye level can be determined by obtaining the first included angle. Each negative pressure face mask assembly can be adapted to an angle between the underjaw and the neck within a set range. The model of the negative pressure face mask assembly adapted to the patient can be determined more accurately according to the determination of the first included angle.

In one example, the model selection method further includes: obtaining a second included angle formed between the underjaw and the neck of the patient when the patient is looking up; and determining the model of the negative pressure face mask assembly according to the width, the curvature, the first included angle and the second included angle.

In this example, an angle between the underjaw and the neck of the patient when the patient is looking up can be determined by obtaining the second included angle. Each negative pressure face mask assembly can be adapted to an angle between the underjaw and the neck within a set range. The model of the negative pressure face mask assembly adapted to the patient can be determined more accurately according to the determination of the second included angle.

It should be noted that the ranges of the first and second included angles adapted to a certain model of the negative pressure face mask assembly are relative wide. The model of the negative pressure face mask assembly adapted to the patient can be further determined according to the first included angle and the second included angle.

In one example, the model of the negative pressure face mask assembly determined according to the interval distance is taken as a standard in the case that the model of the negative pressure face mask assembly determined according to the interval distance is inconsistent with a model of the negative pressure face mask assembly determined according to the perimeter.

In this example, a priority of the model selection according to the interval distance is higher than that of the model selection according to the perimeter. In general cases, the accuracy of the model selection according to the interval distance is higher than that of the model selection according to the perimeter. In this way, the accuracy of the model selection of the negative pressure face mask assembly can be ensured.

In one example, the model of the negative pressure face mask assembly determined according to the perimeter is taken as a standard in the case that the model of the negative pressure face mask assembly determined according to the perimeter is inconsistent with the model of the negative pressure face mask assembly determined according to the second included angle.

In this example, a priority of the model selection according to the perimeter is higher than that of the model selection according to the second included angle. In general cases, the accuracy of the model selection according to the perimeter is higher than that of the model selection according to the second included angle. In this way, the accuracy of the model selection of the negative pressure face mask assembly can be ensured.

In one example, the model of the negative pressure face mask assembly determined according to the second included angle is taken as a standard in the case that the model of the negative pressure face mask assembly determined according to the first included angle is inconsistent with the model of the negative pressure face mask assembly determined according to the second included angle.

In this example, a priority of the model selection according to the second included angle is higher than that of the model selection according to the second included angle. In general cases, the accuracy of the model selection according to the second included angle is higher than that of the model selection according to the second included angle. In this way, the accuracy of the model selection of the negative pressure face mask assembly can be ensured.

During model selection, when the models selected according to different parameters are inconsistent, the priorities of model selection are: model selection according to the interval distance, model selection according to the perimeter, model selection according to the second included angle, and model selection according to the first included angle. In this way, high accuracy of the model selection of the negative pressure face mask assembly can be ensured effectively.

In one example, the models of the negative pressure face mask assembly corresponding to the patient's gender include a first-type negative pressure face mask assembly, a second-type negative pressure face mask assembly, and a third-type negative pressure face mask assembly. A size of the first-type negative pressure face mask assembly is larger than that of the second-type negative pressure face mask assembly, and the size of the second-type negative pressure face mask assembly is larger than that of the third-type negative pressure face mask assembly. An interval distance between the first-type negative pressure face mask assemblies is larger than an interval distance between the second-type negative pressure face mask assemblies, and an interval distance between the second-type negative pressure face mask assemblies is larger than an interval distance between the third-type negative pressure face mask assemblies. A curative of the first-type negative pressure face mask assembly is greater than that of the second-type negative pressure face mask assembly, and the curative of the second-type negative pressure face mask assembly is greater than that of the third-type negative pressure face mask assembly.

In this example, there are a variety of models for negative pressure face mask assemblies. Moreover, each negative pressure face mask assembly has a different size to fit different patients.

In one example, the model selection method further includes: obtaining a gender of the patient, wherein under the condition that the patient is a female, an inner surface of the negative pressure face mask assembly forms a cambered surface structure; and under the condition that the patient is a male, the inner surface of the negative pressure face mask assembly forms a cambered surface structure, and a make-way notch or a flexible component is arranged in the middle of the arc-shaped structure.

In this example, the gender of the patient is determined first. Under the condition that the patient is a female, the inner surface of the negative pressure face mask assembly forms the cambered surface structure; The curved surface structure fits to the patient's neck and forms a good seal. A make-way notch is not formed in the middle of the arc-shaped structure, in order to avoid the occurrence of air leakage of the negative pressure face mask assembly. Under the condition that the patient is a male, a make-way notch should be formed in the middle of the arc-shaped structure, in order to make way for the prominentia laryngea of the male patients, wherein the shape of the notch matches the prominentia laryngea. A good seal is formed. Alternatively, a flexible component is arranged in the middle of the arc-shaped structure. The flexible component is elastically deformed under extrusion to fit the prominentia laryngea of the patient. For example, the arc-shaped structure includes an annular seal for docked with the patient. The annular sealing part is made of rubber, silicone, etc. The flexible component may also be circumferentially mounted on a thinned part of the seal. The thinned part is softer, making it easier to conform to the prominentia laryngea of the patient. The flexible component may also be an independent rubber part, silicone part or plastic part that can be elastically deformed.

According to an embodiment of the present disclosure, a model selection apparatus for a negative pressure face mask assembly is provided. As shown in FIG. 15Q, the model selection apparatus 8000 includes a first module 8100, a second module 8200 and a processor 8300. The first module 8100 is configured to obtain an interval distance between both earlobes of a patient. The second module 8200 is configured to obtain a perimeter of the patient's neck. The processor 8300 is configured to the interval distance and the perimeter; determine a width of the negative pressure face mask assembly according to the interval distance; determine a curvature of a side of the negative pressure face mask assembly in contact with the patient according to the perimeter; and determine a model of the negative pressure face mask assembly according to the width and the curvature.

Specifically, the model selection apparatus according to the embodiment of the present disclosure may be a component in an electronic device, such as an integrated circuit or a chip. The electronic device may be a terminal or other device other than the terminal. Exemplarily, the electronic device may be a mobile phone, a tablet computer, a laptop, PDA, a vehicular electronic device, a mobile Internet device (MID), an augmented reality (AR)/virtual reality (VR) device, a robot, a wearable device, an ultra-mobile personal computer (UMPC), a netbook or personal digital assistant (PDA), etc., or may also be a server, a network attached storage (NAS), a personal computer (PC), a television (TV), a teller machine or a self-service machine, etc., which are not specifically limited in the embodiments of the present disclosure.

The model selection apparatus in the embodiments of the present disclosure may be an apparatus with an operating system. The operating system may be a Windows operating system, an Android (Android) operating system, an iOS operating system, or other possible operating systems, which will not be specifically limited in the embodiments of the present disclosure.

The model selection apparatus provided in the embodiment of the present disclosure can realize various processes in the embodiment of the model selection method in FIG. 15F, which will not be repeated here in order to avoid repetition.

Optionally, a mode of the first module 8100 to obtain the interval distance between both earlobes of the patient may be, but not limited to manual data entry, data import via relevant links, etc. A mode of the second module 8200 to obtain the perimeter of the patient's neck may be, but not limited to manual data entry, data import via relevant links, etc., which may be selected by those skilled in the art according to actual needs.

The model selection apparatus enables accurate model selection of the negative pressure face mask assembly.

In one example, the model selection apparatus further includes a third module, configured to obtain a first included angle formed between the underjaw and the neck of the patient when the patient views at eye level.

The processor 8300 is further configured to determine the model of the negative pressure face mask assembly according to the width, the curvature and the first included angle.

In this example, the third module can determine an angle between the underjaw and the neck of the patient when the patient views at eye level by obtaining the first included angle. Each negative pressure face mask assembly can be adapted to an angle between the underjaw and the neck within a set range. The model of the negative pressure face mask assembly adapted to the patient can be determined more accurately according to the determination of the first included angle. The mode of the third module to obtain the first included angle may be, but not limited to manual data entry, data import via relevant links, etc., which may be selected by those skilled in the art according to actual needs.

In one example, the third module is further configured to obtain a second included angle formed between the underjaw and the neck of the patient when looking up. The processor 8300 is further configured to determine the model of the negative pressure face mask assembly according to the width, the curvature, the first included angle and the second included angle.

In this example, the third module can determine the angle between the underjaw and the neck of the patient when the patient is looking up by obtaining the second included angle. Each negative pressure face mask assembly can be adapted to an angle between the underjaw and the neck within a set range. The model of the negative pressure face mask assembly adapted to the patient can be determined more accurately according to the determination of the second included angle. A mode of the third module to obtain the second included angle may be, but not limited to manual data entry, data import via relevant links, etc., which may be selected by those skilled in the art according to actual needs.

In one example, the processor 8300 is further configured to take the model of the negative pressure face mask assembly determined according to the interval distance as a standard in the case that the model of the negative pressure face mask assembly determined according to the interval distance is inconsistent with a model of the negative pressure face mask assembly determined according to the perimeter.

In this example, a priority of the model selection according to the interval distance is higher than that of the model selection according to the perimeter. In general cases, an accuracy of the model selection according to the interval distance is higher than that of the model selection according to the perimeter. In this way, the accuracy of the model selection of the negative pressure face mask assembly can be ensured.

In one example, the processor 8300 is further configured to take the model of the negative pressure face mask assembly determined according to the perimeter as a standard in the case that the model of the negative pressure face mask assembly determined according to the perimeter is inconsistent with the model of the negative pressure face mask assembly determined according to the second included angle.

In this example, a priority of the model selection according to the perimeter is higher than that of the model selection according to the second included angle. In general cases, an accuracy of the model selection according to the perimeter is higher than that of the model selection according to the second included angle. In this way, the accuracy of the model selection of the negative pressure face mask assembly can be ensured.

In one example, the processor 8300 is further configured to take the model of the negative pressure face mask assembly determined according to the second included angle as a standard in the case that the model of the negative pressure face mask assembly determined according to the first included angle is inconsistent with the model of the negative pressure face mask assembly determined according to the second included angle.

In this example, a priority of the model selection according to the second included angle is higher than that of the model selection according to the first included angle. In general cases, the accuracy of the model selection according to the second included angle is higher than that of the model selection according to the first included angle. In this way, the accuracy of the model selection of the negative pressure face mask assembly can be ensured.

In one example, the model selection apparatus further includes: a gender obtaining module 8400, configured to obtain a gender of the patient. The processor 8300 is further configured to, under the condition that the patient is a female, set an inner surface of the negative pressure face mask assembly as a cambered surface structure. Under the condition that the patient is a male, the inner surface of the negative pressure face mask assembly is set as a cambered surface structure, and a make-way notch or a flexible component is arranged in the middle of the cambered surface structure. The mode of the gender obtaining module 8400 to obtain the gender may be, but not limited to manual data entry, data import via relevant links, etc., which may be selected by those skilled in the art according to actual needs.

In a specific embodiment of the present disclosure, the model of the negative pressure face mask assembly is selected according to a plurality of parameters. For illustrative purposes, negative pressure face mask assemblies for men and women are defined as "Man-Mask" and "Woman-Mask", respectively. The models of male negative pressure face mask assemblies include "Man-Mask L", "Man-Mask M", and "Man-Mask S". The first-type negative pressure face mask assembly is "Man-Mask L". The second-type negative pressure face mask assembly is "Man-Mask M". The third-type negative pressure face mask assembly is "Man-Mask S". The female negative pressure face mask assemblies include "Woman-Mask L", "Woman-Mask M", and "Woman-Mask S". The first-type negative pressure face mask assembly is "Woman-Mask L". The second-type negative pressure face mask assembly is "Woman-Mask M". The third-type negative pressure face mask assembly is "Woman-Mask S".

The interval distances, perimeters, first included angles and second included angles of various types of negative pressure face mask assemblies are shown in Table 1. It should be pointed out that, in Table 1, l₁, S, θ₂, and θ₁ are called a first parameter, a second parameter, a third parameter, and a fourth parameter, respectively according to priorities. The first parameter has the highest priority, and the fourth parameter has the lowest priority. When the first parameter, the second parameter, the third parameter, and the fourth parameter cannot be met at the same time, the model of the negative pressure face mask assembly recommended by the highest level parameter shall prevail.

For example, according to measurements, if the first parameter l₁ of a male patient is > 13 cm, but the second parameter S<45 or the third and fourth parameters are not within a recommended range, the model of the negative pressure face mask assembly that should fit the male patient is "Man-Mask L".

**Table 1**

| Gender | Negative pressure face mask assembly | l₁(cm) | S(cm) | *θ*₂(°) | *θ*₁(°) |
|---|---|---|---|---|---|
| Male | Man-Mask L | *l*₁ > 13 | S > 45 | 65-90 | 35-55 |
| | Man-Mask M | 10≤*l*₁≤13 | 35≤S≤45 | 70-85 | 41-50 |
| | Man-Mask S | *l*₁ < 10 | S < 35 | 68-83 | 38-48 |
| Female | Woman-Mask L | *l*₁ > 12.5 | S > 45 | 65-90 | 35-55 |
| | Woman-Mask M | 9.5≤*l*₁≤12.5 | 34≤S≤44 | 70-85 | 41-50 |
| | Woman-Mask S | *l*₁ < 9.5 | S < 34 | 68-83 | 38-48 |

For example, the first parameter l₁ of the male patient is 12 cm according to measurements. The second parameter S is 44 cm. The third parameter θ₂ is 86°, and the fourth parameter θ₁ is 51°. If the model determined according to the first and second parameters is "Man-Mask M", but the model determined according to the third and fourth parameters is "Man-Mask L", the model of the negative pressure face mask assembly that should fit the male patient is "Man-Mask M".

For example, the first parameter l₁ of the female patient is 9 cm according to measurements. The second parameter S is 33cm. The third parameter θ₂ is 69°, and the fourth parameter θ₁ is 37°. If the model determined according to the first parameter, the second parameter and the third parameter is "Woman-Mask S", but the model determined according to the fourth parameter is not with the ranges in Table 1, the model of the negative pressure face mask assembly that should fit the female patient is "Woman-Mask S".

For example, the first parameter of the female patient is 9 cm according to measurements. The second parameter S is 36cm. The third parameter is 84°, and the fourth parameter is 37°. The model determined according to the first parameter is "Woman-Mask S". The model determined according to the second parameter is "Woman-Mask M". The model determined according to the third parameter is "Woman-Mask M". The model determined according to the fourth parameter is not within the ranges in Table 1. The model of the negative pressure face mask assembly that should fit the female patient is "Woman-Mask S".

In order to facilitate an acquisition of the first to fourth parameters above, the present disclosure provides a model selection apparatus FU applied to the model selection of the negative pressure face mask assembly.

According to an embodiment of the present disclosure, the model selection apparatus is used to obtain the first parameter of the patient. As shown in FIG. 15G, the model selection apparatus FU01 includes a sheet body. The sheet body is provided with a plurality of notches. Each notch is of a arc-shaped structure. Different sizes of notches correspond to different models of negative pressure face mask assemblies, respectively. Both ends of each notch are used to fit both earlobes of the patient separately to determine the model of the negative pressure face mask assembly.

The sheet body may be made of a cardboard, a wood board, a plastic product, a textile product, a silicone product, etc. There are three models for male patients and three models for female patients, namely "Man-Mask L", "Man-Mask M", and "Man-Mask S"; "Woman-Mask L", "Woman-Mask M", "Woman-Mask S". The notches of the male patients and the notches of the female patients are located on the same side or on different sides of the sheet body. Next to each notch, the model of the negative pressure face mask assembly is correspondingly marked.

When in use, the set notches are clamped between both earlobes of the patient through the underjaw, front neck or back neck of the patient, and the appropriate size of the negative pressure face mask assembly can be determined by comparing model information of the negative pressure face mask assemblies marked next to the notches.

In one example, as shown in FIG. 15G, the sheet body includes a first part and a second part that are connected together. The first part and the second part can be folded in half. A folded line is formed between the first part and the second part. The notches corresponding to the male patient are located on one side of the first part away from the folded line; and the notches corresponding to the female patient are located on one side of the second part away from the folded line.

In this way, the sheet body can be easily stored, so that the sheet body takes up less space. Moreover, the notches for the male patients and the notches for the female patients are arranged opposite to each other, so that the notches corresponding to different genders can be distinguished.

According to an embodiment of the present disclosure, a model selection apparatus FU02 is provided. The model selection apparatus FU02 is used to obtain a second parameter of a patient. The model selection apparatus is of a strip-like structure. The strip-like structure has scales showing the size models of the negative pressure face mask assemblies, as shown in FIG. 15H. This scales correspond to a scale range of the second parameter S in Table 1. For example, the model selection apparatus includes a model selection apparatus for male patients and a model selection apparatus for female patients. The model selection apparatus for male patients is marked with a size range corresponding to "Man-Mask L", "Man-Mask M", and "Man-Mask S". The model selection apparatus for female patients is marked with a size range corresponding to "Woman-Mask L", "Woman-Mask M", and "Woman-Mask S".

During use, the corresponding model selection apparatus FU02 is selected according to the gender of the patient. After the model selection apparatus is wrapped around the patient's neck, a range corresponding to a position corresponding to a start end of the model selection apparatus is the model of the negative pressure face mask assembly suitable for the patient.

For example, the patient is a male. A male model selection apparatus is selected for measurement. After the model selection apparatus wrapped around the patient's neck, the start end of the model selection apparatus F02 is within an S range. The model applicable to this patient is "Man-Mask S".

According to an embodiment of the present disclosure, a model selection apparatus F03-1 for a negative pressure face mask assembly is provided. This model selection apparatus is used to obtain a first included angle θ₁ and a second included angle θ₂. As shown in FIG. 15G, the model selection apparatus F03-1 includes a sheet structure, and a plurality of model selection protrusions Fu0333 that protrude outward are arranged at an edge of the sheet structure. Each model selection protrusion Fu0333 includes a first hypotenuse and a second hypotenuse at a set included angle. The plurality of model selection protrusions Fu0333 are set at different included angles to correspond to different models of negative pressure face mask assemblies, respectively. The model selection protrusions Fu0333 are clamped between the underjaw and the neck of the patient to determine the model of the negative pressure face mask assembly.

The sheet structure may be made of a cardboard, a wood board, a plastic product, a textile product, a silicone product, etc. There are three models for male patients and three models for female patients, namely "Man-Mask L", "Man-Mask M", and "Man-Mask S"; "Woman-Mask L", "Woman-Mask M", "Woman-Mask S". The model selection protrusions Fu0333 of the male patients and the model selection protrusions Fu0333 of the female patients are located on the same side or on different sides of the sheet structure. Next to each model selection protrusion Fu0333, the model of the negative pressure face mask assembly is correspondingly marked.

When in use, the set model selection protrusions Fu0333 are clamped between the underjaw and the neck of the patient through a lower side of the patient's underjaw, and the appropriate size of the negative pressure face mask assembly can be determined by comparing model information of the negative pressure face mask assemblies marked next to the model selection protrusions Fu0333.

It should be noted that when the model selection apparatus F03-1 selects a model according to the measured first included angle θ₁ and second included angle θ₂, an included angle between both sides of the model selection protrusion Fu0333 should correspond to different range values. The specific range values can be determined by referring to Table 1.

Certainly, the data in Table 1 are only used as examples, and those skilled in the art can determine the ranges of various parameters corresponding to different types of negative pressure face mask assemblies according to actual needs.

In one example, the sheet body includes a third part and a fourth part that are connected together. The third part and the fourth part can be folded in half. A folded line is formed between the third part and the fourth part. The model selection protrusions Fu0333 corresponding to the male patients are located on one side of the third part away from the folded line; and the model selection protrusions Fu0333 corresponding to the female patients are located on one side of the fourth part away from the folded line.

In this way, the sheet structure can be easily folded and stored, so that the sheet structure takes up less space. Moreover, the model selection protrusions Fu0333 corresponding to the male patients and the model selection protrusions Fu0333 for the female patients are arranged opposite to each other, so that the model selection protrusions Fu0333 corresponding to different genders can be distinguished.

It should be emphasized that positions where the above parameters are acquired are shown only with respect to an identifier relative to the patient, and do not represent a restriction on the acquired positions. For example, the position where the first parameter is acquired is changed to both sides of the patient's underjaw or left and right jaw bones, which also falls within the protection scope of this patent. For another example, the position where the second parameter is acquired is changed to upper or lower positions of the neck, or to include circumferential dimensions of the underjaw and posterior occipital bone, which may be considered to be equivalent changes in the this disclosure.

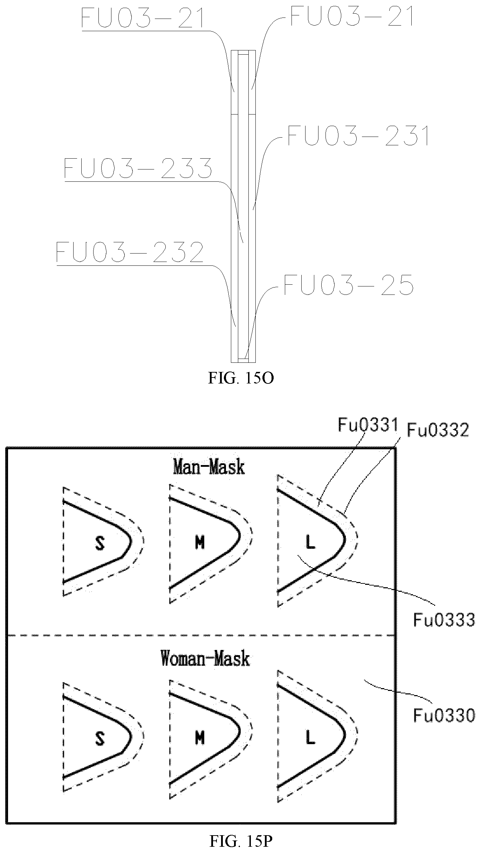

According to an embodiment of the present disclosure, a model selection apparatus applied to the above model selection method is provided. As shown in FIG. 15P, the apparatus includes a sheet structure Fu0330. A plurality of model selection protrusions Fu0333 are arranged in the sheet structure Fu0330. The model selection protrusions Fu0333 are connected to the sheet structure Fu0330 by easy-to-tear parts. The plurality of model selection protrusions Fu0333 are set at different included angles to correspond to different models of negative pressure face mask assemblies, respectively. The model selection protrusions Fu0333 are clamped between the underjaw and the neck of the patient to determine the model of the negative pressure face mask assembly.

The sheet structure Fu0330 may be made of a cardboard, a wood board, a plastic product, a textile product, a silicone product, etc. For example, the sheet structure Fu0330 and the model selection protrusions Fu0333 are of an integrated structure. For example, a thin sheet material is used as a whole, and the sheet structure Fu0330 and the model selection protrusions Fu0333 are formed by providing easy-to-tear lines Fu0332. The easy-to-tear part allows at least part of the model selection protrusion Fu0333 to be separated from the sheet structure Fu0330. The easy-to-tear part includes easy-to-tear lines Fu0332 or a plurality of easy-to-tear strips Fu0331 separated by the easy-to-tear lines Fu0332.

In this example, the model selection apparatus has a regular structure and is easy to store. During use, the operator only needs to separate at least part of the model selection protrusions Fu0333 by the easy-to-tear parts.

In one example, as shown in FIG. 15P, the model selection protrusion Fu0333 can be bent to protrude laterally from the sheet structure Fu0330, that is, the model selection protrusion Fu0333 is partially separated from the sheet structure Fu0330. Alternatively, the model selection protrusion Fu0333 can be removed from the sheet structure Fu0330, that is, the model selection protrusion Fu0333 is completely separated from the sheet structure Fu0330. The model selection protrusions Fu0333 can be used in both of the above methods.

In one example, as shown in FIG. 15P, the model selection protrusion Fu0333 has a triangular structure. An angle of the triangular structure that is used to contact with the patient forms a chamfer.

For example, the chamfer can prevent the triangular structure from stinging the patient's skin when used. In a stored state, three sides of the triangular structure are respectively connected to the sheet structure Fu0330 through the easy-to-tear lines Fu0332. During use, the easy-to-tear lines Fu0332 at both sides for forming the chamfer are cut off, so that the triangular structure can rotate around a side opposite to the chamfer as an axis to protrude from the sheet structure Fu0330. Alternatively, the easy-to-tear lines Fu0332 are arranged at three sides of the triangular structure. The easy-to-tear lines Fu0332 at three sides are cut off, such that the triangular structure is removed from the sheet structure Fu0330.

In one example, as shown in FIG. 15P, each easy-to-tear part includes an easy-to-tear strip Fu0331. The model selection protrusion Fu0333 is of a triangular structure. Two sides of the triangular structure which are used to contact with the patient are connected to the sheet structure Fu0330 through the easy-to-tear strips Fu0331.

For example, a plurality of easy-to-tear strips Fu0331 are separated by the easy-to-tear lines Fu0332. The easy-to-tear strips Fu0331 are arranged at both sides for forming the chamfer. The easy-to-tear strips Fu0331 can be removed as a whole, thereby forming two sides of the triangular structure in contact with the patient. For example, the easy-to-tear strips Fu0331 corresponding to two sides forms an integral structure. The operator only needs to tear from one end of the easy-to-tear strip Fu0331 to separate the two sides. The easy-to-tear strip Fu0331 makes it easier to form the model selection protrusion Fu0333.

According to an embodiment of the present disclosure, a model selection apparatus applied to a negative pressure face mask is provided. This apparatus is used to measure an included angle from the underjaw to the neck of the patient. As shown in FIG. 15J, the model selection apparatus includes a first arm FU03-21, a second arm FU03-22 and a scaleplate FU03-23.

One end of the second arm FU03-22 is rotatably connected to one end of the first arm FU03-21. For example, one end of the first arm FU03-21 and one end of the second arm FU03-22 are connected together by a rotating shaft FU03-24. The second arm FU03-22 can rotate around the first arm FU03-21.

For example, the rotating shaft FU03-24 forms an integral structure with the first arm FU03-21, forms an integral structure with the second arm FU03-22 or is arranged separately from the first arm FU03-21 and the second arm FU03-22.

The scaleplate FU03-23 is connected to the other end of the first arm FU03-21. For example, the scaleplate FU03-23 extends toward a side of the first arm FU03-21. The scaleplate FU03-23 is provided with a model selection scale line. The second arm FU03-22 can swing along the scaleplate FU03-23 relative to the first arm FU03-21, so that the first arm FU03-21 and the second arm FU03-22 are formed at a set included angle. The model of the negative pressure face mask is determined according to a position of the second arm FU03-22 on the model selection scale line.

The model selection scale line can display an included angle between the second arm FU03-22 and the first arm FU03-21 before. A model of the negative pressure face mask is determined according to the size of the included angle. For example, the larger the included angle is, and the larger the size of the fitted negative pressure face mask; and the smaller the included angle is, and the smaller the size of the fitted negative pressure face mask is.

For example, the first arm FU03-21, the second arm FU03-22 and the scaleplate FU03-23 are made of a metal, plastic, ceramic, etc. The first arm FU03-21 and the second arm FU03-22 may be rod-shaped, sheet-shaped, etc.

During use, one ends of the first arm FU03-21 and the second arm FU03-22, which are connected, are abutted against the patient's neck. One of the first arm FU03-21 and the second arm FU03-22 is abutted against the patient's underjaw, and the other against a part of the patient from neck to chest. The size of the included angle between the underjaw and the neck is determined according to a position of the second arm FU03-22 on the model selection scale line of the scaleplate FU03-23.

For example, an included angle between the first arm FU03-21 and the second arm FU03-22 is measured separately when the patient views at eye level and when the patient raises his head to a maximum angle. These two included angles define a movement range of the patient's head. The model of the negative pressure face mask is determined according to the two included angles to ensure the accuracy of model selection.

An included angle between the first arm FU03-21 and the second arm FU03-22 is measured only when the patient views at eye level and when the patient raises his head, and the model of the negative pressure face mask is determined according to the size of this included angle.

In the embodiment of the present disclosure, according to the model selection apparatus, the size of an included angle between the underjaw and the neck can be determined by the swing of the second arm FU03-22 relative to the first arm FU03-21 and the position of the second arm FU03-22 on the model selection scale line, and the model of the negative pressure face mask can be determined according to the size of this included angle.

The apparatus has characteristics of being easy to use and carry, and high measurement accuracy.

Moreover, the negative pressure face mask can be made on the basis of the size of the included angle, such that the negative pressure face mask can be made more accurately.

In one example, the scaleplate FU03-23 is provided with an angle scale line. The angle scale line can display the included angle between the first arm FU03-21 and the second arm FU03-22 accurately. The first arm FU03-21 and/or the second arm FU03-22 are/is provided with a length scale line. The length scale line can display a depth from a front section of the patient's underjaw to the neck accurately. The above two scales allow for more precise determination of a rise magnitude of the patient's head, such that the model of the negative pressure face mask can be selected more accurately.

Alternatively, the first arm FU03-21 and the second arm FU03-22 may be respectively provided a length scale line, such that the depth from the front section of the patient's underjaw to the neck can be further accurately displayed, allowing for more precise model selection for the negative pressure face mask.

It should be noted that a mode of determining a starting point of the included angle measurement and the included angle can be determined according to actual needs. For example, the sides corresponding to the first arm FU03-21 and the second arm FU03-22 are selected as a reference point for reading the starting point and the included angle.

In other examples, there is no precise angle scale line on the scaleplate FU03-23. Instead, the appropriate models are marked on the scaleplate FU03-23 corresponding to different models of negative pressure face masks. For example, the negative pressure face masks are divided into small, medium and large sizes. For example, small masks are marked as S; medium masks are marked as M; and large masks are marked as L. Adaptation ranges of S, M, and L of the included angles are sequentially marked on the scaleplate FU03-23 from small to large. A range into which the second arm FU03-22 falls indicates which model is suitable for the patient.

Moreover, the negative pressure face mask can be made on the basis of magnitudes of the included angle and the depth, such that the negative pressure face mask can be made more accurately.

Certainly, the model selection scale line on the scaleplate FU03-23 is not limited to the above embodiments, and may be selected by those skilled in the art according to actual needs.

In one example, the scale FU03-23 can slide relative to the first arm FU03-21 to determine different models.

In this example, one end of the scaleplate FU03-23 is slidably connected to the first arm FU03-21. Compared with the fixed connection between the scaleplate FU03-23 and the first arm FU03-21, a measurable range of a sliding connection is increased. For example, there is a large difference in underjaw sizes between children and adults. By adjusting the position of the scaleplate FU03-23 on the first arm FU03-21, the depth from the patient's underjaw to the neck can be measured more accurately, making the model selection of the negative pressure face mask more precise.

In one example, as shown in FIG. 15J-FIG. 15K, one ends of the second arm FU03-22 and the first arm FU03-21, which are connected with each other, are fillets. Compared with a case where the first arm FU03-21 and the second arm FU03-22 are not provided with fillets, the fillets can prevent the model selection apparatus from causing damage to the patient's neck when contacting the patient's neck.

Certainly, a diameter of each fillet may be selected by those skilled in the art according to actual needs.

In one example, as shown in FIG. 15J-FIG. 15K, the length of the second arm FU03-22 is greater than the length of the first arm FU03-21.

In this example, the length of the second arm FU03-22 is greater than the length of the first arm FU03-21, and the model selection scale line on the scaleplate FU03-23 is arranged next to the second arm FU03-22. In this way, the included angle between the first arm FU03-21 and the second arm FU03-22 can be read conveniently.

In one example, as shown in FIG. 15J-FIG. 15K, one side of the scaleplate FU03-23 away from the rotating shaft FU03-24 is in an arc shape. The rotating shaft FU03-24 is located on a side of an inner arc of the arc shape.

In this example, the arc-shaped scaleplate FU03-23 can determine the size of the included angle between the first arm FU03-21 and the second arm FU03-22 more easily.

Further, as shown in FIG. 15J-FIG. 15K, a side of the scaleplate FU03-23 opposite to the rotating shaft FU03-24 is arranged in parallel with a side of the scaleplate FU03-23 away from the rotating shaft FU03-24. In this example, the scaleplate FU03-23 is in an arc shape as a whole. On the one hand, the scaleplate FU03-23 is easy for users to hold; and on the other hand, radians on both sides of the scaleplate FU03-23 are consistent, and the included angle between the first arm FU03-21 and the second arm FU03-22 can be reflected more accurately by comparing two arc-shaped edges.

Certainly, in other examples, the scaleplate FU03-23 may also be in a straight line.

In one example, as shown in FIG. 15J, one end of the second arm FU03-22 opposite to the rotating shaft FU03-24 protrude outward from one side of the scaleplate FU03-23 away from the rotating shaft FU03-24.

In this example, the second arm FU03-22 protrudes outward from the outside of the scaleplate FU03-23, and the operator can realize an adjustment of the angle between the first arm FU03-21 and the second arm FU03-22 by moving an outward protruding part of the second arm FU03-22. In this way, the included angle between the underjaw and the neck of the patient is measured more easily.

Moreover, because the operator's hand is located outside the first arm FU03-21 and the scaleplate FU03-23, the hand can be avoided from being clamped by the first arm FU03-21 and the second arm FU03-22.

In one example, as shown in FIG. 15O, a limiting structure FU03-25 is arranged on at least one of the first arm FU03-21 and the second arm FU03-22. The limiting structure FU03-25 is constructed to limit an opening angle of the second arm FU03-22 relative to the first arm FU03-21.

For example, a limiting structure FU03-25 which protrudes outward is arranged on one sides of both ends of the first arm FU03-21 close to the second arm FU03-22, respectively. The limiting structures FU03-25 can effectively prevent the second arm FU03-22 from swinging too much or swinging toward an area outside the scaleplate FU03-23. In this way, a convenience of using the model selection apparatus can be ensured.

In general cases, an angle at which the patient looks up is not greater than 90°. For example, a maximum included angle between the first arm FU03-21 and the second arm FU03-22 can be set to 90°by setting the limiting structures FU03-25.

Certainly, a selection of the maximum angle is not limited to the above embodiments, and may be selected by those skilled in the art according to actual needs.

In one example, as shown in FIG. 15K-FIG. 15M, the scaleplate FU03-23 includes a first sheet FU03-231 and a second sheet FU03-232. The first sheet FU03-231 is arranged in parallel with the second sheet FU03-232. A gap FU03-233 is arranged between the first sheet FU03-231 and the second sheet FU03-232. The first arm FU03-21 is fixed to at least one of the first sheet FU03-231 and the second sheet FU03-232. The second arm FU03-22 is located in the gap FU03-233.

In this example, the second arm FU03-22 slides along the gap FU03-233 between the first sheet FU03-231 and the second sheet FU03-232. The first sheet FU03-231 and the second sheet FU03-232 play a limiting role. The second arm FU03-22 is sandwiched between the first sheet FU03-231 and the second sheet FU03-232 which arranged in parallel. In this way, the movement of the second arm FU03-22 in a direction perpendicular to its swing direction can be effectively limited, to prevent the second arm FU03-22 from being bent and causing damage to the model selection apparatus.

The first sheet FU03-231 and the second sheet FU03-232 are metal sheets, plastic sheets, etc. The first sheet FU03-231 and the second sheet FU03-232 can be formed by bending from a monolithic arc-shaped sheet. Alternatively, the first sheet FU03-231 and the second sheet FU03-232 are connected together by means of welding, bonding, riveting, etc.

In one example, both ends of the first sheet FU03-231 are connected to both ends of the second sheet FU03-232, respectively. The first arm FU03-21 and one of the first sheet FU03-231 and the second sheet FU03-232 form an integral structure and are located in the same plane. The second arm FU03-22 is located on a side of the first arm FU03-21 close to the gap FU03-233.

In this example, the first sheet FU03-231 and the second sheet FU03-232 have an annular structure as a whole. Since both ends of the first sheet FU03-231 are connected to both ends of the second sheet FU03-232, respectively. A limiting structure FU03-25 is formed at both ends of the first sheet FU03-231 and both ends of the second sheet FU03-232, thereby limiting a swing range of the second arm FU03-22. The second arm FU03-22 is located in the same plane with the first sheet FU03-231 or the second sheet FU03-232, and the second arm FU03-22 is located at a side of the first arm FU03-21 close to the gap FU03-233, so that the overall thickness of the model selection apparatus can be reduced, and the model selection apparatus takes up less space and is more convenient to carry.

For example, the first arm FU03-21 and the first sheet FU03-231 or the second sheet FU03-232 can form an integrated structure by means of stamping, injection molding, etc.

Alternatively, the first arm FU03-21 and the first sheet FU03-231 or the second sheet FU03-232 are welded into a structure by means of welding.

Certainly, a connection mode between the first arm FU03-21 and the first sheet FU03-231 or the second sheet FU03-232 is not limited to the above embodiments. This connection mode may be selected by those skilled in the art according to actual needs.

In one example, as shown in FIG. 15N-FIG. 15O, the model selection apparatus includes two first arms FU03-21 arranged in parallel. Both ends of the first sheet FU03-231 are connected to both ends of the second sheet FU03-232, respectively. One of the two first arms FU03-21 is connected to the first sheet FU03-231, and the other is connected to the second sheet FU03-232. The second arm FU03-22 is located between the two first arms FU03-21.

In this example, the first sheet FU03-231 and the second sheet FU03-232 have an annular structure as a whole. Since both ends of the first sheet FU03-231 are connected to both ends of the second sheet FU03-232, respectively. A limiting structure FU03-25 is formed at both ends of the first sheet FU03-231 and both ends of the second sheet FU03-232, thereby limiting a swing range of the second arm FU03-22.

The second arm FU03-22 is sandwiched between the first sheet FU03-231 and the second sheet FU03-232, and is sandwiched between the two first arms FU03-21, so that the second arm FU03-22 can be effectively protected, and the second arm FU03-22 is placed to move in a direction perpendicular to the swing direction, thereby preventing damages to the model selection apparatus.

Moreover, the first sheet FU03-231 is set in parallel with the second sheet FU03-232, and the two first arms FU03-21 are arranged in parallel. Therefore, the connecting structure between the first arm FU03-21 and the scaleplate FU03-23 is more symmetrical, and has high overall structural strength.

The model selection apparatus has a characteristic of firm structure.

According to an embodiment of the present disclosure, a method for making a negative pressure face mask assembly is provided. The method includes:
acquiring information of a curved surface on a curved surface of a patient's neck;
making a three-dimensional model of a liner assembly according to the acquired information of the curved surface;
making the liner assembly according to the three-dimensional model; and
making the negative pressure face mask assembly according to the liner assembly.

Specifically, the information of the curved surface of the patient's neck is acquired. The information may be acquired by scanning with a 3D scanner, a stereo image, etc.; or depth point cloud information may also be formed with a depth detector; or the information on the curved surface having a three-dimensional form is synthesized from a dynamic video or a plurality of static photos.

The three-dimensional model of the liner assembly is formed by means of mold injection molding, a 3D printing technology, etc. The above method can realize a fit between the liner assembly 1 and the skin of the patient's neck, thereby achieving good comfort and sealability of the liner assembly 1.

### <XIII. Negative pressure generation apparatus>

According to an embodiment of the present disclosure, a negative pressure therapy apparatus is provided. The negative pressure therapy apparatus includes a negative pressure generation apparatus 7. As shown in FIG. 16D, the negative pressure generation apparatus includes a housing 700 and a suction apparatus 79. As shown in FIG. 16A-FIG. 16B, a chamber is formed inside the housing 700. An air extraction pipe which is communicated with the chamber is arranged on the housing 700. An airway valve 771 is arranged on the air extraction pipe. The suction apparatus is arranged in the housing, and configured to suction air to the chamber. The airway valve 771 is opened under the condition that a pressure intensity in the negative pressure face mask is greater than or equal to a first threshold.

The airway valve 771 is used to control the air extraction pipe to be opened or closed, to suction air to the negative pressure face mask 11. Optionally, the airway valve 771 may be, but not limited to, a manual valve, a pneumatic valve, an electromagnetic valve, etc. This may be selected by those skilled in the art according to actual needs. The air extraction pipe is communicated with the negative pressure face mask 11 through a hose 3. The negative pressure face mask 11 is generally worn outside the patient's respiratory tract (e.g., below the underjaw and around the neck). A negative pressure is formed in the negative pressure face mask 11. The patient's respiratory tract is opened under the negative pressure, which effectively prevents the patient from snoring.

The suction apparatus 79 is arranged in the housing 700. The suction apparatus 79 is configured to suction air to the chamber. The suction apparatus 79 suction air to the chamber, the hose 3 that is communicated with the chamber, and the negative pressure face mask 11, so that the negative pressure is formed in the negative pressure face mask 11. It should be noted that the formation of the negative pressure means that the pressure intensity in the negative pressure face mask 11, the hose 3 and/or the chamber is small relative to atmospheric pressure.

For example, the suction apparatus 79 may be, but not limited to: a pump-out suction apparatus, a jet pump, a diffusion pump, a booster pump, etc.

The suction apparatus 79 may also include a suction compression pump, such as VM series, VAA series, PK series, PC series, VCA series, VCC series, VCH series, PH series, FM series, FAA series, PCF series and the like.

In the embodiment of the present disclosure, the negative pressure generation apparatus 7 is provided with a pressure relief valve 751 and an airway valve 771. The airway valve 771 can be opened when the pressure relief valve 751 and the airway valve 771 are controlled to make the pressure intensity in the negative pressure face mask 11 greater than or equal to a first threshold, such that the negative pressure face mask 11 can be vacuumized for the therapy of the patient. This method cannot maintain a stability of the air pressure in the negative pressure face mask, thereby improving a comfort in the therapy of the patient.

In one example, as shown in FIG. 16A-FIG. 16B, a pressure relief pipe and an air extraction pipe which are communicated with the chamber are arranged on the housing. The pressure relief valve 751 is arranged on the pressure relief pipe. An airway valve 771 is arranged on the air extraction pipe. The pressure relief valve 751 is used to pressure relief of the chamber. The airway valve 771 is used to control the air extraction pipe to be opened or closed, to suction air to the negative pressure face mask 11.

The pressure relief valve 751 may be, but not limited to, a pneumatic valve, an electromagnetic valve, or a self-sealing safety valve, etc. The self-sealing safety valve can achieve automatic opening and closing, without the need for a controller. The airway valve 771 may be, but not limited to, a manual valve, a pneumatic valve, an electromagnetic valve, etc., which may be selected by those skilled in the art according to actual needs.

In this example, the airway valve 771 is opened under the condition that the pressure intensity in the negative pressure face mask 11 is greater than or equal to a first threshold.

The airway valve 771 and the pressure relief valve 751 are opened under the condition that the pressure intensity in the negative pressure face mask 11 is less than or equal to a second threshold, and the second threshold is less than the first threshold.

In this embodiment, the negative pressure generation apparatus 7 is provided with a pressure relief valve 751 and an airway valve 771. The airway valve 771 can be opened when the pressure relief valve 751 and the airway valve 771 are controlled to make the pressure intensity in the negative pressure face mask 11 greater than or equal to the first threshold, such that the negative pressure face mask 11 can be vacuumized for the therapy of the patient.

The airway valve 771 and the pressure relief valve 751 are opened under the condition that the pressure intensity in the negative pressure face mask 11 is less than or equal to the second threshold. The second threshold is less than the first threshold. Under this condition, a mechanical failure of the suction apparatus 79 or a feedback of an apparatus for measuring the pressure intensity in the negative pressure face mask 11 being not timely, results in excessive suction of the suction apparatus 79 in the negative pressure face mask 11. At this time, the pressure relief valve 751 is opened to introduce outside air into the chamber, and the airway valve 771 is opened, so that outside air compensates for the negative pressure face mask 11 and avoids damage to the underjaw and/or neck of the patient caused by excessive air extraction of the suction apparatus 79.

This negative pressure generation apparatus 7 has a characteristic of good safety.

In one example, the airway valve 771 and the pressure relief valve 751 are closed under the condition that the pressure intensity in the negative pressure face mask 11 is greater than the second threshold and less than the first threshold.

In this example, when the pressure intensity in the negative pressure face mask 11 is between the first threshold and the second threshold, the pressure intensity in the negative pressure face mask 11 meets the requirements of negative pressure therapy. Under this condition, the pressure relief valve 751 and the airway valve 771 are both closed, to keep the pressure intensity in the negative pressure face mask 11.

It should be noted that those skilled in the art can set magnitudes of the first threshold and the second threshold according to an actual situation of the patient.

In one example, as shown in FIG. 16A-FIG. 16C, the housing 700 includes an inner shell. The inner shell includes a chamber upper shell 74, a chamber main body 75 and a chamber lower shell 76. The chamber upper shell 74, the chamber main body 75 and the chamber lower shell 76 are connected together. The chamber body 75 is located between the chamber upper shell 74 and the chamber lower shell 76. A chamber is formed inside the inner shell. For example, a chamber is arranged in the chamber upper shell 74, the chamber main body 75 and the chamber lower shell 76, respectively. In this example, the chamber main body 75 has a hollow structure inside. The chamber upper shell 74 and the chamber lower shell 76 are sealed at both ends of the hollow structure. The hollow structure is used to accommodate the suction apparatus 79, etc.

For example, the chamber upper shell 74, the chamber main body 75 and the chamber lower shell 76 are made of plastic, metal, or other materials. In order to form a good seal, a sealing ring is arranged between the chamber upper shell 74 and the chamber main body 75 and between the chamber main body 75 and the chamber lower shell 76, respectively. The inner shell has advantages of simple structure and easy assembly.

In one example, as shown in FIG. 16A-FIG. 16B, the suction apparatus 79 has an air inlet pipe 752. The air inlet pipe 752 is arranged on the chamber main body 75, and communicated with the air extraction pipe.

The suction apparatus 79 is arranged in the chamber main body 75. The chamber upper shell 74 and the chamber lower shell 76 can be detached, which makes the mount and replacement of the suction apparatus 79 easier. The air inlet pipe 752 is arranged on the chamber main body 75, so that when the chamber upper shell 74 or the chamber lower shell 76 are disassembled and mounted, it will not interfered by the air inlet pipe 752. The air inlet pipe 752 is communicated with the air extraction pipe to facilitate the vacuumizing of the suction apparatus 79 to the negative pressure face mask 11.

In one example, as shown in FIG. 16A-FIG. 16B, the pressure relief pipe is arranged on the chamber main body 75. Compared with settings at other positions, this mode can simplify the structures of the chamber upper shell 74 and the chamber lower shell 76, and avoid interference by the pressure relief pipe when the chamber upper shell 74 or the chamber lower shell 76 is disassembled and mounted.

In one example, as shown in FIG. 16A-FIG. 16D, the housing 700 includes an outer shell. The outer shell is arranged outside an inner shell. The outer shell can effectively protect the inner shell, thereby extending a service life of the negative pressure generation apparatus 7. The air extraction pipe is arranged on the outer shell, and the air extraction pipe forms a seal with the air inlet pipe 752 on an inner side of the outer shell, so that a suction efficiency of the suction apparatus 79 is guaranteed.

In one example, as shown in FIG. 16A-FIG. 16D, the outer shell includes a bottom shell 77 and an outer cover 71. An accommodating space is formed inside the bottom shell 77. A top and one side of the bottom shell 77 form an open side. The inner shell is arranged in the accommodating space. An upper cover covers the open side in a sealing manner. The air extraction pipe is arranged on the bottom shell 77.

In this example, the bottom shell 77 has a hexahedral structure as a whole. The top and one side of the bottom shell 77 are open, forming an open side. The inner shell can be mounted into the accommodating space by the top or the side of the bottom shell 77. The structure of the bottom shell 77 facilitates the mount and disassembly of the inner shell. For example, the outer cover 71 is L-shaped as a whole to seal the top and one side of the bottom shell 77.

For example, the suction apparatus 79 is firstly mounted in the chamber main body 75 during assembly.

Then, the chamber upper shell 74 and the chamber lower shell 76 are mounted to upper and lower ends of the chamber main body 75.

Next, an assembly consisting of the inner shell and the suction apparatus 79 is mounted into the accommodating space from the top of the bottom shell 77 or one side of the bottom shell 77.

Finally, the outer cover 71 is hermetically mounted on the base shell 77.

In one example, as shown in FIG. 16A-FIG. 16D, the negative pressure generation apparatus 7 further includes a controller. The controller is used to send control signals to perform the above actions. The controller is arranged between the suction apparatus 79 and the housing 700. The controller is connected to the suction apparatus 79 and the airway valve 771. The controller is used to control the suction apparatus 79 and the airway valve 771 according to a comparison result between the pressure intensity in the negative pressure face mask 11 and the first threshold value as well as the second threshold value.

For example, a control mainboard 73 is located between the outer cover 71 and the chamber upper shell 74. The controller is integrated on the control mainboard 73. The controller is connected to the suction apparatus 79, the airway valve 771 and the like through the control mainboard 73.

In this example, the controller is effectively protected by the housing. For example, a display panel 72 is located between the outer cover 71 and the chamber upper shell 74. The display panel 72 is connected to the controller and located on the control mainboard 73. The outer cover 71 is provided with an observation window. The display panel 72 is opposite to the observation window.

The display panel 72 is used to display parameters of the suction apparatus 79, such as a rotation speed, a temperature, suction efficiency and whether it is in a working condition etc., or displaying the pressure intensity in the negative pressure face mask 11, etc.

An operation button 711 is arranged on the outer cover 71. The operation button 711 is connected to the controller. The operation button 711 is used to control working parameters of the suction apparatus 79, such as a rotation speed, suction efficiency, etc.

In this example, the controller is able to acquire the pressure intensity in the negative pressure face mask 11 in real time and compare this pressure intensity with the first threshold and the second threshold. In response to different comparison results, the controller controls the suction apparatus 79 to start, stop and the airway valve 771 to open and close.

For example, when the pressure intensity in the negative pressure face mask 11 is greater than or equal to the first threshold, the controller controls the suction apparatus 79 to start and the airway valve 771 to open, thereby vacuuming the negative pressure face mask 11. It should be noted that, at this time, since an opening condition of the pressure relief valve 751 is not met, the pressure relief valve 751 is in a closed state.

When the pressure intensity in the negative pressure face mask 11 is less than the first threshold and greater than the second threshold, the controller controls the suction apparatus 79 to close and the airway valve 771 to close, such that the negative pressure face mask 11 is made to maintain a set pressure intensity for the therapy of the patient. It should be noted that, at this time, since an opening condition of the pressure relief valve 751 is not met, the pressure relief valve 751 is in a closed state.

When the pressure intensity in the negative pressure face mask 11 is less than or equal to the second threshold, it indicates that the pressure intensity in the negative pressure face mask 11 is too low, and the controller controls the suction apparatus 79 to close and the airway valve 771 to open. At this time, since the opening condition of the pressure relief valve 751 is met, the pressure relief valve 751 opens automatically or under the control of the controller, and the outside air enters the negative pressure face mask 11 through the pressure relief valve 751 and the airway valve 771 to compensate for the air in the negative pressure face mask 11.

When the pressure intensity in the negative pressure face mask 11 is greater than the second threshold, the pressure relief valve 751 closes automatically, or closes under the control of the controller.

In one example, as shown in FIG. 16D, the negative pressure therapy apparatus includes a negative pressure face mask 11, a hose 3 and the negative pressure generation apparatus 7 mentioned above, wherein the negative pressure face mask 11 is connected to the air extraction pipe through the hose 3.

This negative pressure therapy apparatus has a characteristic of good safety.

In one example, as shown in FIG. 16A-FIG. 16C, the negative pressure therapy apparatus further includes a monitoring module 78. The monitoring module 78 includes a pressure sensing module 781 and a probe 782. The pressure sensing module 781 is connected (e.g., electrically connected via a wire) to the probe 782. The probe 782 is disposed in the hose 3 or the negative pressure face mask 11. The pressure sensing module 781 is arranged in the housing 700.

Specifically, since the hose 3 is communicated with the negative pressure face mask 11, the probe 782 can sense the pressure intensity inside the negative pressure face mask 11 whether it is inside the hose 3 or inside the negative pressure face mask 11. For example, the pressure sensing module 781 is connected to the controller by the control mainboard 73 to transmit the pressure intensity sensed by the probe 782 to the controller. The controller compares this pressure intensity with the first threshold and the second threshold to output a control signal.

The pressure sensing module 781 is located between the outer shell and the inner shell, and can thus be effectively protected by the outer shell.

In other examples, the monitoring module 78 also includes a thermometer, a hygrometer, etc. to facilitate the therapy of the patient.

According to another embodiment of the present disclosure, a control method for a negative pressure therapy apparatus is provided. As shown in FIG. 16E, the method includes the following steps.

Acquiring a pressure intensity in a negative pressure face mask 11. For example, the pressure intensity inside the negative pressure face mask 11 is sensed by the pressure sensing module 781.

Assuming the first threshold is greater than the second threshold, the pressure intensity in the negative pressure face mask 11 is compared with the first threshold and/or the second threshold,, wherein,
the airway valve 771 is opened under the condition that the pressure intensity in the negative pressure face mask 11 is greater than or equal to the first threshold; and
the airway valve 771 is closed and the pressure relief valve 751 is opened under the condition that the pressure intensity in the negative pressure face mask 11 is less than or equal to the second threshold.

For example, the controller compares the pressure intensity of the negative pressure face mask 11 with the first threshold and the second threshold, and send out different control signals according to different comparison results, thereby effectively controlling the negative pressure generation apparatus 7.

For example, the pressure sensing module 781 transmits the pressure intensity sensed by the probe 782 to the controller.

When the pressure intensity in the negative pressure face mask 11 is greater than or equal to the first threshold, the controller controls the suction apparatus 79 to start and the airway valve 771 to open, thereby vacuuming the negative pressure face mask 11. It should be noted that, at this time, since an opening condition of the pressure relief valve 751 is not met, the pressure relief valve 751 is in a closed state.

When the pressure intensity in the negative pressure face mask 11 is less than the first threshold and greater than the second threshold, the controller controls the suction apparatus 79 to close and the airway valve 771 to close, such that the negative pressure face mask 11 is made to maintain a set pressure intensity for the therapy of the patient. It should be noted that, at this time, since an opening condition of the pressure relief valve 751 is not met, the pressure relief valve 751 is in a closed state.

When the pressure intensity in the negative pressure face mask 11 is less than or equal to the second threshold, it indicates that the pressure intensity in the negative pressure face mask 11 is too low, and the controller controls the suction apparatus 79 to close and the airway valve 771 to open. At this time, since the opening condition of the pressure relief valve 751 is met, the pressure relief valve 751 opens automatically or under the control of the controller, and the outside air enters the negative pressure face mask 11 through the pressure relief valve 751 and the airway valve 771 to compensate for the air in the negative pressure face mask 11.

When the pressure intensity in the negative pressure face mask 11 is greater than the second threshold, the pressure relief valve 751 closes automatically, or closes under the control of the controller.

This control method can effectively avoid the patient's skin damage caused by excessive vacuuming of the suction apparatus 79.

It should be noted that because the negative pressure face mask 11 and the patient's skin cannot achieve a stable and good sealing effect, the above control method acts repeatedly under the condition of satisfying the corresponding pressure intensity in the negative pressure face mask 11.

Although some specific embodiments of the present disclosure have been described in detail by examples, those skilled in the art should understand that the above examples are for illustrative purposes only and not to limit the scope of the present disclosure. Those skilled in the art should understand that the above embodiments may be modified without departing from the scope and spirit of the present disclosure. The scope of the present disclosure is only limited by the appended claims.

## Claims

1. A model selection method for a negative pressure face mask assembly, comprising:
obtaining an interval distance between both earlobes of a patient, and determining a width of the negative pressure face mask assembly according to the interval distance;
obtaining a perimeter of a neck of the patient, and determining a curvature of a side of the negative pressure face mask assembly in contact with the patient according to the perimeter; and
determining a model of the negative pressure face mask assembly according to the width and the curvature.

2. The model selection method for the negative pressure face mask assembly according to claim 1, further comprising:
obtaining a first included angle formed between an underjaw and the neck of the patient when the patient views at eye level; and
determining the model of the negative pressure face mask assembly according to the width, the curvature and the first included angle.

3. The model selection method for the negative pressure face mask assembly according to claim 2, further comprising:
obtaining a second included angle formed between the underjaw and the neck of the patient when the patient is looking up; and
determining the model of the negative pressure face mask assembly according to the width, the curvature, the first included angle and the second included angle.

4. The model selection method for the negative pressure face mask assembly according to claim 1, wherein the model of the negative pressure face mask assembly determined according to the interval distance is taken as a standard in the case that the model of the negative pressure face mask assembly determined according to the interval distance is inconsistent with a model of the negative pressure face mask assembly determined according to the perimeter.

5. The model selection method for the negative pressure face mask assembly according to claim 3, wherein the model of the negative pressure face mask assembly determined according to the perimeter is taken as a standard in the case that the model of the negative pressure face mask assembly determined according to the perimeter is inconsistent with the model of the negative pressure face mask assembly determined according to the second included angle.

6. The model selection method for the negative pressure face mask assembly according to claim 3, wherein the model of the negative pressure face mask assembly determined according to the second included angle is taken as a standard in the case that the model of the negative pressure face mask assembly determined according to the first included angle is inconsistent with the model of the negative pressure face mask assembly determined according to the second included angle.

7. The model selection method for the negative pressure face mask assembly according to claim 1, wherein the model of the negative pressure face mask assembly corresponding to a gender of the patient comprises a first-type negative pressure face mask assembly, a second-type negative pressure face mask assembly, and a third-type negative pressure face mask assembly; a size of the first-type negative pressure face mask assembly is greater than a size of the second-type negative pressure face mask assembly, and the size of the second-type negative pressure face mask assembly is greater than a size of the third-type negative pressure face mask assembly; an interval distance of the first-type negative pressure face mask assembly is greater than an interval distance of the second-type negative pressure face mask assembly, and the interval distance of the second-type negative pressure face mask assembly is greater than an interval distance of the third-type negative pressure face mask assembly; and a curvature of the first-type negative pressure face mask assembly is greater than a curvature of the second-type negative pressure face mask assembly, and the curvature of the second-type negative pressure face mask assembly is greater than a curvature of the third-type negative pressure face mask assembly.

8. The model selection method for the negative pressure face mask assembly according to any one of claims 1 to 7, comprising: obtaining the gender of the patient, wherein under the condition that the patient is female, an inner surface of the negative pressure face mask assembly forms a cambered surface structure; and under the condition that the patient is male, the inner surface of the negative pressure face mask assembly forms a cambered surface structure, a make-way notch or a flexible component is arranged in the middle of the cambered surface structure.

9. A model selection apparatus applied to the model selection method according to any one of claims 1 to 8, comprising a sheet body, wherein the sheet body is provided with a plurality of notches each presenting an arc-shaped structure; the plurality of notches have different sizes to correspond to different models of the negative pressure face mask assembly respectively, and both ends of each notch are used to suit both earlobes of the patient respectively to determine the model of the negative pressure face mask assembly.

10. The model selection apparatus according to claim 9, wherein the sheet body comprises a first part and a second part which are connected together; the first part and the second part can be folded in half to form a folded line therebetween; the notches corresponding to a male patient are located on one side of the first part away from the folded line; and the notches corresponding to a female patient are located on one side of the second part away from the folded line.

11. A model selection apparatus applied to the model selection method according to any one of claims 1 to 8, comprising a sheet structure, wherein an edge of the sheet structure is provided with a plurality of model selection protrusions that protrude outward; each model selection protrusion comprises a first side and a second side which are arranged at a set included angle; the plurality of the model selection protrusions are arranged at different set included angles to correspond to different models of negative pressure face mask assemblies respectively; and the model selection protrusions are used to be clamped between the underjaw and the neck of the patient to determine the model of the negative pressure face mask assembly.

12. The model selection apparatus according to claim 11, wherein the sheet structure comprises a third part and a fourth part which are connected together; the third part and the fourth part can be folded in half to form a folded line therebetween; the model selection protrusions corresponding to a male patient are located on one side of the third part away from the folded line; and the model selection protrusions corresponding to a female patient are located on one side of the fourth part away from the folded line.

13. A model selection apparatus applied to the model selection method according to any one of claims 1 to 8, comprising a sheet structure, wherein a plurality of model selection protrusions are provided inside the sheet structure; each model selection protrusions is connected to the sheet structure by an easy-to-tear part; included angles of the plurality of the model selection protrusions are set different to correspond to different models of negative pressure face mask assemblies respectively; and the model selection protrusions are used to be clamped between the underjaw and the neck of the patient to determine the model of the negative pressure face mask assembly.

14. The model selection apparatus according to claim 13, wherein the model selection protrusions may be bent to protrude from the sheet body toward a side; or
the model selection protrusions may be removed from the sheet body.

15. The model selection apparatus according to claim 13, wherein the model selection protrusions are of a triangular structure, and an angle of the triangular structure that is used to contact with the patient forms a chamfer.

16. The model selection apparatus according to claim 13, wherein the the easy-to-tear part comprises easy-to-tear strips, the model selection protrusions are of a triangular structure and two sides of the triangular structure which are used to contact with the patient are connected to the sheet structure by the easy-to-tear strips.

17. A model selection apparatus for a negative pressure face mask assembly, comprising:
a first module, configured to obtain an interval distance between both earlobes of a patient;
a second module, configured to obtain a perimeter of a neck of the patient; and
a processor, configured to obtain the interval distance and the perimeter; determine a width of the negative pressure face mask assembly according to the interval distance; determine a curvature of a side of the negative pressure face mask assembly in contact with the patient according to the perimeter; and determine a model of the negative pressure face mask assembly according to the width and the curvature.

18. The model selection apparatus for the negative pressure face mask assembly according to claim 17, further comprising:
a third module, configured to obtain a first included angle formed between an underjaw and the neck of the patient when the patient views at eye level; and
the processor, further configured to determine the model of the negative pressure face mask assembly according to the width, the curvature and the first included angle.

19. The model selection apparatus for the negative pressure face mask assembly according to claim 18, wherein the third module is further configured to obtain a second included angle formed between the underjaw and the neck of the patient when the patient is looking up; and
the processor is further configured to determine the model of the negative pressure face mask assembly according to the width, the curvature, the first included angle and the second included angle.

20. The model selection apparatus for the negative pressure face mask assembly according to claim 17, wherein the processor is further configured to take the model of the negative pressure face mask assembly determined according to the interval distance as a standard in the case that the model of the negative pressure face mask assembly determined according to the interval distance is inconsistent with a model of the negative pressure face mask assembly determined according to the perimeter.

21. The model selection apparatus for the negative pressure face mask assembly according to claim 19, wherein the processor is further configured to take the model of the negative pressure face mask assembly determined according to the perimeter as a standard in the case that the model of the negative pressure face mask assembly determined according to the perimeter is inconsistent with the model of the negative pressure face mask assembly determined according to the second included angle.

22. The model selection apparatus for the negative pressure face mask assembly according to claim 19, wherein the processor is further configured to take the model of the negative pressure face mask assembly determined according to the second included angle as a standard in the case that the model of the negative pressure face mask assembly determined according to the first included angle is inconsistent with the model of the negative pressure face mask assembly determined according to the second included angle.

23. The model selection apparatus for the negative pressure face mask assembly according to any one of claims 17 to 22, further comprising:
a gender obtaining module, configured to obtain a gender of the patient; and
the processor, further configured to under the condition that the patient is female, an inner surface of the negative pressure face mask assembly forms a cambered surface structure; and under the condition that the patient is male, the inner surface of the negative pressure face mask assembly forms a cambered surface structure, a make-way notch or a flexible component is arranged in the middle of the cambered surface structure.
